# EUROPEAN PATENT APPLICATION

(11) **EP 3 750 996 A1**
(43) Date of publication of application: **16.12.2020**
(21) Application number: 19751969.7
(22) Date of filing: 06.02.2019
(51) Int. Cl.: C12N 15/113, A61K 31/7088, A61K 35/761, A61K 48/00, C12N 15/861

(54) **OLIGODENDROCYTE-SPECIFIC PROMOTER, MIRNA SPECIFIC TO PLP1 GENE, VECTOR INCLUDING SAID PROMOTER AND/OR MIRNA, AND PHARMACEUTICAL COMPOSITION INCLUDING SAID VECTOR**

(30) Priority: 07.02.2018 JP 2018019950
(71) Applicant: National Center of Neurology and Psychiatry, Kodaira-shi Tokyo 187-8551 (JP); Nippon Medical School Foundation, Tokyo 113-8602 (JP); Daiichi Sankyo Company, Limited, Tokyo 103-8426 (JP)
(72) Inventor: INOUE, Ken, Kodaira-shi, Tokyo 187-8551 (JP); LI, Heng, Kodaira-shi, Tokyo 187-8551 (JP); OKADA, Takashi, Tokyo 113-8602 (JP); OHKI, Yu, Tokyo 103-8426 (JP); KOIZUMI, Makoto, Tokyo 103-8426 (JP)
(74) Representative: Hoffmann Eitle
(86) International application number: PCT/JP2019/004227
(87) International publication number: WO 2019/156115

(57) **Abstract**

An object of the present invention is to provide a vector capable of oligodendrocyte-specifically suppressing expression of the PLP1 gene for treating PMD caused by abnormality of the PLP1 gene, and a promoter and miRNA therefor, and a pharmaceutical composition comprising the vector. The oligodendrocyte-specific promoter of the present invention comprises a nucleic acid having a sequence identity of at least 90% to a nucleotide sequence set forth in SEQ ID NO: 1. The miRNA of the present invention specific to the PLP1 gene comprises a pair of nucleotide sequences consisting of a specific antisense sequence and sense sequence.

SEQUENCE LISTING

<110>  National Center of Neurology and Psychiatry
       Nippon Medical School
       DAIICHI SANKYO COMPANY, LIMITED

<120>  An oligodendrocyte-specific promoter, a miRNA specific for the
       PLP1 gene, a vector containing the promotor and / or the miRNA, and
        a pharmaceutical composition containing the vector

<130>  FP18-0144-00

<160>  283

<170>  PatentIn version 3.5

<210>  1
<211>  1793
<212>  DNA
<213>  Homo sapiens

<400>  1
ggaaaggtcg gggctgagac tgactttcgg ctggcacagg gcctggacga atgaggccag      60

tgaggcattt cagatgcaga gtttaccgga atgccagcaa attgcatgat aatattttaa     120

tccaatgtgg taaaaaaaaa agtttttaat taatgcaaaa gtccatgatg aataaaatat     180

caaaaattaa agacaggatc cgactttgca cgaccctgcc tcactcactt cctgctaatc     240

cacgggaccg gggagggtga ctgagttacc cctcaacacg cagaaaaagc tagtggccgc     300

tcagctcgca ggcgcagtgg gtgggtgcgc gtggggagca ggcgcgcgca gggggcggga     360

ccgggccgag gttcccgcgc gcggcgagag tttcgggttt tggttcgcag cgcctgcgcg     420

cagaggagac ggcccgcccc cagcccgccc gggtgcccgc tccgcccccc gggctatgta     480

aagcggccgg gctcgggtcg tgccaccgct ggactcccgt gtccctccgc gcaggcgggc     540

ggccccggag cgctggtgcc ggcagaggcg gcgacggtgg cgcccctcct catcatggtg     600

agaggccggg cggggccggg cacggggtag caccagggcg ggaaacgagt gtcggggccc     660

ctcgggagga agcgtcttgc aaacgaggac ccggggctcc gggttcgact tccagttttc     720

ttggtactca gggcggccct gaggtctggg agaagcggag gggttttggg gtgccggaga     780

ggccgctgta aagggaaacg gtggtgtttc tcaggaggga actcgggacc gcagaagctg     840

ctgcgcctct gggagcctgg gtgggcctgg gccgggagcc cgggagctgc tcccgacccc     900

acgcaggccc gcgctggggc agccgcttcg gcgccccctc cccgggccca gtctcatcac     960

gcggaaccgc tgccccgcgc ccgggtgcgg gcgggaggtg gagccagttc agaggagggg    1020

accctttacg gtaaaagctg ccgtcccttg tggcttctag aagataggaa aacaagtggc    1080

tccgagaggc gcttctgaca cacagcacag cggcgggtag agctggatcc taattgcagg    1140

tgccctgtgc tgccctgttc tggacaggca caaagaccac gactttgtgg gggccgggag    1200

agggtagagg aggaggtggc aggcttggtg aaagagggcc tttgtccagt gctctcctgc    1260

cctgcccccc accagccctt ctgtgggacc attgtacccg catgccagac aagagagtat 1320

tatctgttgc ggccattgtt gggggaaggg aggcagtctt ggggtaaccc ttctccactc 1380

ccccccttct ctgtgcactc agccgtggca gctgtggctt ggcccagaca cagagacccc 1440

cacctccaaa gaggacgtcc ttagtaggtg ccaagcatat aagagtgagg ccagtcccag 1500

aaccgcaggc tcctggcgcg ccccgcatgc ctccagcacg tttacctttc cgaagtggca 1560

ggaatgggga aagcgcacgc ttaggagagc ttcagacaag cttccctctt cctcctccac 1620

gaccagaagc ggaaaggtgc tcccggaccg aaagggaaag aaggtccagc actgccccgc 1680

ttgggaaggc acccacaacc agtctaggga ctaggggtaa ggccggcggg gagcccgcga 1740

atgacctggg ctgacatctc ttcccctcct tacacagaac agaggcttct ccc 1793

<210> 2
<211> 59
<212> DNA
<213> Artificial Sequence

<220>
<223> human PLP1-miRNA 1

<400> 2
aaaggaagaa gaaagaggca ggttttggcc actgactgac ctgcctctct tcttccttt 59

<210> 3
<211> 59
<212> DNA
<213> Artificial Sequence

<220>
<223> human PLP1 miRNA 2

<400> 3
aacaccagga gccacacaac ggttttggcc actgactgac cgttgtgtct cctggtgtt 59

<210> 4
<211> 59
<212> DNA
<213> Artificial Sequence

<220>
<223> human PLP1 miRNA 3

<400> 4
ttccatggga gaacaccata cgttttggcc actgactgac gtatggtgct cccatggaa 59

<210> 5
<211> 59
<212> DNA
<213> Artificial Sequence

<220>
<223> human PLP1 miRNA 4

<400>  5
tgagcaggga aaccagtgta ggttttggcc actgactgac ctacactgtt ccctgctca        59


<210>  6
<211>  59
<212>  DNA
<213>  Artificial Sequence

<220>
<223>  human PLP1 miRNA 5

<400>  6
agggctttct gattgacagc cgttttggcc actgactgac ggctgtcaca gaaagccct        59


<210>  7
<211>  59
<212>  DNA
<213>  Artificial Sequence

<220>
<223>  human PLP1 miRNA 6

<400>  7
accccaaaga aacacaatcc agttttggcc actgactgac tggattgttt ctttggggt        59


<210>  8
<211>  3473
<212>  DNA
<213>  Mus musculus

<400>  8
aatcagaaag cccttttcat tgcaggagaa gaggacaaag atactcagag agaaaaagta        60

aaggacagaa gaaggagact ggagagacca ggatccttcc agctgagcaa agtcagccgc       120

aaaacagact agccaacagg ctacaattgg agtcagagtg ccaaagacat gggcttgtta       180

gagtgttgtg ctagatgtct ggtaggggcc ccctttgctt ccctggtggc cactggattg       240

tgtttctttg gagtggcact gttctgtgga tgtggacatg aagctctcac tggtacagaa       300

aagctaattg agacctattt ctccaaaaac taccaggact atgagtatct cattaatgtg       360

attcatgctt tccagtatgt catctatgga actgcctctt tcttcttcct ttatggggcc       420

ctcctgctgg ctgagggctt ctacaccacc ggcgctgtca ggcagatctt tggcgactac       480

aagaccacca tctgcggcaa gggcctgagc gcaacggtaa cagggggcca gaaggggagg       540

ggttccagag gccaacatca agctcattct ttggagcggg tgtgtcattg tttgggaaaa       600

tggctaggac atcccgacaa gtttgtgggc atcacctatg ccctgactgt tgtatggctc       660

ctggtgtttg cctgctcggc tgtacctgtg tacatttact tcaatacctg gaccacctgt       720

cagtctattg ccttccctag caagacctct gccagtatag gcagtctctg cgctgatgcc       780

agaatgtatg gtgttctccc atggaatgct ttccctggca aggtttgtgg ctccaacctt       840

ctgtccatct gcaaaacagc tgagttccaa atgaccttcc acctgtttat tgctgcgttt 900

gtgggtgctg cggccacact agtttccctg ctcaccttca tgattgctgc cacttacaac 960

ttcgccgtcc ttaaactcat gggccgaggc accaagttct gagctcccat agaaactccc 1020

ctttgtctaa tagcaaggct ctaaccacac agcctacagt gttgtgtttt aactctgcct 1080

ttgccactga ttggccctct tcttacttga tgagtataac aagaaaggag agtcttgcag 1140

tgattaatct ctctctgtgg actctccctc ttagtacctc ttttagtcat tttgctccac 1200

agcaggctcc tgctagaaat gggggatgcc tgagaaggtg actccccagc tgcaagtcgc 1260

agaggagtga aagctctaat tgattttgca agcatctcct gaagaccagg atgtgcttcc 1320

ttctcaaagg gcacttccaa ctgaggagag cagaacggaa aggttctcag gtagagagca 1380

gaaatgtccc tggtcttctt gccatcagta ggagtcaaat acattctctt tgatgcacaa 1440

aaccaagaac tcactcttac cttcctgttt ccactgaaga cagaagaaaa taaaaagaat 1500

gctagcagag caatatagca tttgcccaaa tctgcctcct gcagctggga gaagggtgtc 1560

aaagcaagga tctttcgccc ttagaaagag agctctgacg ccagtggcaa tggactattt 1620

aagccctaac tcagccaacc ttccttacgg caattaggga gcacagtgcc tgtatagaca 1680

aagcggggcg gagggggggg gcatcatctg tccttatagc tcattaggaa gagaaacagt 1740

gttgtcagga tcatctcact cccttctcct tgataacagc taccatgaca accttgtggt 1800

ttccaaggag ctgagaatag aaaggaacta gcttatttga aataagactg tgacctaagg 1860

agcatcagtt ggtggatgct aaaggtgtaa tttgaaatgg ccttcgggta aatgcaagat 1920

acttaactct ttggatagca tgtgttcttc ccccacccct atccgctagt tctggcccct 1980

ggcctctggc ataatatctt cacaatggtg ctttttttcc tggggtttta tccattcact 2040

catagcaggt gattagacga tcttgattag tttcatattt cccaattgtt tatctcttgt 2100

ttggagttgt atcagaaaga cctggaggat gattctttga gcatagttct ttttgaaaac 2160

aagaaagaga aactgggcag aaagcatcac aaaaatattt gaaattgtac ggtcccatga 2220

aattattggg aattccccca agtagtctac catttgtaga actaggcttg ataaatttga 2280

acctcaattt gaataattgg tctggtattt tcttttctaa taaatgacag atgattttac 2340

ttgctaatat tatctcagca ttttgataat ttaggcttac catagaagtt actgtctctt 2400

ggtatatata ggtcacataa tagattctgc cagctgttag ctgttcagtt cataagcttc 2460

catagagctc tggagccgca gagaggacag gcagaatttg aaacctaaag aactcccaga 2520

tttcaggctt atcctgtatt tgttaacttt gggtgaaaga aagaaagaaa gaaagaaaga 2580

aagaaagaaa gaaagaaaga aagaaagaaa gaaagaaaga aagaaagaaa gaaagaaaga 2640

aagaaaaaga aaggaaggaa ggaaggaagg aagaaagaaa gaaagaaaga aagaaagaaa 2700

gaaagaaaga aagaaagaaa gaaagaaaga aagagaaaaa aaaagcccct gatcgaattt 2760

cctggaggaa aagttattgt agctgtttca ttgtagattt gtgctgtcat tccccaaagt 2820

gctttctgct gtgttgaaag agatataaga atttacaaga agacacttga gacttgttct 2880

tgggccaata tataaggtaa acaagcagga tgcacaagag tgaggagagc taaaaggaca 2940

tgtaagaaac caatcaagat caaggaaggt gaaataatct atatctttta ttttgttttg 3000

gtttaatata acagataacc aaccattccc ttaaaaatct cacatgcaca cacacacaca 3060

cacacacaca cacgtacaaa gagagttaat caactgcaag tgtttccttc atttctgata 3120

gagaattttg attttaacaa cataaaggat aaacttttag aaactcatct tacaaaatgt 3180

attttataaa attaaagaaa ataaaattaa gaatgttctc aatcaaacat cgtgtccttt 3240

gagtgaattg ttctatttga cctcaataac aggtacttaa ttatagttag ctcgaggtgc 3300

tcatgtatct ttcaggccat gtaagttatt cttatactac ttctatgaaa aatgtaatag 3360

ataatgcatt attattatta ttgtttcttt tttatactaa agatatgaaa aaatatatgc 3420

aaaatgcaaa acaattaccg aaagaaactc agtaaatact tgtctcaaat tga 3473

<210> 9
<211> 59
<212> DNA
<213> Artificial Sequence

<220>
<223> mouse PLP1-miRNA

<400> 9
actccaaaga aacacaatcc agttttggcc actgactgac tggattgttt ctttggagt 59

<210> 10
<211> 59
<212> DNA
<213> Artificial Sequence

<220>
<223> mouse miR-neg

<400> 10
gtatgcatcg aatgagattc cgttttggcc actgactgac ggaatctctc gatgcatac 59

<210> 11
<211> 27
<212> DNA
<213> Artificial Sequence

<220>
<223> 5′ flanking sequence

<400> 11
ctggaggctt gctgaaggct gtatgct 27

<210> 12

<211>  45
<212>  DNA
<213>  Artificial Sequence

<220>
<223>  3′ flanking sequence

<400>  12
caggacacaa ggcctgttac tagcactcac atggaacaaa tggcc                    45


<210>  13
<211>  5538
<212>  DNA
<213>  Artificial Sequence

<220>
<223>  pW-CAG-Venus-WPRE

<400>  13
gcggccgcac gcgagcttgt cgacattgat tattgactag ttattaatag taatcaatta      60

cggggtcatt agttcatagc ccatatatgg agttccgcgt tacataactt acggtaaatg     120

gcccgcctgg ctgaccgccc aacgaccccc gcccattgac gtcaataatg acgtatgttc     180

ccatagtaac gccaataggg actttccatt gacgtcaatg ggtggagtat ttacggtaaa     240

ctgcccactt ggcagtacat caagtgtatc atatgccaag tacgccccct attgacgtcaa    300

tgacggtaaa tggcccgcct ggcattatgc ccagtacatg accttatggg actttcctac     360

ttggcagtac atctacgtat tagtcatcgc tattaccatg gtcgaggtga gccccacgtt     420

ctgcttcact ctccccatct cccccccctc cccaccccca attttgtatt tatttatttt     480

ttaattattt tgtgcagcga tgggggcggg gggggggggg ggggcgccag gcggggcggg     540

gcggggcgag gggcggggcg gggcgaggcg gagaggtgcg gcggcagcca atcagagcgg     600

cgcgctccga aagtttcctt ttatggcgag gcggcggcgg cggcggccct ataaaaagcg     660

aagcgcgcgg cgggcgggag tcgctgcgtt gccttcgccc cgtgccccgc tccgcgccgc     720

ctcgcgccgc ccgccccggc tctgactgac cgcgttactc ccacaggtga gcgggcggga     780

cggcccttct cctccgggct gtaattagcg cttggtttaa tgacggcttg tttcttttct     840

gtggctgcgt gaaagccttg aggggctccg ggagggccct ttgtgcgggg ggagcggctc     900

ggggggtgcg tgcgtgtgtg tgtgcgtggg gagcgccgcg tgcggctccg cgctgcccgg     960

cggctgtgag cgctgcgggc gcggcgcggg gctttgtgcg ctccgcagtg tgcgcgaggg    1020

gagcgcggcc gggggcggtg ccccgcggtg cggggggggc tgcgagggga acaaaggctg    1080

cgtgcggggt gtgtgcgtgg gggggtgagc agggggtgtg ggcgcgtcgg tcgggctgca    1140

accccccctg cacccccctc cccgagttgc tgagcacggc ccggcttcgg gtgcggggct    1200

ccgtacgggg cgtggcgcgg ggctcgccgt gccgggcggg gggtggcggc aggtgggggt    1260

gccgggcggg gcggggccgc ctcgggccgg ggagggctcg gggggagggc gcggcggccc    1320

ccggagcgcc ggcggctgtc gaggcgcggc gagccgcagc cattgccttt tatggtaatc 1380

gtgcgagagg gcgcagggac ttcctttgtc ccaaatctgt gcggagccga aatctgggag 1440

gcgccgccgc accccctcta gcgggcgcgg ggcgaagcgg tgcggcgccg gcaggaagga 1500

aatgggcggg gagggccttc gtgcgtcgcc gcgccgccgt ccccttctcc ctctccagcc 1560

tcggggctgt ccgcgggggg acggctgcct tcggggggga cggggcaggg cggggttcgg 1620

cttctggcgt gtgaccggcg gctctagagc ctctgctaac catgttcatg ccttcttctt 1680

tttcctacag ctcctgggca acgtgctggt tattgtgctg tctcatcatt ttggcaaaga 1740

attcggccca ggcggcccac catggtgagc aagggcgagg agctgttcac cggggtggtg 1800

cccatcctgg tcgagctgga cggcgacgta aacggccaca agttcagcgt gtccggcgag 1860

ggcgagggcg atgccaccta cggcaagctg accctgaagc tgatctgcac caccggcaag 1920

ctgcccgtgc cctggcccac cctcgtgacc accctgggct acggcctgca gtgcttcgcc 1980

cgctaccccg accacatgaa gcagcacgac ttcttcaagt ccgccatgcc cgaaggctac 2040

gtccaggagc gcaccatctt cttcaaggac gacggcaact acaagacccg cgccgaggtg 2100

aagttcgagg gcgacaccct ggtgaaccgc atcgagctga agggcatcga cttcaaggag 2160

gacggcaaca tcctggggca caagctggag tacaactaca acagccacaa cgtctatatc 2220

accgccgaca agcagaagaa cggcatcaag gccaacttca agatccgcca caacatcgag 2280

gacggcggcg tgcagctcgc cgaccactac cagcagaaca cccccatcgg cgacggcccc 2340

gtgctgctgc ccgacaacca ctacctgagc taccagtcca agctgagcaa agaccccaac 2400

gagaagcgcg atcacatggt cctgctggag ttcgtgaccg ccgccgggat cactctcggc 2460

atggacgagc tgtacaagta aggccaggcc ggccggatcc tctagagtcg acctgcagaa 2520

gcttatcgat aatcaacctc tggattacaa aatttgtgaa agattgactg gtattcttaa 2580

ctatgttgct ccttttacgc tatgtggata cgctgcttta atgcctttgt atcatgctat 2640

tgcttcccgt atggctttca ttttctcctc cttgtataaa tcctggttgc tgtctcttta 2700

tgaggagttg tggcccgttg tcaggcaacg tggcgtggtg tgcactgtgt ttgctgacgc 2760

aacccccact ggttggggca ttgccaccac ctgtcagctc ctttccggga ctttcgcttt 2820

ccccctccct attgccacgg cggaactcat cgccgcctgc cttgcccgct gctggacagg 2880

ggctcggctg ttgggcactg acaattccgt ggtgttgtcg gggaaatcat cgtcctttcc 2940

ttggctgctc gcctgtgttg ccacctggat tctgcgcggg acgtccttct gctacgtccc 3000

ttcggccctc aatccagcgg accttccttc ccgcggcctg ctgccggctc tgcggcctct 3060

tccgcgtctt cgccttcgcc ctcagacgag tcggatctcc ctttgggccg cctccccgca 3120

tcgataccgt cgacctcgag gcaagcttgc tctcgagaga tctacgatcc agacatgata 3180

agatacattg atgagtttgg acaaaccaca actagaatgc agtgaaaaaa atgctttatt 3240

tgtgaaattt gtgatgctat tgctttattt gtaaccatta taagctgcaa taaacaagtt 3300

aacaacaaca attgcattca ttttatgttt caggttcagg gggaggtgtg ggaggttttt 3360

tcggatcgta ggtaaccacg tgcggaccga gcggccgctc tagagcatgg ctacgtagat 3420

aagtagcatg gcgggttaat cattaactac aaggaacccc tagtgatgga gttggccact 3480

ccctctctgc gcgctcgctc gctcactgag gccgggcgac caaaggtcgc ccgacgcccg 3540

ggctttgccc gggcggcctc agtgagcgag cgagcgcgca gctgcctgca ggcatgcaag 3600

cttgtgagca aaaggccagc aaaaggccag gaaccgtaaa aaggccgcgt tgctggcgtt 3660

tttccatagg ctccgccccc ctgacgagca tcacaaaaat cgacgctcaa gtcagaggtg 3720

gcgaaacccg acaggactat aaagatacca ggcgtttccc cctggaagct ccctcgtgcg 3780

ctctcctgtt ccgaccctgc cgcttaccgg atacctgtcc gcctttctcc cttcgggaag 3840

cgtggcgctt tctcaatgct cacgctgtag gtatctcagt tcggtgtagg tcgttcgctc 3900

caagctgggc tgtgtgcacg aaccccccgt tcagcccgac cgctgcgcct tatccggtaa 3960

ctatcgtctt gagtccaacc cggtaagaca cgacttatcg ccactggcag cagccactgg 4020

taacaggatt agcagagcga ggtatgtagg cggtgctaca gagttcttga agtggtggcc 4080

taactacggc tacactagaa ggacagtatt tggtatctgc gctctgctga agccagttac 4140

cttcggaaaa agagttggta gctcttgatc cggcaaacaa accaccgctg gtagcggtgg 4200

tttttttgtt tgcaagcagc agattacgcg cagaaaaaaa ggatctcaag aagatccttt 4260

gatcttttct acggggtctg acgctcagtg gaacgaaaac tcacgttaag ggattttggt 4320

catgggcgcg cccatgagat tatcaaaaag gatcttcacc tagatccttt taaattaaaa 4380

atgaagtttt aaatcaatct aaagtatata tgagtaaact tggtctgaca gttaccaatg 4440

cttaatcagt gaggcaccta tctcagcgat ctgtctattt cgttcatcca tagttgcctg 4500

actccccgtc gtgtagataa ctacgatacg ggagggctta ccatctggcc ccagtgctgc 4560

aatgataccg cgagacccac gctcaccggc tccagattta tcagcaataa accagccagc 4620

cggaagggcc gagcgcagaa gtggtcctgc aactttatcc gcctccatcc agtctattaa 4680

ttgttgccgg gaagctagag taagtagttc gccagttaat agtttgcgca acgttgttgc 4740

cattgctaca ggcatcgtgg tgtcacgctc gtcgtttggt atggcttcat tcagctccgg 4800

ttcccaacga tcaaggcgag ttacatgatc ccccatgttg tgcaaaaaag cggttagctc 4860

cttcggtcct ccgatcgttg tcagaagtaa gttggccgca gtgttatcac tcatggttat 4920

ggcagcactg cataattctc ttactgtcat gccatccgta agatgctttt ctgtgactgg 4980

tgagtactca accaagtcat tctgagaata gtgtatgcgg cgaccgagtt gctcttgccc 5040

ggcgtcaata cgggataata ccgcgccaca tagcagaact ttaaaagtgc tcatcattgg 5100

aaaacgttct tcggggcgaa aactctcaag gatcttaccg ctgttgagat ccagttcgat 5160

gtaacccact cgtgcaccca actgatcttc agcatctttt actttcacca gcgtttctgg 5220

gtgagcaaaa acaggaaggc aaaatgccgc aaaaaaggga ataagggcga cacggaaatg 5280

ttgaatactc atactcttcc tttttcaata ttattgaagc atttatcagg gttattgtct 5340

cagacctgca ggcagctgcg cgctcgctcg ctcactgagg ccgcccgggc aaagcccggg 5400

cgtcgggcga cctttggtcg cccggcctca gtgagcgagc gagcgcgcag agagggagtg 5460

gccaactcca tcactagggg ttccttgtag ttaatgatta acccgccatg ctacttatct 5520

acgtagccat gctctaga 5538

<210> 14
<211> 733
<212> DNA
<213> Artificial Sequence

<220>
<223> Venus

<400> 14
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagct gatctgcacc accggcaagc tgcccgtgcc ctggcccacc 180

ctcgtgacca ccctgggcta cggcctgcag tgcttcgccc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca ccgccgacaa gcagaagaac 480

ggcatcaagg ccaacttcaa gatccgccac aacatcgagg acggcggcgt gcagctcgcc 540

gaccactacc agcagaacac ccccatcggc gacggccccg tgctgctgcc cgacaaccac 600

tacctgagct accagtccaa gctgagcaaa gaccccaacg agaagcgcga tcacatggtc 660

ctgctggagt tcgtgaccgc cgccgggatc actctcggca tggacgagct gtacaagtaa 720

ggccaggccg gcc 733

<210> 15
<211> 5155
<212> DNA
<213> Artificial Sequence

<220>
<223> pscW-PABPN1

<400> 15

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt ggagatcggt acttcgcgaa 420

tgcgtcgaga tgcccaatcc atgggcctgc aggcagctgc gcgctcgctc gctcactgag 480

gccgcccggg caaagcccgg gcgtcgggcg acctttggtc gcccggcctc agtgagcgag 540

cgagcgcgca gagagggagt ggggttatcg gcgcgccgcg gccgctctag aaaaaaacct 600

cccacacctc cccctgaacc tgaaacataa aatgaatgca attgttgttg ttaacttgtt 660

tattgcagct tataatggtt acaaataaag caatagcatc acaaatttca caaataaagc 720

atttttttca ctgcattcta gttgtggttt gtccaaactc atcaatgtat cttatcatgt 780

ctggatcccc ggatccgggg ccggcctggc caggcctcgt acgatcgatc ctaggatatc 840

acgcgtctta agtcagtagg ggctgtacca gctggtggcc cggcccggcc cccggtacac 900

ccggccccgg ggccggctgt tgaagccgct gtagaaccgg ctccggctgc tgttgtagtt 960

ggtggtccgg gcccggtacc gggcccgggg gaagccccgg tcggtggtgc tgatgccggg 1020

ccggttggtc cgcttgggga tcaccttgat ctgccggccc cggaacaggc tctcgtccag 1080

ggccaggctg gtccgcacgc tctccttgtc gctgaactcg atgtaggcga agcccttggg 1140

gtggccgctg aacttgtcgc acaggatggt cacccggttc acgctgccgc agccgtggaa 1200

gtgggcctcc agctcctcgg cggtggcgcc gtagtccacg ttgcccacgt agatgctccg 1260

ggcgtcggcc tccatcttct cctcgatgct catgatcacg gggccggcgt tgccgggggg 1320

ggggctcatg ttcatctgct tctccacctc gttctgcagc tccttcagct tctcggcctc 1380

ctcctccatc tcccgcaccc gggccttgat ggcctccagc tcggggtcct cgatggcgcc 1440

gtcgccgggg tcgccctcca ccaggccggg ctcctcctcc tcctcctggc tgccgggggc 1500

gccgctgccg gggccggggc cgggggcgcc ggggggggcc cggggccggg ggggctcctc 1560

ctcgggctcg ggctcgggct cgggctccag cagcagctcc tcgggctcca gctcctcgct 1620

ctccaggccg ttgccgtagt cgccggcgcc gccgggggcg ccctcgccgg cctcgccgcc 1680

ggcgccgggc accaggtgcc gccgccggcc ggggccgctg ccccggccgc cggcggcgcc 1740

cgctgctgct gccgccgccg ccgccgccgc tgctgctgct gctgctgctg ccatggtggg 1800

ccgcctgggc cgaattcgtc gactgcagaa ttcgaagctt gagctcgaga tctgagtccg 1860

gtagcgctag cttttcacga cacctgaaat ggaagaaaaa aactttgaac cactgtctga 1920

ggcttgagaa tgaaccaaga tccaaactca aaaagggcaa attccaagga gaattacatc 1980

aagtgccaag ctggcctaac ttcagtctcc acccactcag tgtggggaaa ctccatcgca 2040

taaaacccct ccccccaacc taaagacgac gtactccaaa agctcgagaa ctaatcgagg 2100

tgcctggacg gcgcccggta ctccgtggag tcacatgaag cgacggctga ggacggaaag 2160

gcccttttcc tttgtgtggg tgactcaccc gcccgctctc ccgagcgccg cgtcctccat 2220

tttgagctcc ctgcagcagg gccgggaagc ggccatcttt ccgctcacgc aactggtgcc 2280

gaccgggcca gccttgccgc ccagggcggg gcgatacacg gcggcgcgag gccaggcacc 2340

agagcaggcc ggccagcttg agactacccc cgtccgattc tcggtggccg cgctcgcagg 2400

ccccgcctcg ccgaacatgt gcgctgggac gcacgggccc cgtcgccgcc cgcgggaacc 2460

acacacggca cttacctgtg ttctggcggc aaacccgttg cgaaaaagaa cgttcacggc 2520

gactactgca cttatatacg gttctccccc accctcggga aaaaggcgga gccagtacac 2580

gacatcactt tcccagttta ccccgcgcca ccttctctag gcaccggttc aattgccgac 2640

ccctcccccc aacttctcgg ggactgtggg cgatgtgcgc tctgcccact gacgggcacc 2700

ggagcctcac tagtgcggcc gcaggaaccc ctagtgatgg agttggccac tccctctctg 2760

cgcgctcgct cgctcactga ggccgggcga ccaaaggtcg cccgacgccc gggctttgcc 2820

cgggcggcct cagtgagcga gcgagcgcgc agctgcctgc aggcctagga tttgggatcg 2880

gatgccggga ccgacgagtg cagaggcgtg caagcgagct tggcgtaatc atggtcatag 2940

ctgtttcctg tgtgaaattg ttatccgctc acaattccac acaacatacg agccggaagc 3000

ataaagtgta aagcctgggg tgcctaatga gtgagctaac tcacattaat tgcgttgcgc 3060

tcactgcccg ctttccagtc gggaaacctg tcgtgccagc tgcattaatg aatcggccaa 3120

cgcgcgggga gaggcggttt gcgtattggg cgctcttccg cttcctcgct cactgactcg 3180

ctgcgctcgg tcgttcggct gcggcgagcg gtatcagctc actcaaaggc ggtaatacgg 3240

ttatccacag aatcagggga taacgcagga aagaacatgt gagcaaaagg ccagcaaaag 3300

gccaggaacc gtaaaaaggc cgcgttgctg gcgtttttcc ataggctccg cccccctgac 3360

gagcatcaca aaaatcgacg ctcaagtcag aggtggcgaa acccgacagg actataaaga 3420

taccaggcgt ttccccctgg aagctccctc gtgcgctctc ctgttccgac cctgccgctt 3480

accggatacc tgtccgcctt tctcccttcg ggaagcgtgg cgctttctca tagctcacgc 3540

tgtaggtatc tcagttcggt gtaggtcgtt cgctccaagc tgggctgtgt gcacgaaccc 3600

cccgttcagc ccgaccgctg cgccttatcc ggtaactatc gtcttgagtc caacccggta 3660

agacacgact tatcgccact ggcagcagcc actggtaaca ggattagcag agcgaggtat 3720

gtaggcggtg ctacagagtt cttgaagtgg tggcctaact acggctacac tagaagaaca 3780

gtatttggta tctgcgctct gctgaagcca gttaccttcg gaaaaagagt tggtagctct 3840

tgatccggca aacaaaccac cgctggtagc ggtggttttt ttgtttgcaa gcagcagatt 3900

acgcgcagaa aaaaaggatc tcaagaagat cctttgatct tttctacggg gtctgacgct 3960

cagtggaacg aaaactcacg ttaagggatt ttggtcatga gattatcaaa aaggatcttc 4020

acctagatcc ttttaaatta aaaatgaagt tttaaatcaa tctaaagtat atatgagtaa 4080

acttggtctg acagttacca atgcttaatc agtgaggcac ctatctcagc gatctgtcta 4140

tttcgttcat ccatagttgc ctgactcccc gtcgtgtaga taactacgat acgggagggc 4200

ttaccatctg gccccagtgc tgcaatgata ccgcgagacc cacgctcacc ggctccagat 4260

ttatcagcaa taaaccagcc agccggaagg gccgagcgca gaagtggtcc tgcaacttta 4320

tccgcctcca tccagtctat taattgttgc cgggaagcta gagtaagtag ttcgccagtt 4380

aatagtttgc gcaacgttgt tgccattgct acaggcatcg tggtgtcacg ctcgtcgttt 4440

ggtatggctt cattcagctc cggttcccaa cgatcaaggc gagttacatg atcccccatg 4500

ttgtgcaaaa aagcggttag ctccttcggt cctccgatcg ttgtcagaag taagttggcc 4560

gcagtgttat cactcatggt tatggcagca ctgcataatt ctcttactgt catgccatcc 4620

gtaagatgct tttctgtgac tggtgagtac tcaaccaagt cattctgaga atagtgtatg 4680

cggcgaccga gttgctcttg cccggcgtca atacgggata ataccgcgcc acatagcaga 4740

actttaaaag tgctcatcat tggaaaacgt tcttcggggc gaaaactctc aaggatctta 4800

ccgctgttga gatccagttc gatgtaaccc actcgtgcac ccaactgatc ttcagcatct 4860

tttactttca ccagcgtttc tgggtgagca aaaacaggaa ggcaaaatgc cgcaaaaaag 4920

ggaataaggg cgacacggaa atgttgaata ctcatactct tcctttttca atattattga 4980

agcatttatc agggttattg tctcatgagc ggatacatat ttgaatgtat ttagaaaaat 5040

aaacaaatag gggttccgcg cacatttccc cgaaaagtgc cacctgacgt ctaagaaacc 5100

attattatca tgacattaac ctataaaaat aggcgtatca cgaggccctt tcgtc 5155

<210> 16
<211> 5865
<212> DNA
<213> Artificial Sequence

<220>
<223> Pscw-hCNP-Venus-PLP1 miRNA

<400> 16
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt ggagatcggt acttcgcgaa 420

tgcgtcgaga tgcccaatcc atgggcctgc aggcagctgc gcgctcgctc gctcactgag 480

gccgcccggg caaagcccgg gcgtcgggcg acctttggtc gcccggcctc agtgagcgag 540

cgagcgcgca gagagggagt ggggttatcg gcgcgccgcg gccgcacgcg agcttgtcga 600

cattgattat tgactagtta atgcaaaagt ccatgatgaa taaaatatca aaaattaaag 660

acaggatccg actttgcacg accctgcctc actcacttcc tgctaatcca cgggaccggg 720

gagggtgact gagttacccc tcaacacgca gaaaaagcta gcggccgctc agctcgcagg 780

cgcagtgggt gggtgcgcgt ggggagcagg cgcgcgcagg gggcgggacc gggccgaggt 840

tcccgcgcgc ggcgagagtt tcgggttttg gttcgcagcg cctgcgcgca gaggagacgg 900

cccgccccca gcccgcccgg gtgcccgctc cgccccccgg gctatgtaaa gcggccgggc 960

tcgggttgtg ccaccgctgg actcccgtgt ccctccgcgc aggcgggcgg ccccggagcg 1020

ctggtgccgg cagaggcggc gacggtggcg cccctcctca tcatggtgag aggccgggcg 1080

gggccgggca cggggtagca ccagggcggg aaacgagtgt cggggcccct cgggaggaag 1140

cgtcttgcaa acgaggaccc ggggctccgg gttcgacttc cagttttctt ggtactcagg 1200

gcggccctga ggtctgggag aagcggaggg gttttggggt gccggagagg ccgctgtaaa 1260

gggaaacggt ggtgtttctc aggagggaac tcgggaccgc agaagctgct gcgcctctgg 1320

gagcctgggt gggcctgggc cgggagcccg ggagctgctc ccgaccccac gcaggcccgc 1380

gctggggcag ccgcttcggc gccccctccc cgggcccagt ctcatcacgc ggaaccgctg 1440

ccccgcgccc gggtgcgggc gggaggtgga gccagttcag aggaggggac cctttacggt 1500

aaaagctgcc gtcccttgtg gcttctagaa gataggaaaa caagtggctc cgagaggcgc 1560

ttctgacaca cagcacagcg gcgggtagag ctggatccta attgcaggtg ccctgtgctg 1620

ccctgttctg gacaggcaca aagaccacga ctttgtgggg gccgggagag ggtagaggag 1680

gaggtggcag gcttggtgaa agaggccctt tgtccagtgc tctcctgccc tgccccccac 1740

cagcccttct gtgggaccat tgtacccgca tgccagacaa gagagtatta tctgttgcgg 1800

ccattgttgg gggaagggag gcagtcttgg ggtaaccctt ctccactccc ccccttctct 1860

gtgcactcag ccgtggcagc tgtggcttgg cccagacaca gagaccccca cctccaaaga 1920

ggacgtcctt agtaggtgcc aagcatataa gagtgaggcc agtcccagaa ccgcaggctc 1980

ctggcgcgcc ccgcatgcct ccagcacgtt tacctttccg aagtggcagg aatggggaaa 2040

gcgcacgctt aggagagctt cagacaagct tccctcttcc tcctccacga ccagaagcgg 2100

aaaggtgctc ccggaccgaa agggaaagaa ggtccagcac tgccccgctt gggaaggcac 2160

ccacaaccag tctagggact aggggtaagg ccggcgggga gcccgcgaat gacctgggct 2220

gacatctctt cccctcctta cacagaacag aggcttctcc cgaattcggc ccaggcggcc 2280

caccatggtg agcaagggcg aggagctgtt caccggggtg gtgcccatcc tggtcgagct 2340

ggacggcgac gtaaacggcc acaagttcag cgtgtccggc gagggcgagg gcgatgccac 2400

ctacggcaag ctgaccctga agctgatctg caccaccggc aagctgcccg tgccctggcc 2460

caccctcgtg accaccctgg gctacggcct gcagtgcttc gcccgctacc ccgaccacat 2520

gaagcagcac gacttcttca agtccgccat gcccgaaggc tacgtccagg agcgcaccat 2580

cttcttcaag gacgacggca actacaagac ccgcgccgag gtgaagttcg agggcgacac 2640

cctggtgaac cgcatcgagc tgaagggcat cgacttcaag gaggacggca acatcctggg 2700

gcacaagctg gagtacaact acaacagcca caacgtctat atcaccgccg acaagcagaa 2760

gaacggcatc aaggccaact tcaagatccg ccacaacatc gaggacggcg gcgtgcagct 2820

cgccgaccac taccagcaga acacccccat cggcgacggc cccgtgctgc tgcccgacaa 2880

ccactacctg agctaccagt ccaagctgag caaagacccc aacgagaagc gcgatcacat 2940

ggtcctgctg gagttcgtga ccgccgccgg gatcactctc ggcatggacg agctgtacaa 3000

gtaaggccag gccggccgga tcctggaggc ttgctgaagg ctgtatgctg actccaaaga 3060

aacacaatcc agttttggcc actgactgac tggattgttt ctttggagtc aggacacaag 3120

gcctgttact agcactcaca tggaacaaat ggcccagatc tggccgcact cgaggcaagc 3180

ttgctctcga gagatctacg atccagacat gataagatac attgatgagt ttggacaaac 3240

cacaactaga atgcagtgaa aaaaatgctt tatttgtgaa atttgtgatg ctattgcttt 3300

atttgtaacc attataagct gcaataaaca agttaacaac aacaattgca ttcattttat 3360

gtttcaggtt cagggggagg tgtgggaggt tttttcggat cgtaggtaac cacgtgcgga 3420

ccgagcggcc gcaggaaccc ctagtgatgg agttggccac tccctctctg cgcgctcgct 3480

cgctcactga ggccgggcga ccaaaggtcg cccgacgccc gggctttgcc cgggcggcct 3540

cagtgagcga gcgagcgcgc agctgcctgc aggcctagga tttgggatcg gatgccggga 3600

ccgacgagtg cagaggcgtg caagcgagct tggcgtaatc atggtcatag ctgtttcctg 3660

tgtgaaattg ttatccgctc acaattccac acaacatacg agccggaagc ataaagtgta 3720

aagcctgggg tgcctaatga gtgagctaac tcacattaat tgcgttgcgc tcactgcccg 3780

ctttccagtc gggaaacctg tcgtgccagc tgcattaatg aatcggccaa cgcgcgggga 3840

gaggcggttt gcgtattggg cgctcttccg cttcctcgct cactgactcg ctgcgctcgg 3900

tcgttcggct gcggcgagcg gtatcagctc actcaaaggc ggtaatacgg ttatccacag 3960

aatcagggga taacgcagga aagaacatgt gagcaaaagg ccagcaaaag gccaggaacc 4020

gtaaaaaggc cgcgttgctg gcgtttttcc ataggctccg cccccctgac gagcatcaca 4080

aaaatcgacg ctcaagtcag aggtggcgaa acccgacagg actataaaga taccaggcgt 4140

ttccccctgg aagctccctc gtgcgctctc ctgttccgac cctgccgctt accggatacc 4200

tgtccgcctt tctcccttcg ggaagcgtgg cgctttctca tagctcacgc tgtaggtatc 4260

tcagttcggt gtaggtcgtt cgctccaagc tgggctgtgt gcacgaaccc cccgttcagc 4320

ccgaccgctg cgccttatcc ggtaactatc gtcttgagtc caacccggta agacacgact 4380

tatcgccact ggcagcagcc actggtaaca ggattagcag agcgaggtat gtaggcggtg 4440

ctacagagtt cttgaagtgg tggcctaact acggctacac tagaagaaca gtatttggta 4500

tctgcgctct gctgaagcca gttaccttcg gaaaaagagt tggtagctct tgatccggca 4560

aacaaaccac cgctggtagc ggtggttttt ttgtttgcaa gcagcagatt acgcgcagaa 4620

aaaaaggatc tcaagaagat cctttgatct tttctacggg gtctgacgct cagtggaacg 4680

aaaactcacg ttaagggatt ttggtcatga gattatcaaa aaggatcttc acctagatcc 4740

ttttaaatta aaaatgaagt tttaaatcaa tctaaagtat atatgagtaa acttggtctg 4800

acagttacca atgcttaatc agtgaggcac ctatctcagc gatctgtcta tttcgttcat 4860

ccatagttgc ctgactcccc gtcgtgtaga taactacgat acgggagggc ttaccatctg 4920

gccccagtgc tgcaatgata ccgcgagacc cacgctcacc ggctccagat ttatcagcaa 4980

taaaccagcc agccggaagg gccgagcgca gaagtggtcc tgcaacttta tccgcctcca 5040

tccagtctat taattgttgc cgggaagcta gagtaagtag ttcgccagtt aatagtttgc 5100

gcaacgttgt tgccattgct acaggcatcg tggtgtcacg ctcgtcgttt ggtatggctt 5160

cattcagctc cggttcccaa cgatcaaggc gagttacatg atcccccatg ttgtgcaaaa 5220

aagcggttag ctccttcggt cctccgatcg ttgtcagaag taagttggcc gcagtgttat 5280

cactcatggt tatggcagca ctgcataatt ctcttactgt catgccatcc gtaagatgct 5340

tttctgtgac tggtgagtac tcaaccaagt cattctgaga atagtgtatg cggcgaccga 5400

gttgctcttg cccggcgtca atacgggata ataccgcgcc acatagcaga actttaaaag 5460

tgctcatcat tggaaaacgt tcttcggggc gaaaactctc aaggatctta ccgctgttga 5520

gatccagttc gatgtaaccc actcgtgcac ccaactgatc ttcagcatct tttactttca 5580

ccagcgtttc tgggtgagca aaaacaggaa ggcaaaatgc cgcaaaaaag ggaataaggg 5640

cgacacggaa atgttgaata ctcatactct tcctttttca atattattga agcatttatc 5700

agggttattg tctcatgagc ggatacatat ttgaatgtat ttagaaaaat aaacaaatag 5760

gggttccgcg cacatttccc cgaaaagtgc cacctgacgt ctaagaaacc attattatca 5820

tgacattaac ctataaaaat aggcgtatca cgaggccctt tcgtc 5865

<210>  17
<211>  5865
<212>  DNA
<213>  Artificial Sequence

<220>
<223>  Pscw-hCNP-Venus-miRneg

<400>  17
tcgcgcgttt cggtgatgac ggtgaaaacc tctgacacat gcagctcccg gagacggtca      60

cagcttgtct gtaagcggat gccgggagca gacaagcccg tcagggcgcg tcagcgggtg     120

ttggcgggtg tcggggctgg cttaactatg cggcatcaga gcagattgta ctgagagtgc     180

accatatgcg gtgtgaaata ccgcacagat gcgtaaggag aaaataccgc atcaggcgcc     240

attcgccatt caggctgcgc aactgttggg aagggcgatc ggtgcgggcc tcttcgctat     300

tacgccagct ggcgaaaggg ggatgtgctg caaggcgatt aagttgggta acgccagggt     360

tttcccagtc acgacgttgt aaaacgacgg ccagtgaatt ggagatcggt acttcgcgaa     420

tgcgtcgaga tgcccaatcc atgggcctgc aggcagctgc gcgctcgctc gctcactgag     480

gccgcccggg caaagcccgg gcgtcgggcg acctttggtc gcccggcctc agtgagcgag     540

cgagcgcgca gagagggagt ggggttatcg gcgcgccgcg gccgcacgcg agcttgtcga     600

cattgattat tgactagtta atgcaaaagt ccatgatgaa taaaatatca aaaattaaag      660

acaggatccg actttgcacg accctgcctc actcacttcc tgctaatcca cgggaccggg     720

gagggtgact gagttacccc tcaacacgca gaaaaagcta gcggccgctc agctcgcagg     780

cgcagtgggt gggtgcgcgt ggggagcagg cgcgcgcagg gggcgggacc gggccgaggt     840

tcccgcgcgc ggcgagagtt tcgggttttg gttcgcagcg cctgcgcgca gaggagacgg     900

cccgccccca gcccgcccgg gtgcccgctc cgccccccgg gctatgtaaa gcggccgggc     960

tcgggttgtg ccaccgctgg actcccgtgt ccctccgcgc aggcgggcgg ccccggagcg    1020

ctggtgccgg cagaggcggc gacggtggcg cccctcctca tcatggtgag aggccgggcg    1080

gggccgggca cggggtagca ccagggcggg aaacgagtgt cggggcccct cgggaggaag    1140

cgtcttgcaa acgaggaccc ggggctccgg gttcgacttc cagttttctt ggtactcagg    1200

gcggccctga ggtctgggag aagcggaggg gttttggggt gccggagagg ccgctgtaaa    1260

gggaaacggt ggtgtttctc aggagggaac tcgggaccgc agaagctgct gcgcctctgg    1320

gagcctgggt gggcctgggc cgggagcccg ggagctgctc ccgaccccac gcaggcccgc    1380

gctggggcag ccgcttcggc gccccctccc cgggcccagt ctcatcacgc ggaaccgctg    1440

ccccgcgccc gggtgcgggc gggaggtgga gccagttcag aggaggggac cctttacggt    1500

aaaagctgcc gtcccttgtg gcttctagaa gataggaaaa caagtggctc cgagaggcgc    1560

ttctgacaca cagcacagcg gcgggtagag ctggatccta attgcaggtg ccctgtgctg    1620

ccctgttctg gacaggcaca aagaccacga ctttgtgggg gccgggagag ggtagaggag 1680

gaggtggcag gcttggtgaa agagggcctt tgtccagtgc tctcctgccc tgccccccac 1740

cagcccttct gtgggaccat tgtacccgca tgccagacaa gagagtatta tctgttgcgg 1800

ccattgttgg gggaagggag gcagtcttgg ggtaaccctt ctccactccc ccccttctct 1860

gtgcactcag ccgtggcagc tgtggcttgg cccagacaca gagaccccca cctccaaaga 1920

ggacgtcctt agtaggtgcc aagcatataa gagtgaggcc agtcccagaa ccgcaggctc 1980

ctggcgcgcc ccgcatgcct ccagcacgtt tacctttccg aagtggcagg aatggggaaa 2040

gcgcacgctt aggagagctt cagacaagct tccctcttcc tcctccacga ccagaagcgg 2100

aaaggtgctc ccggaccgaa agggaaagaa ggtccagcac tgccccgctt gggaaggcac 2160

ccacaaccag tctagggact aggggtaagg ccggcgggga gcccgcgaat gacctgggct 2220

gacatctctt cccctcctta cacagaacag aggcttctcc cgaattcggc ccaggcggcc 2280

caccatggtg agcaagggcg aggagctgtt caccggggtg gtgcccatcc tggtcgagct 2340

ggacggcgac gtaaacggcc acaagttcag cgtgtccggc gagggcgagg gcgatgccac 2400

ctacggcaag ctgaccctga agctgatctg caccaccggc aagctgcccg tgccctggcc 2460

caccctcgtg accaccctgg gctacggcct gcagtgcttc gcccgctacc ccgaccacat 2520

gaagcagcac gacttcttca agtccgccat gcccgaaggc tacgtccagg agcgcaccat 2580

cttcttcaag gacgacggca actacaagac ccgcgccgag gtgaagttcg agggcgacac 2640

cctggtgaac cgcatcgagc tgaagggcat cgacttcaag gaggacggca acatcctggg 2700

gcacaagctg gagtacaact acaacagcca caacgtctat atcaccgccg acaagcagaa 2760

gaacggcatc aaggccaact tcaagatccg ccacaacatc gaggacggcg gcgtgcagct 2820

cgccgaccac taccagcaga acacccccat cggcgacggc cccgtgctgc tgcccgacaa 2880

ccactacctg agctaccagt ccaagctgag caaagacccc aacgagaagc gcgatcacat 2940

ggtcctgctg gagttcgtga ccgccgccgg gatcactctc ggcatggacg agctgtacaa 3000

gtaaggccag gccggccgga tcctggaggc ttgctgaagg ctgtatgctg gtatgcatcg 3060

aatgagattc cgttttggcc actgactgac ggaatctctc gatgcatacc aggacacaag 3120

gcctgttact agcactcaca tggaacaaat ggcccagatc tggccgcact cgaggcaagc 3180

ttgctctcga gagatctacg atccagacat gataagatac attgatgagt ttggacaaac 3240

cacaactaga atgcagtgaa aaaaatgctt tatttgtgaa atttgtgatg ctattgcttt 3300

atttgtaacc attataagct gcaataaaca agttaacaac aacaattgca ttcattttat 3360

gtttcaggtt cagggggagg tgtgggaggt tttttcggat cgtaggtaac cacgtgcgga 3420

ccgagcggcc gcaggaaccc ctagtgatgg agttggccac tccctctctg cgcgctcgct 3480

cgctcactga ggccgggcga ccaaaggtcg cccgacgccc gggctttgcc cgggcggcct 3540

cagtgagcga gcgagcgcgc agctgcctgc aggcctagga tttgggatcg gatgccggga 3600

ccgacgagtg cagaggcgtg caagcgagct tggcgtaatc atggtcatag ctgtttcctg 3660

tgtgaaattg ttatccgctc acaattccac acaacatacg agccggaagc ataaagtgta 3720

aagcctgggg tgcctaatga gtgagctaac tcacattaat tgcgttgcgc tcactgcccg 3780

ctttccagtc gggaaacctg tcgtgccagc tgcattaatg aatcggccaa cgcgcgggga 3840

gaggcggttt gcgtattggg cgctcttccg cttcctcgct cactgactcg ctgcgctcgg 3900

tcgttcggct gcggcgagcg gtatcagctc actcaaaggc ggtaatacgg ttatccacag 3960

aatcagggga taacgcagga aagaacatgt gagcaaaagg ccagcaaaag gccaggaacc 4020

gtaaaaaggc cgcgttgctg gcgtttttcc ataggctccg cccccctgac gagcatcaca 4080

aaaatcgacg ctcaagtcag aggtggcgaa acccgacagg actataaaga taccaggcgt 4140

ttccccctgg aagctccctc gtgcgctctc ctgttccgac cctgccgctt accggatacc 4200

tgtccgcctt tctcccttcg ggaagcgtgg cgctttctca tagctcacgc tgtaggtatc 4260

tcagttcggt gtaggtcgtt cgctccaagc tgggctgtgt gcacgaaccc cccgttcagc 4320

ccgaccgctg cgccttatcc ggtaactatc gtcttgagtc caacccggta agacacgact 4380

tatcgccact ggcagcagcc actggtaaca ggattagcag agcgaggtat gtaggcggtg 4440

ctacagagtt cttgaagtgg tggcctaact acggctacac tagaagaaca gtatttggta 4500

tctgcgctct gctgaagcca gttaccttcg gaaaaagagt tggtagctct tgatccggca 4560

aacaaaccac cgctggtagc ggtggttttt ttgtttgcaa gcagcagatt acgcgcagaa 4620

aaaaaggatc tcaagaagat cctttgatct tttctacggg gtctgacgct cagtggaacg 4680

aaaactcacg ttaagggatt ttggtcatga gattatcaaa aaggatcttc acctagatcc 4740

ttttaaatta aaaatgaagt tttaaatcaa tctaaagtat atatgagtaa acttggtctg 4800

acagttacca atgcttaatc agtgaggcac ctatctcagc gatctgtcta tttcgttcat 4860

ccatagttgc ctgactcccc gtcgtgtaga taactacgat acgggagggc ttaccatctg 4920

gccccagtgc tgcaatgata ccgcgagacc cacgctcacc ggctccagat ttatcagcaa 4980

taaaccagcc agccggaagg gccgagcgca gaagtggtcc tgcaacttta tccgcctcca 5040

tccagtctat taattgttgc cgggaagcta gagtaagtag ttcgccagtt aatagtttgc 5100

gcaacgttgt tgccattgct acaggcatcg tggtgtcacg ctcgtcgttt ggtatggctt 5160

cattcagctc cggttcccaa cgatcaaggc gagttacatg atcccccatg ttgtgcaaaa 5220

aagcggttag ctccttcggt cctccgatcg ttgtcagaag taagttggcc gcagtgttat 5280

cactcatggt tatggcagca ctgcataatt ctcttactgt catgccatcc gtaagatgct 5340

tttctgtgac tggtgagtac tcaaccaagt cattctgaga atagtgtatg cggcgaccga 5400

gttgctcttg cccggcgtca atacgggata ataccgcgcc acatagcaga actttaaaag 5460

tgctcatcat tggaaaacgt tcttcggggc gaaaactctc aaggatctta ccgctgttga 5520

gatccagttc gatgtaaccc actcgtgcac ccaactgatc ttcagcatct tttactttca 5580

ccagcgtttc tgggtgagca aaaacaggaa ggcaaaatgc cgcaaaaaag ggaataaggg 5640

cgacacggaa atgttgaata ctcatactct tcctttttca atattattga agcatttatc 5700

agggttattg tctcatgagc ggatacatat ttgaatgtat ttagaaaaat aaacaaatag 5760

gggttccgcg cacatttccc cgaaaagtgc cacctgacgt ctaagaaacc attattatca 5820

tgacattaac ctataaaaat aggcgtatca cgaggccctt tcgtc 5865

<210> 18
<211> 24
<212> DNA
<213> Artificial Sequence

<220>
<223> PLP1 forward primer

<400> 18
gttccagagg ccaacatcaa gctc 24

<210> 19
<211> 24
<212> DNA
<213> Artificial Sequence

<220>
<223> PLP1 reverse primer

<400> 19
agccatacaa cagtcagggc atag 24

<210> 20
<211> 19
<212> DNA
<213> Artificial Sequence

<220>
<223> Olig2 forward primer

<400> 20
gggaggtcat gccttacgc 19

<210> 21
<211> 19
<212> DNA
<213> Artificial Sequence

<220>
<223> Olig2 reverse primer

<400> 21

ctccagcgag ttggtgagc                                                    19

<210>  22
<211>  22
<212>  DNA
<213>  Artificial Sequence

<220>
<223>  actin forward primer

<400>  22
cacagcttct ttgcagctcc tt                                                 22

<210>  23
<211>  19
<212>  DNA
<213>  Artificial Sequence

<220>
<223>  actin reverse primer

<400>  23
gacgaccagc gcagcgata                                                     19

<210>  24
<211>  59
<212>  DNA
<213>  Artificial Sequence

<220>
<223>  human PLP1 miRNA 7

<400>  24
acaaatgcag caataaacag ggttttggcc actgactgac cctgtttagc tgcatttgt         59

<210>  25
<211>  59
<212>  DNA
<213>  Artificial Sequence

<220>
<223>  human PLP1 miRNA 8

<400>  25
aatagactgg caggtggtcc agttttggcc actgactgac tggaccacgc cagtctatt         59

<210>  26
<211>  59
<212>  DNA
<213>  Artificial Sequence

<220>
<223>  human PLP1 miRNA 9

<400>  26
aaagaatgag cttgatgttg ggttttggcc actgactgac ccaacatcgc tcattcttt         59

<210>  27
<211>  59
<212>  DNA
<213>  Artificial Sequence

<220>
<223>  human PLP1 miRNA 10

<400>  27
agatactcat agtcttggta ggttttggcc actgactgac ctaccaagta tgagtatct          59


<210>  28
<211>  59
<212>  DNA
<213>  Artificial Sequence

<220>
<223>  human PLP1 miRNA 11

<400>  28
aagcccatgt ctttgggact cgttttggcc actgactgac gagtcccaga catgggctt          59


<210>  29
<211>  59
<212>  DNA
<213>  Artificial Sequence

<220>
<223>  pre-miRNA

<400>  29
agaaacacaa tccagtggcc agttttggcc actgactgac tggccactat tgtgtttct          59


<210>  30
<211>  59
<212>  DNA
<213>  Artificial Sequence

<220>
<223>  pre-miRNA

<400>  30
aaataggtct caattagctt tgttttggcc actgactgac aaagctaaga gacctattt          59


<210>  31
<211>  59
<212>  DNA
<213>  Artificial Sequence

<220>
<223>  pre-miRNA

<400>  31
aagaaacaca atccagtggc cgttttggcc actgactgac ggccactgtt gtgtttctt          59


<210>  32
<211>  59
<212>  DNA

<213>  Artificial Sequence

<220>
<223>  pre-miRNA

<400>  32
taaacaggtg gaaggtcatt tgttttggcc actgactgac aaatgacccc acctgttta        59


<210>  33
<211>  59
<212>  DNA
<213>  Artificial Sequence

<220>
<223>  pre-miRNA

<400>  33
ttgtagtcgc caaagatctg cgttttggcc actgactgac gcagatctgg cgactacaa        59


<210>  34
<211>  59
<212>  DNA
<213>  Artificial Sequence

<220>
<223>  pre-miRNA

<400>  34
aattagagcc tccattcctt tgttttggcc actgactgac aaaggaatag gctctaatt        59


<210>  35
<211>  59
<212>  DNA
<213>  Artificial Sequence

<220>
<223>  pre-miRNA

<400>  35
ttaaggacgg caaagttgta agttttggcc actgactgac ttacaactgc cgtccttaa        59


<210>  36
<211>  59
<212>  DNA
<213>  Artificial Sequence

<220>
<223>  pre-miRNA

<400>  36
tttaaggacg gcaaagttgt agttttggcc actgactgac tacaacttcc gtccttaaa        59


<210>  37
<211>  59
<212>  DNA
<213>  Artificial Sequence

<220>

<223>  pre-miRNA

<400>  37
atgtctttgg gactctgact cgttttggcc actgactgac gagtcagacc caaagacat        59


<210>  38
<211>  59
<212>  DNA
<213>  Artificial Sequence

<220>
<223>  pre-miRNA

<400>  38
tatctatcct gtgtctacca ggttttggcc actgactgac ctggtagaca ggatagata        59


<210>  39
<211>  59
<212>  DNA
<213>  Artificial Sequence

<220>
<223>  pre-miRNA

<400>  39
aaattacttt ctgatcctca ggttttggcc actgactgac ctgaggatga aagtaattt        59


<210>  40
<211>  59
<212>  DNA
<213>  Artificial Sequence

<220>
<223>  pre-miRNA

<400>  40
tctaacaagc ccatgtcttt ggttttggcc actgactgac caaagacagg cttgttaga        59


<210>  41
<211>  59
<212>  DNA
<213>  Artificial Sequence

<220>
<223>  pre-miRNA

<400>  41
aattactttc tgatcctcag ggttttggcc actgactgac cctgaggaag aaagtaatt        59


<210>  42
<211>  59
<212>  DNA
<213>  Artificial Sequence

<220>
<223>  pre-miRNA

<400>  42

agtaaatgta cacaggcaca ggttttggcc actgactgac ctgtgcctgt acatttact 59

<210> 43
<211> 59
<212> DNA
<213> Artificial Sequence

<220>
<223> pre-miRNA

<400> 43
taagtaaggt tggctgagtt agttttggcc actgactgac taactcagaa ccttactta 59

<210> 44
<211> 59
<212> DNA
<213> Artificial Sequence

<220>
<223> pre-miRNA

<400> 44
ttctgtgggt gaaagatcct tgttttggcc actgactgac aaggatctca cccacagaa 59

<210> 45
<211> 59
<212> DNA
<213> Artificial Sequence

<220>
<223> pre-miRNA

<400> 45
agaagatgct gacaacaccc tgttttggcc actgactgac agggtgttca gcatcttct 59

<210> 46
<211> 59
<212> DNA
<213> Artificial Sequence

<220>
<223> pre-miRNA

<400> 46
aattgtagcc ggctggctag tgttttggcc actgactgac actagccacg gctacaatt 59

<210> 47
<211> 59
<212> DNA
<213> Artificial Sequence

<220>
<223> pre-miRNA

<400> 47
agatttgggc aaacgctctt agttttggcc actgactgac taagagcgtg cccaaatct 59

<210>  48
<211>  59
<212>  DNA
<213>  Artificial Sequence

<220>
<223>  pre-miRNA

<400>  48
attctacgct cccttatgct ggttttggcc actgactgac cagcataaga gcgtagaat        59


<210>  49
<211>  59
<212>  DNA
<213>  Artificial Sequence

<220>
<223>  pre-miRNA

<400>  49
tggtaataga gagaccagaa tgttttggcc actgactgac attctggtct ctattacca        59


<210>  50
<211>  59
<212>  DNA
<213>  Artificial Sequence

<220>
<223>  pre-miRNA

<400>  50
tatagatggc aagaggacca agttttggcc actgactgac ttggtccttg ccatctata        59


<210>  51
<211>  59
<212>  DNA
<213>  Artificial Sequence

<220>
<223>  pre-miRNA

<400>  51
aaaccagtgt agctgcagcc cgttttggcc actgactgac gggctgcata cactggttt        59


<210>  52
<211>  21
<212>  DNA
<213>  Artificial Sequence

<220>
<223>  human PLP1 antisense sequence

<400>  52
aaaggaagaa gaaagaggca g                                                 21


<210>  53
<211>  19
<212>  DNA

<213>  Artificial Sequence

<220>
<223>  human PLP1 sense sequence

<400>  53
ctgcctctct tcttccttt                                                    19


<210>  54
<211>  21
<212>  DNA
<213>  Artificial Sequence

<220>
<223>  human PLP1 antisense sequence

<400>  54
aacaccagga gccacacaac g                                                 21


<210>  55
<211>  19
<212>  DNA
<213>  Artificial Sequence

<220>
<223>  human PLP1 sense sequence

<400>  55
cgttgtgtct cctggtgtt                                                    19


<210>  56
<211>  21
<212>  DNA
<213>  Artificial Sequence

<220>
<223>  human PLP1 antisense sequence

<400>  56
ttccatggga gaacaccata c                                                 21


<210>  57
<211>  19
<212>  DNA
<213>  Artificial Sequence

<220>
<223>  human PLP1 sense sequence

<400>  57
gtatggtgct cccatggaa                                                    19


<210>  58
<211>  21
<212>  DNA
<213>  Artificial Sequence

<220>

<223>  human PLP1 antisense sequence

<400>  58
tgagcaggga aaccagtgta g                                                21


<210>  59
<211>  19
<212>  DNA
<213>  Artificial Sequence

<220>
<223>  human PLP1 sense sequence

<400>  59
ctacactgtt ccctgctca                                                   19


<210>  60
<211>  21
<212>  DNA
<213>  Artificial Sequence

<220>
<223>  human PLP1 antisense sequence

<400>  60
agggctttct gattgacagc c                                                21


<210>  61
<211>  19
<212>  DNA
<213>  Artificial Sequence

<220>
<223>  human PLP1 sense sequence

<400>  61
ggctgtcaca gaaagccct                                                   19


<210>  62
<211>  21
<212>  DNA
<213>  Artificial Sequence

<220>
<223>  human PLP1 antisense sequence

<400>  62
accccaaaga aacacaatcc a                                                21


<210>  63
<211>  19
<212>  DNA
<213>  Artificial Sequence

<220>
<223>  human PLP1 sense sequence

<400>  63

tggattgttt ctttggggt                                                    19

<210>  64
<211>  21
<212>  DNA
<213>  Artificial Sequence

<220>
<223>  human PLP1 antisense sequence

<400>  64
acaaatgcag caataaacag g                                                 21

<210>  65
<211>  19
<212>  DNA
<213>  Artificial Sequence

<220>
<223>  human PLP1 sense sequence

<400>  65
cctgtttagc tgcatttgt                                                    19

<210>  66
<211>  21
<212>  DNA
<213>  Artificial Sequence

<220>
<223>  human PLP1 antisense sequence

<400>  66
aatagactgg caggtggtcc a                                                 21

<210>  67
<211>  19
<212>  DNA
<213>  Artificial Sequence

<220>
<223>  human PLP1 sense sequence

<400>  67
tggaccacgc cagtctatt                                                    19

<210>  68
<211>  21
<212>  DNA
<213>  Artificial Sequence

<220>
<223>  human PLP1 antisense sequence

<400>  68
aaagaatgag cttgatgttg g                                                 21

<210>  69
<211>  19
<212>  DNA
<213>  Artificial Sequence

<220>
<223>  human PLP1 sense sequence

<400>  69
ccaacatcgc tcattcttt                                                    19


<210>  70
<211>  21
<212>  DNA
<213>  Artificial Sequence

<220>
<223>  human PLP1 antisense sequence

<400>  70
agatactcat agtcttggta g                                                 21


<210>  71
<211>  19
<212>  DNA
<213>  Artificial Sequence

<220>
<223>  human PLP1 sense sequence

<400>  71
ctaccaagta tgagtatct                                                    19


<210>  72
<211>  21
<212>  DNA
<213>  Artificial Sequence

<220>
<223>  human PLP1 antisense sequence

<400>  72
agaaacacaa tccagtggcc a                                                 21


<210>  73
<211>  19
<212>  DNA
<213>  Artificial Sequence

<220>
<223>  human PLP1 sense sequence

<400>  73
tggccactat tgtgtttct                                                    19


<210>  74
<211>  21
<212>  DNA

<213>  Artificial Sequence

<220>
<223>  human PLP1 antisense sequence

<400>  74
aaataggtct caattagctt t                                                21


<210>  75
<211>  19
<212>  DNA
<213>  Artificial Sequence

<220>
<223>  human PLP1 sense sequence

<400>  75
aaagctaaga gacctattt                                                   19


<210>  76
<211>  21
<212>  DNA
<213>  Artificial Sequence

<220>
<223>  human PLP1 antisense sequence

<400>  76
aagaaacaca atccagtggc c                                                21


<210>  77
<211>  19
<212>  DNA
<213>  Artificial Sequence

<220>
<223>  human PLP1 sense sequence

<400>  77
ggccactgtt gtgtttctt                                                   19


<210>  78
<211>  21
<212>  DNA
<213>  Artificial Sequence

<220>
<223>  human PLP1 antisense sequence

<400>  78
taaacaggtg gaaggtcatt t                                                21


<210>  79
<211>  19
<212>  DNA
<213>  Artificial Sequence

<220>

<223>  human PLP1 sense sequence

<400>  79
aaatgacccc acctgttta                                                    19


<210>  80
<211>  21
<212>  DNA
<213>  Artificial Sequence

<220>
<223>  human PLP1 antisense sequence

<400>  80
ttgtagtcgc caaagatctg c                                                 21


<210>  81
<211>  19
<212>  DNA
<213>  Artificial Sequence

<220>
<223>  human PLP1 sense sequence

<400>  81
gcagatctgg cgactacaa                                                    19


<210>  82
<211>  21
<212>  DNA
<213>  Artificial Sequence

<220>
<223>  human PLP1 antisense sequence

<400>  82
aattagagcc tccattcctt t                                                 21


<210>  83
<211>  19
<212>  DNA
<213>  Artificial Sequence

<220>
<223>  human PLP1 sense sequence

<400>  83
aaaggaatag gctctaatt                                                    19


<210>  84
<211>  21
<212>  DNA
<213>  Artificial Sequence

<220>
<223>  human PLP1 antisense sequence

<400>  84

ttaaggacgg caaagttgta a                                                21

<210>  85
<211>  19
<212>  DNA
<213>  Artificial Sequence

<220>
<223>  human PLP1 sense sequence

<400>  85
ttacaactgc cgtccttaa                                                   19

<210>  86
<211>  21
<212>  DNA
<213>  Artificial Sequence

<220>
<223>  human PLP1 antisense sequence

<400>  86
tttaaggacg gcaaagttgt a                                                21

<210>  87
<211>  19
<212>  DNA
<213>  Artificial Sequence

<220>
<223>  human PLP1 sense sequence

<400>  87
tacaacttcc gtccttaaa                                                   19

<210>  88
<211>  21
<212>  DNA
<213>  Artificial Sequence

<220>
<223>  human PLP1 antisense sequence

<400>  88
atgtctttgg gactctgact c                                                21

<210>  89
<211>  19
<212>  DNA
<213>  Artificial Sequence

<220>
<223>  human PLP1 sense sequence

<400>  89
gagtcagacc caaagacat                                                   19

<210>  90
<211>  21
<212>  DNA
<213>  Artificial Sequence

<220>
<223>  human PLP1 antisense sequence

<400>  90
tatctatcct gtgtctacca g                                                21


<210>  91
<211>  19
<212>  DNA
<213>  Artificial Sequence

<220>
<223>  human PLP1 sense sequence

<400>  91
ctggtagaca ggatagata                                                   19


<210>  92
<211>  21
<212>  DNA
<213>  Artificial Sequence

<220>
<223>  human PLP1 antisense sequence

<400>  92
aaattacttt ctgatcctca g                                                21


<210>  93
<211>  19
<212>  DNA
<213>  Artificial Sequence

<220>
<223>  human PLP1 sense sequence

<400>  93
ctgaggatga aagtaattt                                                   19


<210>  94
<211>  21
<212>  DNA
<213>  Artificial Sequence

<220>
<223>  human PLP1 antisense sequence

<400>  94
tctaacaagc ccatgtcttt g                                                21


<210>  95
<211>  19
<212>  DNA

<213>  Artificial Sequence

<220>
<223>  human PLP1 sense sequence

<400>  95
caaagacagg cttgttaga                                                    19


<210>  96
<211>  21
<212>  DNA
<213>  Artificial Sequence

<220>
<223>  human PLP1 antisense sequence

<400>  96
aattactttc tgatcctcag g                                                 21


<210>  97
<211>  19
<212>  DNA
<213>  Artificial Sequence

<220>
<223>  human PLP1 sense sequence

<400>  97
cctgaggaag aaagtaatt                                                    19


<210>  98
<211>  21
<212>  DNA
<213>  Artificial Sequence

<220>
<223>  human PLP1 antisense sequence

<400>  98
agtaaatgta cacaggcaca g                                                 21


<210>  99
<211>  19
<212>  DNA
<213>  Artificial Sequence

<220>
<223>  human PLP1 sense sequence

<400>  99
ctgtgcctgt acatttact                                                    19


<210>  100
<211>  21
<212>  DNA
<213>  Artificial Sequence

<220>

<223>  human PLP1 antisense sequence

<400>  100
taagtaaggt tggctgagtt a                                                21


<210>  101
<211>  19
<212>  DNA
<213>  Artificial Sequence

<220>
<223>  human PLP1 sense sequence

<400>  101
taactcagaa ccttactta                                                   19


<210>  102
<211>  21
<212>  DNA
<213>  Artificial Sequence

<220>
<223>  human PLP1 antisense sequence

<400>  102
ttctgtgggt gaaagatcct t                                                21


<210>  103
<211>  19
<212>  DNA
<213>  Artificial Sequence

<220>
<223>  human PLP1 sense sequence

<400>  103
aaggatctca cccacagaa                                                   19


<210>  104
<211>  21
<212>  DNA
<213>  Artificial Sequence

<220>
<223>  human PLP1 antisense sequence

<400>  104
agaagatgct gacaacaccc t                                                21


<210>  105
<211>  19
<212>  DNA
<213>  Artificial Sequence

<220>
<223>  human PLP1 sense sequence

<400>  105

agggtgttca gcatcttct                                                    19

<210>  106
<211>  21
<212>  DNA
<213>  Artificial Sequence

<220>
<223>  human PLP1 antisense sequence

<400>  106
aattgtagcc ggctggctag t                                                21

<210>  107
<211>  19
<212>  DNA
<213>  Artificial Sequence

<220>
<223>  human PLP1 sense sequence

<400>  107
actagccacg gctacaatt                                                   19

<210>  108
<211>  21
<212>  DNA
<213>  Artificial Sequence

<220>
<223>  human PLP1 antisense sequence

<400>  108
agatttgggc aaacgctctt a                                                21

<210>  109
<211>  19
<212>  DNA
<213>  Artificial Sequence

<220>
<223>  human PLP1 sense sequence

<400>  109
taagagcgtg cccaaatct                                                   19

<210>  110
<211>  21
<212>  DNA
<213>  Artificial Sequence

<220>
<223>  human PLP1 antisense sequence

<400>  110
attctacgct cccttatgct g                                                21

<210>  111
<211>  19
<212>  DNA
<213>  Artificial Sequence

<220>
<223>  human PLP1 sense sequence

<400>  111
cagcataaga gcgtagaat                                                    19


<210>  112
<211>  21
<212>  DNA
<213>  Artificial Sequence

<220>
<223>  human PLP1 antisense sequence

<400>  112
tggtaataga gagaccagaa t                                                 21


<210>  113
<211>  19
<212>  DNA
<213>  Artificial Sequence

<220>
<223>  human PLP1 sense sequence

<400>  113
attctggtct ctattacca                                                    19


<210>  114
<211>  21
<212>  DNA
<213>  Artificial Sequence

<220>
<223>  human PLP1 antisense sequence

<400>  114
tatagatggc aagaggacca a                                                 21


<210>  115
<211>  19
<212>  DNA
<213>  Artificial Sequence

<220>
<223>  human PLP1 sense sequence

<400>  115
ttggtccttg ccatctata                                                    19


<210>  116
<211>  21
<212>  DNA

<213>  Artificial Sequence

<220>
<223>  human PLP1 antisense sequence

<400>  116
aaaccagtgt agctgcagcc c                                                21


<210>  117
<211>  19
<212>  DNA
<213>  Artificial Sequence

<220>
<223>  human PLP1 sense sequence

<400>  117
gggctgcata cactggttt                                                   19


<210>  118
<211>  23
<212>  DNA
<213>  Artificial Sequence

<220>
<223>  hPLP1-001

<400>  118
ugaccuucca ccuguuuaud tdt                                              23


<210>  119
<211>  23
<212>  DNA
<213>  Artificial Sequence

<220>
<223>  hPLP1-001anti

<400>  119
auaaacaggu ggaaggucad tdt                                              23


<210>  120
<211>  23
<212>  DNA
<213>  Artificial Sequence

<220>
<223>  hPLP1-002

<400>  120
gugccugugu acauuuacud tdt                                              23


<210>  121
<211>  23
<212>  DNA
<213>  Artificial Sequence

<220>

<223>  hPLP1-002anti

<400>  121
aguaaaugua cacaggcacd tdt                                              23


<210>  122
<211>  23
<212>  DNA
<213>  Artificial Sequence

<220>
<223>  hPLP1-003

<400>  122
uugcccaaau cugccuauud tdt                                              23


<210>  123
<211>  23
<212>  DNA
<213>  Artificial Sequence

<220>
<223>  hPLP1-003anti

<400>  123
aauaggcaga uuugggcaad tdt                                              23


<210>  124
<211>  23
<212>  DNA
<213>  Artificial Sequence

<220>
<223>  hPLP1-004

<400>  124
aggaauggag gcucuaauud tdt                                              23


<210>  125
<211>  23
<212>  DNA
<213>  Artificial Sequence

<220>
<223>  hPLP1-004anti

<400>  125
aauuagagcc uccauuccud tdt                                              23


<210>  126
<211>  23
<212>  DNA
<213>  Artificial Sequence

<220>
<223>  hPLP1-005

<400>  126

uggccacugg auuguguuud tdt                                              23


<210>  127
<211>  23
<212>  DNA
<213>  Artificial Sequence

<220>
<223>  hPLP1-005anti

<400>  127
aaacacaauc caguggccad tdt                                              23


<210>  128
<211>  23
<212>  DNA
<213>  Artificial Sequence

<220>
<223>  hPLP1-006

<400>  128
gcgggugugu cauuguuugd tdt                                              23


<210>  129
<211>  23
<212>  DNA
<213>  Artificial Sequence

<220>
<223>  hPLP1-006anti

<400>  129
caaacaauga cacacccgcd tdt                                              23


<210>  130
<211>  23
<212>  DNA
<213>  Artificial Sequence

<220>
<223>  hPLP1-007

<400>  130
gaccuuccac cuguuuauud tdt                                              23


<210>  131
<211>  23
<212>  DNA
<213>  Artificial Sequence

<220>
<223>  hPLP1-007anti

<400>  131
aauaaacagg uggaaggucd tdt                                              23

<210>  132
<211>  23
<212>  DNA
<213>  Artificial Sequence

<220>
<223>  hPLP1-008

<400>  132
acucccuucu ccuugauaad tdt                                              23


<210>  133
<211>  23
<212>  DNA
<213>  Artificial Sequence

<220>
<223>  hPLP1-008anti

<400>  133
uuaucaagga gaagggagud tdt                                              23


<210>  134
<211>  23
<212>  DNA
<213>  Artificial Sequence

<220>
<223>  hPLP1-009

<400>  134
cugcaaguca caaaggaaud tdt                                              23


<210>  135
<211>  23
<212>  DNA
<213>  Artificial Sequence

<220>
<223>  hPLP1-009anti

<400>  135
auuccuuugu gacuugcagd tdt                                              23


<210>  136
<211>  23
<212>  DNA
<213>  Artificial Sequence

<220>
<223>  hPLP1-010

<400>  136
gguccucuug ccaucuauad tdt                                              23


<210>  137
<211>  23
<212>  DNA

<213>  Artificial Sequence

<220>
<223>  hPLP1-010anti

<400>  137
uauagauggc aagaggaccd tdt                                              23


<210>  138
<211>  23
<212>  DNA
<213>  Artificial Sequence

<220>
<223>  hPLP1-011

<400>  138
ccucguuagg gaagagaaad tdt                                              23


<210>  139
<211>  23
<212>  DNA
<213>  Artificial Sequence

<220>
<223>  hPLP1-011anti

<400>  139
uuucucuucc cuaacgaggd tdt                                              23


<210>  140
<211>  23
<212>  DNA
<213>  Artificial Sequence

<220>
<223>  hPLP1-012

<400>  140
ggagaagagg acaaagauad tdt                                              23


<210>  141
<211>  23
<212>  DNA
<213>  Artificial Sequence

<220>
<223>  hPLP1-012anti

<400>  141
uaucuuuguc cucuucuccd tdt                                              23


<210>  142
<211>  23
<212>  DNA
<213>  Artificial Sequence

<220>

<223>  hPLP1-013

<400>  142
ggccaacauc aagcucauud tdt                                               23


<210>  143
<211>  23
<212>  DNA
<213>  Artificial Sequence

<220>
<223>  hPLP1-013anti

<400>  143
aaugagcuug auguuggccd tdt                                               23


<210>  144
<211>  23
<212>  DNA
<213>  Artificial Sequence

<220>
<223>  hPLP1-014

<400>  144
aggccaacau caagcucaud tdt                                               23


<210>  145
<211>  23
<212>  DNA
<213>  Artificial Sequence

<220>
<223>  hPLP1-014anti

<400>  145
augagcuuga uguuggccud tdt                                               23


<210>  146
<211>  23
<212>  DNA
<213>  Artificial Sequence

<220>
<223>  hPLP1-015

<400>  146
cucccuucuc cuugauaacd tdt                                               23


<210>  147
<211>  23
<212>  DNA
<213>  Artificial Sequence

<220>
<223>  hPLP1-015anti

<400>  147

guuaucaagg agaagggagd tdt                                              23


<210>  148
<211>  23
<212>  DNA
<213>  Artificial Sequence

<220>
<223>  hPLP1-016

<400>  148
gccucuuucu ucuuccuuud tdt                                              23


<210>  149
<211>  23
<212>  DNA
<213>  Artificial Sequence

<220>
<223>  hPLP1-016anti

<400>  149
aaaggaagaa gaaagaggcd tdt                                              23


<210>  150
<211>  23
<212>  DNA
<213>  Artificial Sequence

<220>
<223>  hPLP1-017

<400>  150
cugugccugu guacauuuad tdt                                              23


<210>  151
<211>  23
<212>  DNA
<213>  Artificial Sequence

<220>
<223>  hPLP1-017anti

<400>  151
uaaauguaca caggcacagd tdt                                              23


<210>  152
<211>  23
<212>  DNA
<213>  Artificial Sequence

<220>
<223>  hPLP1-018

<400>  152
aaggaaugga ggcucuaaud tdt                                              23

<210>  153
<211>  23
<212>  DNA
<213>  Artificial Sequence

<220>
<223>  hPLP1-018anti

<400>  153
auuagagccu ccauuccuud tdt                                              23


<210>  154
<211>  23
<212>  DNA
<213>  Artificial Sequence

<220>
<223>  hPLP1-019

<400>  154
agggguuugu cagcaucuud tdt                                              23


<210>  155
<211>  23
<212>  DNA
<213>  Artificial Sequence

<220>
<223>  hPLP1-019anti

<400>  155
aagaugcuga caacacccud tdt                                              23


<210>  156
<211>  23
<212>  DNA
<213>  Artificial Sequence

<220>
<223>  hPLP1-020

<400>  156
acagcugagu uccaaaugad tdt                                              23


<210>  157
<211>  23
<212>  DNA
<213>  Artificial Sequence

<220>
<223>  hPLP1-020anti

<400>  157
ucauuuggaa cucagcugud tdt                                              23


<210>  158
<211>  23
<212>  DNA

<213>  Artificial Sequence

<220>
<223>  hPLP1-021

<400>  158
gccacuggau uguguuucud tdt                                              23


<210>  159
<211>  23
<212>  DNA
<213>  Artificial Sequence

<220>
<223>  hPLP1-021anti

<400>  159
agaaacacaa uccaguggcd tdt                                              23


<210>  160
<211>  23
<212>  DNA
<213>  Artificial Sequence

<220>
<223>  hPLP1-022

<400>  160
acaacuuugc cguccuuaad tdt                                              23


<210>  161
<211>  23
<212>  DNA
<213>  Artificial Sequence

<220>
<223>  hPLP1-022anti

<400>  161
uuaaggacgg caaaguugud tdt                                              23


<210>  162
<211>  23
<212>  DNA
<213>  Artificial Sequence

<220>
<223>  hPLP1-023

<400>  162
gcugaugcca gaauguaugd tdt                                              23


<210>  163
<211>  23
<212>  DNA
<213>  Artificial Sequence

<220>

<223>  hPLP1-023anti

<400>  163
cauacauucu ggcaucagcd tdt                                              23


<210>  164
<211>  23
<212>  DNA
<213>  Artificial Sequence

<220>
<223>  hPLP1-024

<400>  164
uugccguccu uaaacucaud tdt                                              23


<210>  165
<211>  23
<212>  DNA
<213>  Artificial Sequence

<220>
<223>  hPLP1-024anti

<400>  165
augaguuuaa ggacggcaad tdt                                              23


<210>  166
<211>  23
<212>  DNA
<213>  Artificial Sequence

<220>
<223>  hPLP1-025

<400>  166
cugccucuuu cuucuuccud tdt                                              23


<210>  167
<211>  23
<212>  DNA
<213>  Artificial Sequence

<220>
<223>  hPLP1-025anti

<400>  167
aggaagaaga aagaggcagd tdt                                              23


<210>  168
<211>  23
<212>  DNA
<213>  Artificial Sequence

<220>
<223>  hPLP1-026

<400>  168

ccacuggauu guguuucuud tdt                                                23

<210>  169
<211>  23
<212>  DNA
<213>  Artificial Sequence

<220>
<223>  hPLP1-026anti

<400>  169
aagaaacaca auccaguggd tdt                                                23

<210>  170
<211>  23
<212>  DNA
<213>  Artificial Sequence

<220>
<223>  hPLP1-027

<400>  170
ggaucagaaa guaauuucud tdt                                                23

<210>  171
<211>  23
<212>  DNA
<213>  Artificial Sequence

<220>
<223>  hPLP1-027anti

<400>  171
agaaauuacu uucugauccd tdt                                                23

<210>  172
<211>  23
<212>  DNA
<213>  Artificial Sequence

<220>
<223>  hPLP1-028

<400>  172
uagccagccg gcuacaauud tdt                                                23

<210>  173
<211>  23
<212>  DNA
<213>  Artificial Sequence

<220>
<223>  hPLP1-028anti

<400>  173
aauuguagcc ggcuggcuad tdt                                                23

<210>  174
<211>  23
<212>  DNA
<213>  Artificial Sequence

<220>
<223>  hPLP1-029

<400>  174
agcguagaau cuguguagad tdt                                              23


<210>  175
<211>  23
<212>  DNA
<213>  Artificial Sequence

<220>
<223>  hPLP1-029anti

<400>  175
ucuacacaga uucuacgcud tdt                                              23


<210>  176
<211>  23
<212>  DNA
<213>  Artificial Sequence

<220>
<223>  hPLP1-030

<400>  176
ucccuucucc uugauaacad tdt                                              23


<210>  177
<211>  23
<212>  DNA
<213>  Artificial Sequence

<220>
<223>  hPLP1-030anti

<400>  177
uguuaucaag gagaagggad tdt                                              23


<210>  178
<211>  23
<212>  DNA
<213>  Artificial Sequence

<220>
<223>  hPLP1-031

<400>  178
uuccaaggag cugagaauad tdt                                              23


<210>  179
<211>  23
<212>  DNA

<213>  Artificial Sequence

<220>
<223>  hPLP1-031anti

<400>  179
uauucucagc uccuuggaad tdt                                              23


<210>  180
<211>  23
<212>  DNA
<213>  Artificial Sequence

<220>
<223>  hPLP1-032

<400>  180
ugccagaaug uauggguuud tdt                                              23


<210>  181
<211>  23
<212>  DNA
<213>  Artificial Sequence

<220>
<223>  hPLP1-032anti

<400>  181
aacaccauac auucuggcad tdt                                              23


<210>  182
<211>  23
<212>  DNA
<213>  Artificial Sequence

<220>
<223>  hPLP1-033

<400>  182
augccuucca guaugucaud tdt                                              23


<210>  183
<211>  23
<212>  DNA
<213>  Artificial Sequence

<220>
<223>  hPLP1-033anti

<400>  183
augacauacu ggaaggcaud tdt                                              23


<210>  184
<211>  23
<212>  DNA
<213>  Artificial Sequence

<220>

<223>  hPLP1-034

<400>  184
caacuuugcc guccuuaaad tdt                                              23


<210>  185
<211>  23
<212>  DNA
<213>  Artificial Sequence

<220>
<223>  hPLP1-034anti

<400>  185
uuuaaggacg gcaaaguugd tdt                                              23


<210>  186
<211>  23
<212>  DNA
<213>  Artificial Sequence

<220>
<223>  hPLP1-035

<400>  186
ccaacaucaa gcucauucud tdt                                              23


<210>  187
<211>  23
<212>  DNA
<213>  Artificial Sequence

<220>
<223>  hPLP1-035anti

<400>  187
agaaugagcu ugauguuggd tdt                                              23


<210>  188
<211>  23
<212>  DNA
<213>  Artificial Sequence

<220>
<223>  hPLP1-036

<400>  188
uggccuuaca ccucguuagd tdt                                              23


<210>  189
<211>  23
<212>  DNA
<213>  Artificial Sequence

<220>
<223>  hPLP1-036anti

<400>  189

cuaacgaggu guaaggccad tdt                                              23


<210>  190
<211>  23
<212>  DNA
<213>  Artificial Sequence

<220>
<223>  hPLP1-037

<400>  190
aggacauccc gacaaguuud tdt                                              23


<210>  191
<211>  23
<212>  DNA
<213>  Artificial Sequence

<220>
<223>  hPLP1-037anti

<400>  191
aaacuugucg ggauguccud tdt                                              23


<210>  192
<211>  23
<212>  DNA
<213>  Artificial Sequence

<220>
<223>  hPLP1-038

<400>  192
gucagagucc caaagacaud tdt                                              23


<210>  193
<211>  23
<212>  DNA
<213>  Artificial Sequence

<220>
<223>  hPLP1-038anti

<400>  193
augucuuugg gacucugacd tdt                                              23


<210>  194
<211>  23
<212>  DNA
<213>  Artificial Sequence

<220>
<223>  hPLP1-039

<400>  194
uugcaggaga agaggacaad tdt                                              23

<210>  195
<211>  23
<212>  DNA
<213>  Artificial Sequence

<220>
<223>  hPLP1-039anti

<400>  195
uuguccucuu cuccugcaad tdt                                              23


<210>  196
<211>  23
<212>  DNA
<213>  Artificial Sequence

<220>
<223>  hPLP1-040

<400>  196
gguagacaca ggauagauad tdt                                              23


<210>  197
<211>  23
<212>  DNA
<213>  Artificial Sequence

<220>
<223>  hPLP1-040anti

<400>  197
uaucuauccu gugucuaccd tdt                                              23


<210>  198
<211>  23
<212>  DNA
<213>  Artificial Sequence

<220>
<223>  hPLP1-041

<400>  198
ucagccaacc uuacuuacad tdt                                              23


<210>  199
<211>  23
<212>  DNA
<213>  Artificial Sequence

<220>
<223>  hPLP1-041anti

<400>  199
uguaaguaag guuggcugad tdt                                              23


<210>  200
<211>  23
<212>  DNA

<213>  Artificial Sequence

<220>
<223>  hPLP1-042

<400>  200
auggaacugc cucuuucuud tdt                                              23


<210>  201
<211>  23
<212>  DNA
<213>  Artificial Sequence

<220>
<223>  hPLP1-042anti

<400>  201
aagaaagagg caguuccaud tdt                                              23


<210>  202
<211>  23
<212>  DNA
<213>  Artificial Sequence

<220>
<223>  hPLP1-043

<400>  202
acucagccaa ccuuacuuad tdt                                              23


<210>  203
<211>  23
<212>  DNA
<213>  Artificial Sequence

<220>
<223>  hPLP1-043anti

<400>  203
uaaguaaggu uggcugagud tdt                                              23


<210>  204
<211>  23
<212>  DNA
<213>  Artificial Sequence

<220>
<223>  hPLP1-044

<400>  204
cuuccacuga uggaaacaad tdt                                              23


<210>  205
<211>  23
<212>  DNA
<213>  Artificial Sequence

<220>

<223>  hPLP1-044anti

<400>  205
uuguuuccau caguggaagd tdt                                              23


<210>  206
<211>  23
<212>  DNA
<213>  Artificial Sequence

<220>
<223>  hPLP1-045

<400>  206
augaccuucc accuguuuad tdt                                              23


<210>  207
<211>  23
<212>  DNA
<213>  Artificial Sequence

<220>
<223>  hPLP1-045anti

<400>  207
uaaacaggug gaaggucaud tdt                                              23


<210>  208
<211>  23
<212>  DNA
<213>  Artificial Sequence

<220>
<223>  hPLP1-046

<400>  208
ucuccugagg aucagaaagd tdt                                              23


<210>  209
<211>  23
<212>  DNA
<213>  Artificial Sequence

<220>
<223>  hPLP1-046anti

<400>  209
cuuucugauc cucaggagad tdt                                              23


<210>  210
<211>  23
<212>  DNA
<213>  Artificial Sequence

<220>
<223>  hPLP1-047

<400>  210

gaggaucaga aaguaauuud tdt                                              23

<210>  211
<211>  23
<212>  DNA
<213>  Artificial Sequence

<220>
<223>  hPLP1-047anti

<400>  211
aaauuacuuu cugauccucd tdt                                              23

<210>  212
<211>  23
<212>  DNA
<213>  Artificial Sequence

<220>
<223>  hPLP1-048

<400>  212
ggaucuuuca cccacagaad tdt                                              23

<210>  213
<211>  23
<212>  DNA
<213>  Artificial Sequence

<220>
<223>  hPLP1-048anti

<400>  213
uucugugggu gaaagauccd tdt                                              23

<210>  214
<211>  23
<212>  DNA
<213>  Artificial Sequence

<220>
<223>  hPLP1-049

<400>  214
cugaggauca gaaaguaaud tdt                                              23

<210>  215
<211>  23
<212>  DNA
<213>  Artificial Sequence

<220>
<223>  hPLP1-049anti

<400>  215
auuacuuucu gauccucagd tdt                                              23

<210>  216
<211>  23
<212>  DNA
<213>  Artificial Sequence

<220>
<223>  hPLP1-050

<400>  216
cugguagaca caggauagad tdt                                              23


<210>  217
<211>  23
<212>  DNA
<213>  Artificial Sequence

<220>
<223>  hPLP1-050anti

<400>  217
ucuauccugu gucuaccagd tdt                                              23


<210>  218
<211>  23
<212>  DNA
<213>  Artificial Sequence

<220>
<223>  hPLP1-051

<400>  218
gcaguugcug guggcuaaud tdt                                              23


<210>  219
<211>  23
<212>  DNA
<213>  Artificial Sequence

<220>
<223>  hPLP1-051anti

<400>  219
auuagccacc agcaacugcd tdt                                              23


<210>  220
<211>  23
<212>  DNA
<213>  Artificial Sequence

<220>
<223>  hPLP1-052

<400>  220
aagacauggg cuuguuagad tdt                                              23


<210>  221
<211>  23
<212>  DNA

<213>  Artificial Sequence

<220>
<223>  hPLP1-052anti

<400>  221
ucuaacaagc ccaugucuud tdt                                              23


<210>  222
<211>  23
<212>  DNA
<213>  Artificial Sequence

<220>
<223>  hPLP1-053

<400>  222
uugcugccac uuacaacuud tdt                                              23


<210>  223
<211>  23
<212>  DNA
<213>  Artificial Sequence

<220>
<223>  hPLP1-053anti

<400>  223
aaguuguaag uggcagcaad tdt                                              23


<210>  224
<211>  23
<212>  DNA
<213>  Artificial Sequence

<220>
<223>  hPLP1-054

<400>  224
ucguuaggga agagaaacad tdt                                              23


<210>  225
<211>  23
<212>  DNA
<213>  Artificial Sequence

<220>
<223>  hPLP1-054anti

<400>  225
uguuucucuu cccuaacgad tdt                                              23


<210>  226
<211>  23
<212>  DNA
<213>  Artificial Sequence

<220>

<223>  hPLP1-055

<400>  226
uccacugaug gaaacaaagd tdt                                              23


<210>  227
<211>  23
<212>  DNA
<213>  Artificial Sequence

<220>
<223>  hPLP1-055anti

<400>  227
cuuuguuucc aucaguggad tdt                                              23


<210>  228
<211>  23
<212>  DNA
<213>  Artificial Sequence

<220>
<223>  hPLP1-056

<400>  228
agagcguuug cccaaaucud tdt                                              23


<210>  229
<211>  23
<212>  DNA
<213>  Artificial Sequence

<220>
<223>  hPLP1-056anti

<400>  229
agauuugggc aaacgcucud tdt                                              23


<210>  230
<211>  23
<212>  DNA
<213>  Artificial Sequence

<220>
<223>  hPLP1-057

<400>  230
ugauugcugc cacuuacaad tdt                                              23


<210>  231
<211>  23
<212>  DNA
<213>  Artificial Sequence

<220>
<223>  hPLP1-057anti

<400>  231

uuguaagugg cagcaaucad tdt                                                23

<210>  232
<211>  23
<212>  DNA
<213>  Artificial Sequence

<220>
<223>  hPLP1-058

<400>  232
ggagcguaga aucuguguad tdt                                                23

<210>  233
<211>  23
<212>  DNA
<213>  Artificial Sequence

<220>
<223>  hPLP1-058anti

<400>  233
uacacagauu cuacgcuccd tdt                                                23

<210>  234
<211>  22
<212>  DNA
<213>  Artificial Sequence

<220>
<223>  hPLP1-059

<400>  234
ugaggaucag aaaguaauud td                                                 22

<210>  235
<211>  23
<212>  DNA
<213>  Artificial Sequence

<220>
<223>  hPLP1-059anti

<400>  235
aauuacuuuc ugauccucad tdt                                                23

<210>  236
<211>  23
<212>  DNA
<213>  Artificial Sequence

<220>
<223>  hPLP1-060

<400>  236
gguguuguca gcaucuucud tdt                                                23

<210>  237
<211>  23
<212>  DNA
<213>  Artificial Sequence

<220>
<223>  hPLP1-060anti

<400>  237
agaagaugcu gacaacaccd tdt                                              23


<210>  238
<211>  23
<212>  DNA
<213>  Artificial Sequence

<220>
<223>  hPLP1-061

<400>  238
gucaaagcaa ggaucuuucd tdt                                              23


<210>  239
<211>  23
<212>  DNA
<213>  Artificial Sequence

<220>
<223>  hPLP1-061anti

<400>  239
gaaagauccu ugcuuugacd tdt                                              23


<210>  240
<211>  23
<212>  DNA
<213>  Artificial Sequence

<220>
<223>  hPLP1-062

<400>  240
agaucuuugg cgacuacaad tdt                                              23


<210>  241
<211>  23
<212>  DNA
<213>  Artificial Sequence

<220>
<223>  hPLP1-062anti

<400>  241
uuguagucgc caaagaucud tdt                                              23


<210>  242
<211>  23
<212>  DNA

<213>  Artificial Sequence

<220>
<223>  hPLP1-063

<400>  242
ugcaggagaa gaggacaaad tdt                                              23


<210>  243
<211>  23
<212>  DNA
<213>  Artificial Sequence

<220>
<223>  hPLP1-063anti

<400>  243
uuuguccucu ucuccugcad tdt                                              23


<210>  244
<211>  23
<212>  DNA
<213>  Artificial Sequence

<220>
<223>  hPLP1-064

<400>  244
ugguggcuaa ugguguaacd tdt                                              23


<210>  245
<211>  23
<212>  DNA
<213>  Artificial Sequence

<220>
<223>  hPLP1-064anti

<400>  245
guuacaccau uagccaccad tdt                                              23


<210>  246
<211>  23
<212>  DNA
<213>  Artificial Sequence

<220>
<223>  hPLP1-065

<400>  246
agcuaauuga gaccuauuud tdt                                              23


<210>  247
<211>  23
<212>  DNA
<213>  Artificial Sequence

<220>

<223>  hPLP1-065anti

<400>  247
aaauaggucu caauuagcud tdt                                              23


<210>  248
<211>  23
<212>  DNA
<213>  Artificial Sequence

<220>
<223>  hPLP1-066

<400>  248
gugcugaugc cagaauguad tdt                                              23


<210>  249
<211>  23
<212>  DNA
<213>  Artificial Sequence

<220>
<223>  hPLP1-066anti

<400>  249
uacauucugg caucagcacd tdt                                              23


<210>  250
<211>  23
<212>  DNA
<213>  Artificial Sequence

<220>
<223>  hPLP1-067

<400>  250
uuuccaagga gcugagaaud tdt                                              23


<210>  251
<211>  23
<212>  DNA
<213>  Artificial Sequence

<220>
<223>  hPLP1-067anti

<400>  251
auucucagcu ccuuggaaad tdt                                              23


<210>  252
<211>  23
<212>  DNA
<213>  Artificial Sequence

<220>
<223>  hPLP1-068

<400>  252

ugugccugug uacauuuacd tdt                                                23

<210>  253
<211>  23
<212>  DNA
<213>  Artificial Sequence

<220>
<223>  hPLP1-068anti

<400>  253
guaaauguac acaggcacad tdt                                                23

<210>  254
<211>  23
<212>  DNA
<213>  Artificial Sequence

<220>
<223>  hPLP1-069

<400>  254
cagguacaga gaaggaaugd tdt                                                23

<210>  255
<211>  23
<212>  DNA
<213>  Artificial Sequence

<220>
<223>  hPLP1-069anti

<400>  255
cauuccuucu cuguaccugd tdt                                                23

<210>  256
<211>  23
<212>  DNA
<213>  Artificial Sequence

<220>
<223>  hPLP1-070

<400>  256
gcauaaggga gcguagaaud tdt                                                23

<210>  257
<211>  23
<212>  DNA
<213>  Artificial Sequence

<220>
<223>  hPLP1-070anti

<400>  257
auucuacgcu cccuuaugcd tdt                                                23

<210>  258
<211>  23
<212>  DNA
<213>  Artificial Sequence

<220>
<223>  hPLP1-071

<400>  258
ucagguacag agaaggaaud tdt                                              23


<210>  259
<211>  23
<212>  DNA
<213>  Artificial Sequence

<220>
<223>  hPLP1-071anti

<400>  259
auuccuucuc uguaccugad tdt                                              23


<210>  260
<211>  23
<212>  DNA
<213>  Artificial Sequence

<220>
<223>  hPLP1-072

<400>  260
gcugcagcua cacugguuud tdt                                              23


<210>  261
<211>  23
<212>  DNA
<213>  Artificial Sequence

<220>
<223>  hPLP1-072anti

<400>  261
aaaccagugu agcugcagcd tdt                                              23


<210>  262
<211>  23
<212>  DNA
<213>  Artificial Sequence

<220>
<223>  hPLP1-073

<400>  262
ucucccaugg aaugcuuucd tdt                                              23


<210>  263
<211>  23
<212>  DNA

<213>  Artificial Sequence

<220>
<223>  hPLP1-073anti

<400>  263
gaaagcauuc caugggagad tdt                                              23


<210>  264
<211>  23
<212>  DNA
<213>  Artificial Sequence

<220>
<223>  hPLP1-074

<400>  264
ugccuuccag uaugucaucd tdt                                              23


<210>  265
<211>  23
<212>  DNA
<213>  Artificial Sequence

<220>
<223>  hPLP1-074anti

<400>  265
gaugacauac uggaaggcad tdt                                              23


<210>  266
<211>  23
<212>  DNA
<213>  Artificial Sequence

<220>
<223>  hPLP1-075

<400>  266
gcugccacuu acaacuuugd tdt                                              23


<210>  267
<211>  23
<212>  DNA
<213>  Artificial Sequence

<220>
<223>  hPLP1-075anti

<400>  267
caaaguugua aguggcagcd tdt                                              23


<210>  268
<211>  23
<212>  DNA
<213>  Artificial Sequence

<220>

<223>  hPLP1-076

<400>  268
acuuugccgu ccuuaaacud tdt                                               23


<210>  269
<211>  23
<212>  DNA
<213>  Artificial Sequence

<220>
<223>  hPLP1-076anti

<400>  269
aguuuaagga cggcaaagud tdt                                               23


<210>  270
<211>  23
<212>  DNA
<213>  Artificial Sequence

<220>
<223>  hPLP1-077

<400>  270
accuuacuua cagcauaagd tdt                                               23


<210>  271
<211>  23
<212>  DNA
<213>  Artificial Sequence

<220>
<223>  hPLP1-077anti

<400>  271
cuuaugcugu aaguaaggud tdt                                               23


<210>  272
<211>  23
<212>  DNA
<213>  Artificial Sequence

<220>
<223>  hPLP1-078

<400>  272
uggacuacug aagcccuaad tdt                                               23


<210>  273
<211>  23
<212>  DNA
<213>  Artificial Sequence

<220>
<223>  hPLP1-078anti

<400>  273

uuagggcuuc aguaguccad tdt                                              23

<210>  274
<211>  23
<212>  DNA
<213>  Artificial Sequence

<220>
<223>  hPLP1-079

<400>  274
cuuccaguau gucaucuaud tdt                                              23

<210>  275
<211>  23
<212>  DNA
<213>  Artificial Sequence

<220>
<223>  hPLP1-079anti

<400>  275
auagaugaca uacuggaagd tdt                                              23

<210>  276
<211>  23
<212>  DNA
<213>  Artificial Sequence

<220>
<223>  hPLP1-080

<400>  276
ucuggucucu cuauuaccad tdt                                              23

<210>  277
<211>  23
<212>  DNA
<213>  Artificial Sequence

<220>
<223>  hPLP1-080anti

<400>  277
ugguaauaga gagaccagad tdt                                              23

<210>  278
<211>  23
<212>  DNA
<213>  Artificial Sequence

<220>
<223>  hPLP1-081

<400>  278
gucccaaaga caugggcuud tdt                                              23

<210>  279
<211>  23
<212>  DNA
<213>  Artificial Sequence

<220>
<223>  hPLP1-081anti

<400>  279
aagcccaugu cuuugggacd tdt                                              23


<210>  280
<211>  21
<212>  DNA
<213>  Artificial Sequence

<220>
<223>  human PLP1 forward primer

<400>  280
gctccaacct tctgtccatc t                                                21


<210>  281
<211>  20
<212>  DNA
<213>  Artificial Sequence

<220>
<223>  human PLP1 reverse primer

<400>  281
acggcaaagt tgtaagtggc                                                  20


<210>  282
<211>  24
<212>  DNA
<213>  Artificial Sequence

<220>
<223>  human beta-action forward primer

<400>  282
gacaggatgc agaaggagat tact                                             24


<210>  283
<211>  22
<212>  DNA
<213>  Artificial Sequence

<220>
<223>  human beta-actin reverse primer

<400>  283
tgatccacat ctgctggaag gt                                               22

## Description

### Technical Field

The present invention relates to an oligodendrocyte-specific promoter, in particular, to an oligodendrocyte-specific promoter comprising a nucleic acid having a sequence identity of at least 90% to a nucleotide sequence set forth in SEQ ID NO: 1. The present invention also relates to an miRNA specific to a PLP1 gene. Further, the present invention relates to a vector comprising the promoter and/or the miRNA, and a pharmaceutical composition comprising the vector.

### Background Art

Hypomyelinating leukodystrophies is a collective term for brain diseases in children caused by poor development of the brain white matter, and currently 11 diseases have been known. A representative disease among them is Pelizaeus-Merzbacher disease (PMD). PMD is a rare intractable disease that is characterized in that myelination of the central nervous system is disordered and causes developmental failure of motor functions and neurological symptoms immediately after birth. The estimated incidence in Japan is 1.45 individuals per 100000 newborn male infants. Currently, there is no fundamental cure.

Myelin is generated by oligodendrocytes (oligodendroglia), one of glial cells, in the brain, and has a multilayer structure of insulating phospholipid present around the axon of a neuron. A primary protein constituting myelin is proteolipid protein (PLP), Myelin basic protein (MBP), human 2',3'-cyclic nucleotide phosphodiesterase (CNP), and so on are also known as other constituents. PMD is caused by abnormalities of a gene for proteolipid protein (PLP1 gene). The most frequent mutation among such abnormalities is PLP1 duplications (about 60%). PLP1 duplication leads to overexpression of the PLP1 gene, thereby normalization of its expression level is expected to provide therapeutic effect.

Non Patent Literature 1 disclosed expression of adeno-associated virus (AAV)-mediated green fluorescent protein (GFP) driven by myelin basic protein (MBP) promoter or glial fibrillary acidic protein (GFAP) promoter in the brain of mice under development, and reported that the expression of GFAP promoter-driven GFP was highly specific to astrocytes after injection of a vector into the brain of neonatal mice and adult mice. Non Patent Literature 1 also reported the selectivity of the MBP promoter for oligodendrocytes was poor after AAV delivery to right after birth, but was superior after injection of a vector 10 days after birth. This document suggested that direct injection of AAV driven by a cell type-specific promoter into the developed brain after birth generates targeted long-term transgene expression in glial cells.

Non Patent Literature 2 disclosed development of target gene therapy for oligodendrocytes in Pelizaeus-Merzbacher-like disease, and reported that a GJC2/Cx47 gene was inserted into the downstream of a myelin basic protein promoter, and administered to 10-day-old mice via single intracerebral injection into the internal capsule with an adeno-associated virus (AAV.MBP.Cx47myc) vector.

Each of Non Patent Literatures 1 and 2 discloses gene therapy and a promoter therefor with use of an AAV vector, but did not describe at all gene therapy for abnormality of the PLP1 gene and a promoter or miRNA therefor.

### Citation List

### Non Patent Literature

Non Patent Literature 1: von Jonquieres G et al., Glial promoter selectivity following AAV-delivery to the immature brain. PLoS One. 2013 Jun 14; 8(6) : e65646.
Non Patent Literature 2: Georgiou E et al., Gene therapy targeting oligodendrocytes provides therapeutic benefit in a leukodystrophy model. Brain. 2017 Mar 1; 140(3): 599-616.

### Summary of Invention

### Technical Problem

An object of the present invention is to provide a vector capable of oligodendrocyte-specifically suppressing expression of the PLP1 gene for treating PMD caused by abnormality of the PLP1 gene, and a promoter and miRNA therefor, and a pharmaceutical composition comprising the vector.

### Solution to Problem

The present inventors found that a human 2',3'-cyclic nucleotide phosphodiesterase (CNP) promoter having a sequence set forth in SEQ ID NO: 1 can drive gene expression in an oligodendrocyte-specific and highly efficient manner, and that expression of the PLP1 gene is successfully suppressed with an AAV vector comprising the promoter and a PLP1 gene-specific miRNA operably linked to the downstream of the promoter, thus completing the present invention.

Specifically, the present invention relates to the followings.
[1] An oligodendrocyte-specific promoter comprising a nucleic acid having a sequence identity of at least 90% to a nucleotide sequence set forth in SEQ ID NO: 1.
[2] The promoter according to [1], comprising a nucleic acid having the nucleotide sequence set forth in SEQ ID NO: 1.
[3] An oligodendrocyte-specific vector comprising the promoter according to [1] or [2].
[4] The vector according to [3], further comprising an miRNA sequence specific to a human PLP1 gene operably linked to the promoter.
[5] An miRNA comprising a pair of nucleotide sequences consisting of an antisense sequence and a sense sequence and being specific to a PLP1 gene, wherein the pair of nucleotide sequences is selected from the group consisting of pairs of an antisense sequence set forth in a left column of Table 1 and a sense sequence set forth in the just right of the antisense sequence in a right column of the table.
[6] The miRNA according to [5], comprising a nucleotide sequence selected from the group consisting of SEQ ID NOs: 2 to 7 and 24 to 51.
[7] The miRNA according to [5], comprising a nucleotide sequence selected from the group consisting of SEQ ID NOs: 2 to 5, 7, 24, 26, 29 to 32, 35, 36, 42, and 51.
[8] The miRNA according to [5], comprising a nucleotide sequence selected from the group consisting of SEQ ID NOs: 2, 29, 31, and 32.
[9] A vector comprising a sequence of the miRNA according to any one of [5] to [8].
[10] The vector according to [9], wherein the sequence of the miRNA is operably linked to an oligodendrocyte-specific promoter.
[11] The vector according to [10], wherein the oligodendrocyte-specific promoter is the promoter according to [1] or [2].
[12] The vector according to any one of [3], [4], and [9] to [11], wherein the vector is an adeno-associated virus (AAV) vector.
[13] A pharmaceutical composition comprising the vector according to any one of [3], [4], and [9] to [12].
[14] The pharmaceutical composition according to [13], wherein the pharmaceutical composition is a pharmaceutical composition for treating a disease associated with abnormality in a PLP1 gene.
[15] The pharmaceutical composition according to [14], wherein the disease is Pelizaeus-Merzbacher disease, spastic paraplegia type 2, or multiple sclerosis.
[16] The pharmaceutical composition according to [14], wherein the disease is Pelizaeus-Merzbacher disease.
[17] The pharmaceutical composition according to [13], wherein the pharmaceutical composition is a PLP1 gene expression suppressor which suppresses expression of the PLP1 gene.
[18] A method for treating a disease associated with abnormality of a PLP1 gene, the method comprising administering an effective amount of the vector according to any one of [3], [4], and [9] to [12] to a patient in need of treatment.
[19] The method according to [18], wherein the disease is Pelizaeus-Merzbacher disease, spastic paraplegia type 2, or multiple sclerosis.
[20] The method according to [18], wherein the disease is Pelizaeus-Merzbacher disease.
[21] A method for suppressing expression of a PLP1 gene, the method comprising administering an effective amount of the vector according to any one of [3], [4], and [9] to [12] to a patient in need of treatment.
[22] The vector according to any one of [3], [4], and [9] to [12] for use in treatment of a disease associated with abnormality of a PLP1 gene.
[23] The vector according to [22], wherein the disease is Pelizaeus-Merzbacher disease, spastic paraplegia type 2, or multiple sclerosis.
[24] The vector according to [22], wherein the disease is Pelizaeus-Merzbacher disease.

### Advantageous Effects of Invention

The AAV vector comprising the promoter of the present invention is capable of expressing a gene in an oligodendrocyte-specific and highly efficient manner, and moreover successfully suppresses expression of the PLP1 gene through inclusion of an miRNA sequence specific to the human PLP1 gene operably linked to the promoter, and thus can serve as a therapeutic drug for PMD caused by PLP1 duplication, etc.

### Brief Description of Drawings

[Figure 1] Figure 1 shows a schematic diagram of an AAV vector of Example 1.
[Figure 2] Figure 2 shows photographs representing results of immunofluorescence staining on specificity of AAV-expressing cells in Example 2.
[Figure 3] Figure 3 shows a graph representing results of site-by-site measurement of suppression of expression (knockdown) in terms of PLP1 gene expression levels on the basis of fluorescence intensity generated by PLP1 immunofluorescence staining in Example 3.
[Figure 4] Figure 4 shows graphs representing results of measurement of suppression of expression (knockdown) in terms of PLP1 gene expression levels through quantitative PCR in Example 4. (A) and (B) show relative expression levels of the PLP1 gene and relative expression levels of the Olig2 gene, respectively.
[Figure 5] Figure 5 shows photographs representing results of histological analysis through immunostaining after administration to PLP1-Tg mice in Example 5.
[Figure 6] Figure 6 shows photographs representing results of micromorphological analysis with an electron microscope after administration to PLP1-Tg mice in Example 5.
[Figure 7] Figure 7 shows a graph representing life-prolonging effect provided by administration to PLP1-Tg mice in Example 5.
[Figure 8] Figure 8 shows a graph representing body weight gain effect provided by administration to PLP1-Tg mice in Example 5.
[Figure 9] Figure 9 shows a graph representing results of measurement of suppression of expression (knockdown) in terms of PLP1 gene expression levels through quantitative PCR in Example 6.
[Figure 10] Figure 10 shows a graph representing results of measurement of suppression of expression (knockdown) in terms of PLP1 gene expression levels through quantitative PCR in Example 7.
[Figure 11] Figure 11 shows a graph representing results of measurement of suppression of expression (knockdown) in terms of PLP1 gene expression levels through quantitative PCR in Example 8.
[Figure 12] Figure 12 shows a graph representing results of measurement of suppression of expression (knockdown) in terms of PLP1 gene expression levels through quantitative PCR in Example 9.
[Figure 13] Figure 13 shows a graph representing results of site-by-site measurement of suppression of expression (knockdown) in terms of PLP1 gene expression levels on the basis of fluorescence intensity generated by PLP1 immunofluorescence staining in Example 10.

### Description of Embodiments

### [Promoter]

The promoter of the present embodiment is an oligodendrocyte-specific promoter comprising a nucleic acid having a sequence identity of at least 90%, preferably having a sequence identity of at least 95%, more preferably having a sequence identity of at least 98%, to a nucleotide sequence set forth in SEQ ID NO: 1, particularly preferably comprising a nucleic acid having the nucleotide sequence set forth in SEQ ID NO: 1. In addition to the nucleic acid having a sequence identity of at least 90% to the nucleotide sequence set forth in SEQ ID NO: 1, the promoter may include in an end of the promoter nucleotide sequences of sites for cloning into vectors such as restriction enzyme sites and an att sequence for Gateway cloning.

The nucleic acid having the nucleotide sequence set forth in SEQ ID NO: 1 is a human CNP promoter, and derived from chr17:40,118,309-40,120,101 of the human genome reference sequence (GRCh37/hg19). The human CNP gene is a gene expressed specifically in oligodendrocytes in the brain, and the specificity is inferred to be determined by the promoter sequence. The present inventors found that the human CNP promoter sequence set forth in SEQ ID NO: 1 is capable of oligodendrocyte-specificcally expressing genes other than the CNP gene that are disposed in the downstream of the promoter.

The promoter of the present embodiment is a promoter that specifically operates in oligodendrocytes, whereby genes operably linked to the downstream of the promoter are expressed specifically in oligodendrocytes. The promoter of the present embodiment can be obtained through a gene engineering approach. For example, a gene for the human CNP promoter is obtained from a human genome, and some nucleotides are deleted, inserted, or substituted to obtain a promoter having a desired nucleotide sequence. Alternatively, a promoter having a desired nucleotide sequence can be obtained through partial or complete synthesis.

### [miRNA]

An miRNA typically includes an antisense sequence and a sense sequence, and can form a hairpin structure, thereby processed into a mature miRNA. The antisense strand in the mature miRNA bonds to a target mRNA to decompose the mRNA or cause translational repression, thereby suppressing expression of the target gene. The miRNA of the present embodiment is an miRNA specific to the PLP1 gene, in particular, to the human PLP1 gene, includes an antisense sequence and a sense sequence, and is capable of suppressing expression of the PLP1 gene.

It is preferable that a 5' flanking sequence, an antisense sequence, a stem-loop sequence, a sense sequence, and a 3' flanking sequence be disposed in the order from the 5' side in the miRNA of the present embodiment. The configuration is required to be such that the 5' flanking sequence has a length of 25 to 100 nucleotides, the stem-loop sequence has a length of 4 to 30 nucleotides, and the 3' flanking sequence has a length of 25 to 100 nucleotides.

Each flanking sequence needs to be a flanking sequence known to be generally expressed in oligodendrocytes, and, for example, a 5' flanking sequence and 3' flanking sequence that are known to be sequences derived from miR155 can be used. In addition to the 5' flanking sequence and 3' flanking sequence derived from miR155, a 5' flanking sequence and 3' flanking sequence of any of miR-138, miR-219, miR-9, miR-23a, miR-388, and miR-297c, which are known to be expressed in oligodendrocytes, can be used.

The miRNA sequence specific to the human PLP1 gene can be designed, for example, by using software such as BLOCK-iT (TM) RNAi Designer (Thermo Fisher Scientific, USA), and, for example, the following human PLP1-miRNA candidate nucleotide sequences can be used.
Human PLP1-miRNA candidate nucleotide sequence 1 (SEQ ID NO: 2): 
5'-
AAAGGAAGAAGAAAGAGGCAGGTTTTGGCCACTGACTGACC
TGCCTCTCTTCTTCCTTT -3'
Human PLP1-miRNA candidate nucleotide sequence 2 (SEQ ID NO: 3): 
5'-
AACACCAGGAGCCACACAACGGTTTTGGCCACTGACTGACCG
TTGTGTCTCCTGGTGTT -3'
Human PLP1-miRNA candidate nucleotide sequence 3 (SEQ ID NO: 4): 
5'-
TTCCATGGGAGAACACCATACGTTTTGGCCACTGACTGACGTA
TGGTGCTCCCATGGAA -3'
Human PLP1-miRNA candidate nucleotide sequence 4 (SEQ ID NO: 5): 
5'-
TGAGCAGGGAAACCAGTGTAGGTTTTGGCCACTGACTGACCT
ACACTGTTCCCTGCTCA-3'
Human PLP1-miRNA candidate nucleotide sequence 5 (SEQ ID NO: 6): 
5'-
AGGGCTTTCTGATTGACAGCCGTTTTGGCCACTGACTGACGG
CTGTCACAGAAAGCCCT -3'
Human PLP1-miRNA candidate nucleotide sequence 6 (SEQ ID NO: 7): 
5'-
ACCCCAAAGAAACACAATCCAGTTTTGGCCACTGACTGACtgg
attgtttctttggggt -3'
Human PLP1-miRNA candidate nucleotide sequence 7 (SEQ ID NO: 24): 
5'-
ACAAATGCAGCAATAAACAGGGTTTTGGCCACTGACTGACCC
TGTTTAGCTGCATTTGT -3'
Human PLP1-miRNA candidate nucleotide sequence 8 (SEQ ID NO: 25): 
5'-
AATAGACTGGCAGGTGGTCCAGTTTTGGCCACTGACTGACTG
GACCACGCCAGTCTATT -3'
Human PLP1-miRNA candidate nucleotide sequence 9 (SEQ ID NO: 26): 
5'-
AAAGAATGAGCTTGATGTTGGGTTTTGGCCACTGACTGACCC
AACATCGCTCATTCTTT -3'
Human PLP1-miRNA candidate nucleotide sequence 10 (SEQ ID NO: 27): 5'- 
AGATACTCATAGTCTTGGTAGGTTTTGGCCACTGACTGACCTA
CCAAGTATGAGTATCT -3'
Human PLP1-miRNA candidate nucleotide sequence 11 (SEQ ID NO: 28):

5'-
AAGCCCATGTCTTTGGGACTCGTTTTGGCCACTGACTGACGA
GTCCCAGACATGGGCTT -3'

Alternatively, the miRNA sequence specific to the human PLP1 gene can be designed in such a manner that artificial siRNAs are designed as described in Examples, and an optimum sequence is selected from the siRNAs to design the miRNA.

The miRNA of the present embodiment comprises a pair of nucleotide sequences consisting of an antisense sequence and a sense sequence, and the pair of nucleotide sequences may be selected from the group consisting of pairs of an antisense sequence set forth in a left column of Table 1 and a sense sequence set forth in the just right of the antisense sequence in a right column of the table.

### [Table 1]

**Table 1. Antisense and sense sequences for miRNA designed for human PLP1 gene**

| Antisense sequence | Sense sequence |
|---|---|
| AAAGGAAGAAGAAAGAGGCAG (SEQ ID NO: 52) | CTGCCTCTCTTCTTCCTTT (SEQ ID NO: 53) |
| AACACCAGGAGCCACACAACG (SEQ ID NO: 54) | CGTTGTGTCTCCTGGTGTT (SEQ ID NO: 55) |
| TTCCATGGGAGAACACCATAC (SEQ ID NO: 56) | GTATGGTGCTCCCATGGAA (SEQ ID NO: 57) |
| TGAGCAGGGAAACCAGTGTAG (SEQ ID NO: 58) | CTACACTGTTCCCTGCTCA (SEQ ID NO: 59) |
| AGGGCTTTCTGATTGACAGCC (SEQ ID NO: 60) | GGCTGTCACAGAAAGCCCT (SEQ ID NO: 61) |
| ACCCCAAAGAAACACAATCCA (SEQ ID NO: 62) | TGGATTGTTTCTTTGGGGT (SEQ ID NO: 63) |
| ACAAATGCAGCAATAAACAGG (SEQ ID NO: 64) | CCTGTTTAGCTGCATTTGT (SEQ ID NO: 65) |
| AATAGACTGGCAGGTGGTCCA (SEQ ID NO: 66) | TGGACCACGCCAGTCTATT (SEQ ID NO: 67) |
| AAAGAATGAGCTTGATGTTGG (SEQ ID NO: 68) | CCAACATCGCTCATTCTTT (SEQ ID NO: 69) |
| AGATACTCATAGTCTTGGTAG (SEQ ID NO: 70) | CTACCAAGTATGAGTATCT (SEQ ID NO: 71) |
| AGAAACACAATCCAGTGGCCA (SEQ ID NO: 72) | TGGCCACTATTGTGTTTCT (SEQ ID NO: 73) |
| AAATAGGTCTCAATTAGCTTT (SEQ ID NO: 74) | AAAGCTAAGAGACCTATTT (SEQ ID NO: 75) |
| AAGAAACACAATCCAGTGGCC (SEQ ID NO: 76) | GGCCACTGTTGTGTTTCTT (SEQ ID NO: 77) |
| TAAACAGGTGGAAGGTCATTT (SEQ ID NO: 78) | AAATGACCCCACCTGTTTA (SEQ ID NO: 79) |
| TTGTAGTCGCCAAAGATCTGC (SEQ ID NO: 80) | GCAGATCTGGCGACTACAA (SEQ ID NO: 81) |
| AATTAGAGCCTCCATTCCTTT (SEQ ID NO: 82) | AAAGGAATAGGCTCTAATT (SEQ ID NO: 83) |
| TTAAGGACGGCAAAGTTGTAA (SEQ ID NO: 84) | TTACAACTGCCGTCCTTAA (SEQ ID NO: 85) |
| TTTAAGGACGGCAAAGTTGTA (SEQ ID NO: 86) | TACAACTTCCGTCCTTAAA (SEQ ID NO: 87) |
| ATGTCTTTGGGACTCTGACTC (SEQ ID NO: 88) | GAGTCAGACCCAAAGACAT (SEQ ID NO: 89) |
| TATCTATCCTGTGTCTACCAG (SEQ ID NO: 90) | CTGGTAGACAGGATAGATA (SEQ ID NO: 91) |
| AAATTACTTTCTGATCCTCAG (SEQ ID NO: 92) | CTGAGGATGAAAGTAATTT (SEQ ID NO: 93) |
| TCTAACAAGCCCATGTCTTTG (SEQ ID NO: 94) | CAAAGACAGGCTTGTTAGA (SEQ ID NO: 95) |
| AATTACTTTCTGATCCTCAGG (SEQ ID NO: 96) | CCTGAGGAAGAAAGTAATT (SEQ ID NO: 97) |
| AGTAAATGTACACAGGCACAG (SEQ ID NO: 98) | CTGTGCCTGTACATTTACT (SEQ ID NO: 99) |
| TAAGTAAGGTTGGCTGAGTTA (SEQ ID NO: 100) | TAACTCAGAACCTTACTTA (SEQ ID NO: 101) |
| TTCTGTGGGTGAAAGATCCTT (SEQ ID NO: 102) | AAGGATCTCACCCACAGAA (SEQ ID NO: 103) |
| AGAAGATGCTGACAACACCCT (SEQ ID NO: 104) | AGGGTGTTCAGCATCTTCT (SEQ ID NO: 105) |
| AATTGTAGCCGGCTGGCTAGT (SEQ ID NO: 106) | ACTAGCCACGGCTACAATT (SEQ ID NO: 107) |
| AGATTTGGGCAAACGCTCTTA (SEQ ID NO: 108) | TAAGAGCGTGCCCAAATCT (SEQ ID NO: 109) |
| ATTCTACGCTCCCTTATGCTG (SEQ ID NO: 110) | CAGCATAAGAGCGTAGAAT (SEQ ID NO: 111) |
| TGGTAATAGAGAGACCAGAAT (SEQ ID NO: 112) | ATTCTGGTCTCTATTACCA (SEQ ID NO: 113) |
| TATAGATGGCAAGAGGACCAA (SEQ ID NO: 114) | TTGGTCCTTGCCATCTATA (SEQ ID NO: 115) |
| AAACCAGTGTAGCTGCAGCCC (SEQ ID NO: 116) | GGGCTGCATACACTGGTTT (SEQ ID NO: 117) |

The miRNA of the present embodiment preferably includes any of the following pairs of nucleotide sequences.
- A pair of nucleotide sequences consisting of SEQ ID NO: 52 as an antisense sequence and SEQ ID NO: 53 as a sense sequence
- A pair of nucleotide sequences consisting of SEQ ID NO: 54 as an antisense sequence and SEQ ID NO: 55 as a sense sequence
- A pair of nucleotide sequences consisting of SEQ ID NO: 56 as an antisense sequence and SEQ ID NO: 57 as a sense sequence
- A pair of nucleotide sequences consisting of SEQ ID NO: 58 as an antisense sequence and SEQ ID NO: 59 as a sense sequence
- A pair of nucleotide sequences consisting of SEQ ID NO: 62 as an antisense sequence and SEQ ID NO: 63 as a sense sequence
- A pair of nucleotide sequences consisting of SEQ ID NO: 64 as an antisense sequence and SEQ ID NO: 65 as a sense sequence
- A pair of nucleotide sequences consisting of SEQ ID NO: 68 as an antisense sequence and SEQ ID NO: 69 as a sense sequence
- A pair of nucleotide sequences consisting of SEQ ID NO: 72 as an antisense sequence and SEQ ID NO: 73 as a sense sequence
- A pair of nucleotide sequences consisting of SEQ ID NO: 76 as an antisense sequence and SEQ ID NO: 77 as a sense sequence
- A pair of nucleotide sequences consisting of SEQ ID NO: 78 as an antisense sequence and SEQ ID NO: 79 as a sense sequence
- A pair of nucleotide sequences consisting of SEQ ID NO: 84 as an antisense sequence and SEQ ID NO: 85 as a sense sequence
- A pair of nucleotide sequences consisting of SEQ ID NO: 86 as an antisense sequence and SEQ ID NO: 87 as a sense sequence
- A pair of nucleotide sequences consisting of SEQ ID NO: 98 as an antisense sequence and SEQ ID NO: 99 as a sense sequence
- A pair of nucleotide sequences consisting of SEQ ID NO: 116 as an antisense sequence and SEQ ID NO: 117 as a sense sequence

The miRNA of the present embodiment more preferably includes any of the following pairs of nucleotide sequences.
- A pair of nucleotide sequences consisting of SEQ ID NO: 52 as an antisense sequence and SEQ ID NO: 53 as a sense sequence
- A pair of nucleotide sequences consisting of SEQ ID NO: 72 as an antisense sequence and SEQ ID NO: 73 as a sense sequence
- A pair of nucleotide sequences consisting of SEQ ID NO: 76 as an antisense sequence and SEQ ID NO: 77 as a sense sequence
- A pair of nucleotide sequences consisting of SEQ ID NO: 78 as an antisense sequence and SEQ ID NO: 79 as a sense sequence

Moreover, the miRNA of the present embodiment sequence may have a nucleotide sequence selected from the group consisting of SEQ ID NOs: 29 to 51 set forth in Table 2.

### [Table 2]

**Table 2. miRNA Sequences designed for human PLP1 gene**

| SEQ ID NO: | pre-miRNA Sequence |
|---|---|
| 29 | AGAAACACAATCCAGTGGCCAGTTTTGGCCACTGACTGACTGGCCACTATTGTGTTTCT |
| 30 | AAATAGGTCTCAATTAGCTTTGTTTTGGCCACTGACTGACAAAGCTAAGAGACCTATTT |
| 31 | AAGAAACACAATCCAGTGGCCGTTTTGGCCACTGACTGACGGCCACTGTTGTGTTTCTT |
| 32 | TAAACAGGTGGAAGGTCATTTGTTTTGGCCACTGACTGACAAATGACCCCACCTGTTTA |
| 33 | TTGTAGTCGCCAAAGATCTGCGTTTTGGCCACTGACTGACGCAGATCTGGCGACTACAA |
| 34 | AATTAGAGCCTCCATTCCTTTGTTTTGGCCACTGACTGACAAAGGAATAGGCTCTAATT |
| 35 | TTAAGGACGGCAAAGTTGTAAGTTTTGGCCACTGACTGACTTACAACTGCCGTCCTTAA |
| 36 | TTTAAGGACGGCAAAGTTGTAGTTTTGGCCACTGACTGACTACAACTTCCGTCCTTAAA |
| 37 | ATGTCTTTGGGACTCTGACTCGTTTTGGCCACTGACTGACGAGTCAGACCCAAAGACAT |
| 38 | TATCTATCCTGTGTCTACCAGGTTTTGGCCACTGACTGACCTGGTAGACAGGATAGATA |
| 39 | AAATTACTTTCTGATCCTCAGGTTTTGGCCACTGACTGACCTGAGGATGAAAGTAATTT |
| 40 | TCTAACAAGCCCATGTCTTTGGTTTTGGCCACTGACTGACCAAAGACAGGCTTGTTAGA |
| 41 | AATTACTTTCTGATCCTCAGGGTTTTGGCCACTGACTGACCCTGAGGAAGAAAGTAATT |
| 42 | AGTAAATGTACACAGGCACAGGTTTTGGCCACTGACTGACCTGTGCCTGTACATTTACT |
| 43 | TAAGTAAGGTTGGCTGAGTTAGTTTTGGCCACTGACTGACTAACTCAGAACCTTACTTA |
| 44 | TTCTGTGGGTGAAAGATCCTTGTTTTGGCCACTGACTGACAAGGATCTCACCCACAGAA |
| 45 | AGAAGATGCTGACAACACCCTGTTTTGGCCACTGACTGACAGGGTGTTCAGCATCTTCT |
| 46 | AATTGTAGCCGGCTGGCTAGTGTTTTGGCCACTGACTGACACTAGCCACGGCTACAATT |
| 47 | AGATTTGGGCAAACGCTCTTAGTTTTGGCCACTGACTGACTAAGAGCGTGCCCAAATCT |
| 48 | ATTCTACGCTCCCTTATGCTGGTTTTGGCCACTGACTGACCAGCATAAGAGCGTAGAAT |
| 49 | TGGTAATAGAGAGACCAGAATGTTTTGGCCACTGACTGACATTCTGGTCTCTATTACCA |
| 50 | TATAGATGGCAAGAGGACCAAGTTTTGGCCACTGACTGACTTGGTCCTTGCCATCTATA |
| 51 | AAACCAGTGTAGCTGCAGCCCGTTTTGGCCACTGACTGACGGGCTGCATACACTGGTTT |

The miRNA of the present embodiment may have a nucleotide sequence selected from the group consisting of SEQ ID NOs: 2 to 7 and 24 to 51, preferably has a nucleotide sequence selected from the group consisting of SEQ ID NOs: 2 to 5, 7, 24, 26, 29 to 32, 35, 36, 42, and 51, and more preferably has a nucleotide sequence selected from the group consisting of SEQ ID NOs: 2, 29, 31, and 32.

### [Vector]

The oligodendrocyte-specific vector of the present embodiment comprises an oligodendrocyte-specific promoter and/or an miRNA specific to the PLP1 gene. The vector of the present embodiment is a vector for use in treatment of a disease associated with abnormality in the PLP1 gene, or for the purpose of suppression of expression of the PLP1 gene.

The vector needs to be a plasmid or vector capable of infecting human cells and expressing a gene, and examples of the vector include adeno-associated virus (AAV), lentivirus, retrovirus, adenovirus, Sendai virus, and plasmids (including complexes with a liposome or a polymer), and the vector is preferably AAV. AAV is a small, helper-dependent envelope-free virus that is classified into the genus Dependovirus in the family Parvoviridae and infects animals including humans, primates, and rodents. The induction of immunoreaction by AAV is very weak, and no pathogenicity has been found for AAV. AAV is capable of infecting both dividing cells and non-dividing cells, and once infecting host cells, AAV can survive out of the nuclear chromosomes and express a gene with a low frequency of insertion of the gene into the chromosomal genome of the host.

For the AAV, for example, any of the serotypes AAV1, AAV2, AAV3, AAV4, AAV5, AAV6, AAV7, AAV8, and AAV9 is preferably used, and a hybrid mosaic AAV vector and a chimeric AAV vector are also preferably used. For the hybrid mosaic AAV vector, for example, any combinations of AAV1 to AAV9 including an AAV1/2 hybrid are available. Examples of the chimeric AAV vector include Olig001 (Powell SK et al., Gene Ther. 2016 Nov; 23 (11): 807-814), which has high tropism for oligodendrocytes.

The oligodendrocyte-specific promoter in the vector of the present embodiment is not limited to a particular promoter and may be any promoter that operates specifically in oligodendrocytes, and is preferably the promoter of the present embodiment, that is, the oligodendrocyte-specific promoter is, for example, an oligodendrocyte-specific promoter comprising a nucleic acid preferably having a sequence identity of at least 90% to the nucleotide sequence set forth in SEQ ID NO: 1. The miRNA specific to the PLP1 gene in the vector of the present embodiment is not limited to a particular miRNA and may be any miRNA capable of suppressing expression of the PLP1 gene, and preferably is the miRNA of the present embodiment, that is, the miRNA is an miRNA comprising a pair of nucleotide sequences consisting of an antisense sequence and a sense sequence, wherein the pair of nucleotide sequences is selected from the group consisting of pairs of an antisense sequence set forth in a left column of Table 1 and a sense sequence set forth in the just right of the antisense sequence in a right column of the table.

The vector of the present embodiment may comprise the oligodendrocyte-specific promoter of the present embodiment and a transgene operably linked to the promoter. The transgene is not limited to a particular transgene, and, for example, an miRNA sequence specific to the PLP1 gene, in particular, an miRNA sequence specific to the human PLP1 gene is preferably used. The miRNA sequence specific to the human PLP1 gene can be designed, for example, by using software such as BLOCK-iT (TM) RNAi Designer (Thermo Fisher Scientific, USA). Alternatively, the miRNA sequence specific to the human PLP1 gene can be designed in such a manner that artificial siRNAs are designed as described in Examples, and an optimum sequence is selected from the siRNAs to design the miRNA. For such an miRNA specific to the human PLP1 gene, for example, the above-described miRNA of the present embodiment is preferably used.

The vector of the present embodiment may comprise an oligodendrocyte-specific promoter and the miRNA of the present embodiment that is operably linked to the promoter. The oligodendrocyte-specific promoter is not limited to a particular promoter, and examples of the oligodendrocyte-specific promoter include a myelin basic protein gene promoter and a PLP1 gene promoter. For the oligodendrocyte-specific promoter, the above-described oligodendrocyte-specific promoter of the present embodiment is preferably used. Preferably, the vector of the present embodiment may comprise the oligodendrocyte-specific promoter of the present embodiment and one or more miRNAs of the present embodiment that are operably linked to the promoter.

The vector of the present embodiment may comprise a reporter gene to confirm gene expression. Examples of the reporter gene include, but are not limited to, GFP, Venus, and tdTomato.

The vector of the present embodiment can be constructed with ease through gene engineering. For example, an AAV1/2 hybrid vector including the hCNP promoter set forth in SEQ ID NO: 1 and a reporter gene can be produced with a method as described in the following. The hCNP promoter, a coding region for a reporter gene such as Venus, a 3' untranslated region, and an SV40 poly A signal are disposed in the order presented between two NotI sites of a self-complementary AAV vector, and the nucleotide sequence of an miRNA expression cassette is then inserted into the 3' untranslated region. The vector can be cryopreserved at -20°C until use, and, when the vector is used, AAV293 cells (Takara Bio Inc., Japan) are transfected with the vector together with a serotype-specific helper plasmid and the adenovirus helper plasmid pHelper, and then cultured for 72 hours to achieve amplification in cells or in the culture solution. Depending on the scale, purification can be performed by means of column purification, ultrafiltration, ultracentrifugation, or the like. The vector genome titer of the AAV vector obtained can be determined through quantitative PCR using an AAVpro Titration Kit (Takara Bio Inc., Japan).

### [Pharmaceutical composition]

The pharmaceutical composition of the present embodiment comprises the above-described vector of the present embodiment. The pharmaceutical composition of the present embodiment is preferably a pharmaceutical composition for treating a disease associated with abnormality in the PLP1 gene. Examples of the disease associated with abnormality involving the PLP1 gene include Pelizaeus-Merzbacher disease (PMD) and spastic paraplegia type 2, each of which is caused by duplication of the PLP1 gene, point mutation in the PLP1 gene, or deletion or insertion mutation involving the PLP1 gene, or multiple sclerosis. In addition, the pharmaceutical composition of the present embodiment may be a PLP1 gene expression suppressor capable of suppressing expression of the PLP1 gene.

The pharmaceutical composition of the present embodiment is preferably an aqueous solution containing an AAV vector, and may have a titer of 5 × 10¹⁰ to 5 × 10¹⁴ vg/mL, preferably of 5 × 10¹¹ to 5 × 10¹³ vg/mL.

The pharmaceutical composition of the present embodiment may contain a pharmaceutically acceptable additive component such as phosphate-buffered saline (PBS), and may further contain an additional therapeutic agent. The pharmaceutical composition of the present embodiment can be produced by dissolving in an aqueous solution such as water and adding other components, as necessary.

The pharmaceutical composition of the present embodiment may be directly administered to the brain in treating Pelizaeus-Merzbacher disease, and examples of such administration include administration to one site or multiple sites of the brain parenchyma (e.g., the white matter in the cerebral hemisphere, the internal capsule, the cerebellum) or the spinal cord, and intracerebroventricular/intrathecal or intravascular/intraperitoneal administration is also acceptable. Administration can be performed at any time after birth. The amount of injection can vary among administration sites and ages, and an amount of 0.1 to 2 mL/site is appropriate in the case of administration to the brain parenchyma, and it is preferred to administer about 0.5 mL/site. In the case of intracerebroventricular/intrathecal administration, an amount of 0.5 to 2 mL/kg body weight is appropriate, and it is preferred to administer up to about 1 mL/kg body weight. In the case of intravascular or intraperitoneal administration, an amount of 1 to 10 mL/kg body weight is appropriate, and it is preferred to administer up to 5 mL/kg body weight.

### [Therapeutic method]

The therapeutic method of the present embodiment is a method for treating a disease associated with abnormality of the PLP1 gene, the method comprising administering an effective amount of the above-described vector of the present embodiment to a patient in need of treatment. Alternatively, the therapeutic method of the present embodiment is a method for suppressing expression of the PLP1 gene, the method comprising administering an effective amount of the above-described vector of the present embodiment to a patient in need of treatment.

### Examples

Hereinafter, the present invention will be more specifically described with reference to Examples. However, the present invention is not limited to Examples in the following.

### <Example 1 Construction of Oligodendrocyte-Specific AAV Expression Cassette and AAV Vector Including the Same>

A pre-miRNA sequence (PLP1 miRNA, SEQ ID NO: 9) targeting the mRNA of a mouse PLP1 gene (NM_011123.3, SEQ ID NO: 8) and a pre-miRNA sequence as a negative control (miR-neg, SEQ ID NO: 10) were designed by using BLOCK-iT (TM) RNAi Designer (Thermo Fisher Scientific, USA). The miR-neg is an miRNA sequence that can form a hairpin structure, thereby processed into a mature miRNA, but is predicted not to target known vertebrate genes.
PLP1-miRNA (SEQ ID NO: 9): 
5'-
ACTCCAAAGAAACACAATCCAGTTTTGGCCACTGACTGACTG
 GATTGTTTCTTTGGAGT -3'
miR-neg (SEQ ID NO: 10):

5'-
GTATGCATCGAATGAGATTCCGTTTTGGCCACTGACTGACGGA
ATCTCTCGATGCATAC -3'

Each of The PLP1 miRNA sequence and the miR-neg sequence was synthesized as designed, and cloned into a cloning site of a pcDNA (TM) 6.2-GW/miR vector (Thermo Fisher Scientific, USA) included in a BLOCK-iT (TM) Pol II miR RNAi expression vector kit. A 5' flanking sequence (SEQ ID NO: 11) and 3' flanking sequence (SEQ ID NO: 12) derived from mouse miR-155 were respectively disposed at one side and the other side of the PLP1 miRNA sequence or miR-neg sequence. The 5' flanking sequence and 3' flanking sequence derived from mouse miR-155 are respectively the 5' region and 3' region of mouse miR-155 with a portion of hairpin structure excluded therefrom, and the pre-miRNA sequence or miR-neg sequence was disposed between the 5' flanking sequence and the 3' flanking sequence so that these miRNA sequences could be expressed.
5' flanking sequence (SEQ ID NO: 11):
   5'- CTGGAGGCTTGCTGAAGGCTGTATGCT -3'
3' flanking sequence (SEQ ID NO: 12):

5'-
CAGGACACAAGGCCTGTTACTAGCACTCACATGGAACAAATG
GCC -3'

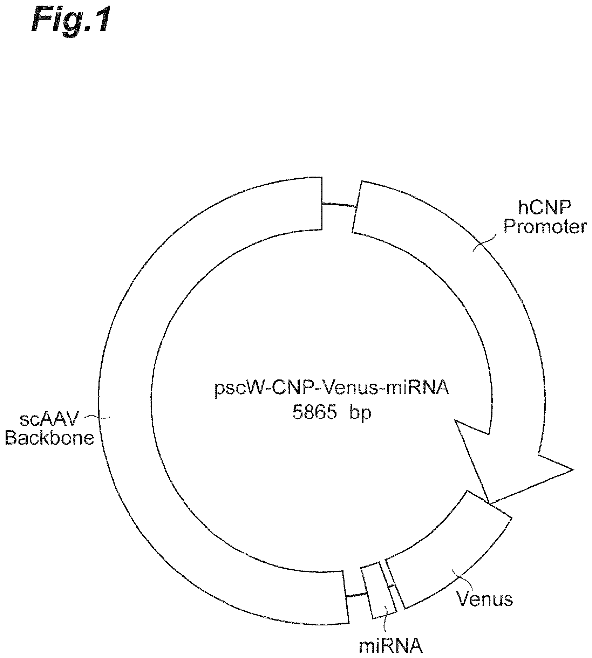

From the resulting pcDNA (TM) 6.2-GW/miR vector, the portion of 5' flanking sequence-PLP1 miRNA sequence (or miR-neg sequence)-3' flanking sequence was PCR-amplified, the amplified product was cleaved with XhoI/BamHI, and the resultant was inserted into a DNA fragment obtained by cleaving a pW-CAG-Venus-WPRE vector (SEQ ID NO: 13) with XhoI/BamHI and cleaved at both ends of WPRE positioned in the 3' side of a venus (SEQ ID NO: 14) sequence to remove the WPRE sequence. Subsequently, the resulting vector was cleaved with SpeI/EcoRI to remove the CAG promoter, and a DNA fragment was inserted into this site in the 5' side of the Venus sequence, the DNA fragment being obtained by PCR-amplifying a human CNP promoter (SEQ ID NO: 1) of 1.8 kb and cleaved at the both ends with SpeI/EcoRI, to enable oligodendrocyte-specific gene expression. The resulting vector was further cleaved with NotI to remove the backbone of single-stranded AAV derived from pW-CAG-Venus-WPRE, and bonded to the backbone of self-complementary adeno-associated virus (scAAV) obtained by cleaving pscW-PABPN1 (SEQ ID NO: 15) with NotI to afford the final constructs Pscw-hCNP-Venus-PLP1 miRNA (SEQ ID NO: 16) and Pscw-hCNP-Venus-miR-neg (SEQ ID NO: 17) (Figure 1).

Preparation of an AAV1/2 hybrid vector (scAAV-AAV1/2 vector) was performed in accordance with a method disclosed in Non Patent Literature 1. Specifically, AAV293 cells were co-transfected using PEI with the Pscw-hCNP-Venus-PLP1 miRNA or Pscw-hCNP-Venus-miR-neg, which was an scAAV construct obtained above, the serotype-specific AAV helper plasmids p5E18RXC1 (Xiao W et al., J Virol (1999) 73: 3994-4003) and pAAV-RC (Agilent Technologies) encoding the rep and cap genes of AAV1 and AAV2, respectively, and the adenovirus helper plasmid pHelper (Agilent Technologies). The cells were collected 72 hours after the transfection, and each scAAV-AAV1/2 vector was purified by using an AAVpro Purification Kit (Takara Bio Inc., Japan). Vector genome titers were determined through quantitative PCR using an AAVpro Titration Kit (Takara Bio Inc., Japan).

The scAAV-AAV1/2 vectors obtained were designated as the AAV-hCNP-Venus-PLP1 miRNA vector (or PLP1 miRNA vector) and the AAV-hCNP-Venus-miR-neg vector (or miR-neg vector).

### <Example 2 Administration of AAV Vectors to Wild-Type Mice and Expression Thereof>

One microliter of a solution of the PLP1 miRNA vector or miR-neg vector obtained in Example 1 (titer: 1.2 × 10¹² vg/mL) was injected into one striatum and the internal capsule of each 10-day-old, wild-type mouse Jcl:B6C3F1 (n = 5 per group). Before the injection, the mice were anesthetized with pentobarbital solution. The injection of the vector was performed with a 33G syringe at a rate of 150 nL/min. After the injection each mouse was left untouched there for 1 minute, and the needle was slowly pulled out of the brain. Thereafter, the mice were returned as a group to their mother, and grown until day 17 after birth.

Gene expression from each vector was evaluated by detecting the green fluorescent protein Venus on day 17 after birth. Types of Venus-expressing cells were identified through double immunostaining with cell markers including Gst-π (for mature oligodendrocytes), NeuN (for neurons), Iba1 (for microglia), and GFAP (astrocytes).

For immunostaining, each Jcl:B6C3F1 mouse was anesthetized with Forane inhalation solution, and subsequently subjected to transcardial perfusion with PBS and then with fresh 4% paraformaldehyde. The brain was removed and further fixed at 4°C overnight, and then subjected to substitution with 30% sucrose/PBS and cryopreserved in the embedding medium OCT Compound (TissueTech, Inc.). A coronal section (20 µm) was obtained, and immunostained to check specificity of infected cells expressing Venus. The section was incubated together with blocking solution (PBS containing 0.05% Triton (TM) X-100 and 5% goat serum) at room temperature for 3 hours, and incubated at 4°C overnight with Gst-π (Cell Signaling Technology, Inc., 1:300), GFAP (Millipore Corporation, 1:400), NeuN (Chemicon, 1:400), or Iba1 (Biocare Medical, LLC., 1:500), as primary antibodies. The section was then washed, and incubated together with an appropriate secondary antibody (Thermo Fisher Scientific, USA) at room temperature for 1 hour. Cell nuclei were visualized with 40,60-diamidino-2-phenylindole (DAPI, Sigma-Aldrich Co. LLC). The slide was mounted with ProLong (R) Diamond Mountant (Thermo Fisher Scientific, USA), and photographed with a KEYENCE fluorescence microscope (KEYENCE CORPORATION, Japan).

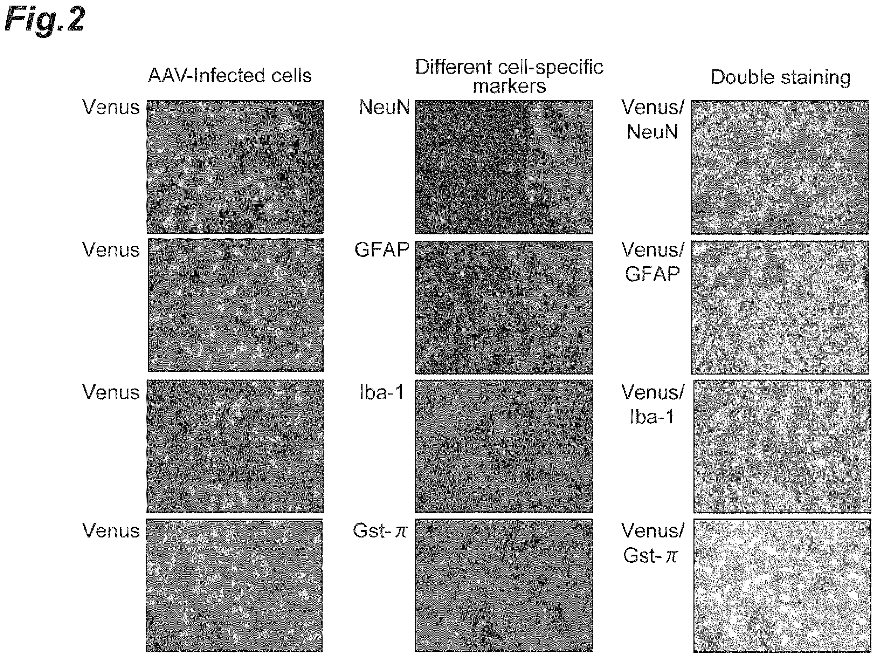

Figure 2 shows the results. It is understood from Figure 2 that Venus-positive cells corresponded to Gst-π-positive oligodendrocytes but did not correspond to NeuN-positive cell neurons, GFAP-positive astrocytes, or Iba1-positive microglia. Thus, Figure 2 demonstrates that the miR-neg vector was expressed in an oligodendrocyte-specific and highly efficient manner.

### <Example 3 Evaluation of Suppression Efficiency of Expression (Knockdown) in Terms of PLP1 Gene Expression Levels - Part 1>

One microliter of a solution of each of the two scAAV-AAV1/2 vectors (titer: 1.2 × 10¹² vg/mL) obtained in Example 1 was injected into one striatum, the internal capsule, and the cerebellum of each 10-day-old, wild-type mouse Jcl:B6C3F1 (n = 3 per group). The injection method was the same as in Example 2. Thereafter, expression of PLP1 protein in Venus-positive cells was examined through immunostaining to evaluate PLP1 miRNA knockdown efficiencies. Immunostaining was performed with the same method as in Example 2 except that an anti-PLP1 rabbit polyclonal antibody (Numata Y et al., J Biol Chem. 2013 Mar 15; 288 (11): 7451-66) was used as a primary antibody, and an anti-rabbit fluorescent antibody (Thermo Fisher Scientific, USA) was used as a secondary antibody. Each site of the callosum, striatum, and internal capsule were photographed with a KEYENCE fluorescence microscope (KEYENCE CORPORATION, Japan).

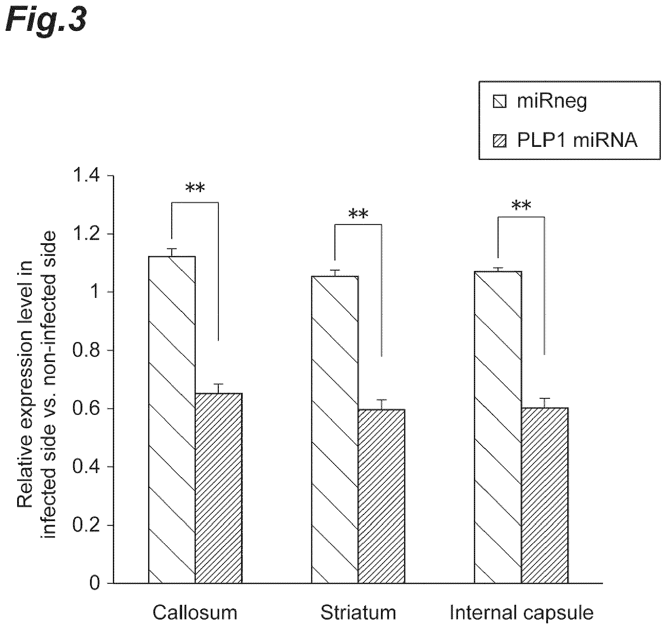

Mean fluorescence intensities of immunostained PLP1 were quantified by using Image J 1.45s (Wayne Rasband, National Institutes of Health, USA), and Figure 3 shows quantitative analysis of fluorescence intensity through PLP1 immunofluorescence staining. It can be seen from Figure 3 that there was almost no difference in expression of PLP1 protein between the side without injection of the miR-neg vector (non-infected side) and the side with vector injection (infected side), whereas for the PLP1 miRNA vector there was 40% of expression-suppressing effect on PLP1 protein in the infected side as compared with the non-infected side. The result that there was no difference in the number of astrocytes and that of microglia between the non-infected side and the infected side indicates the absence of reactive inflammatory response due to AAV administration.

### <Example 4 Evaluation of Suppression Efficiency of Expression (Knockdown) in Terms of PLP1 Gene Expression Levels - Part 2>

One microliter of a solution of each of the two scAAV-AAV1/2 vectors (titer: 1.2 × 10¹² vg/mL) obtained in Example 1 was injected into both striata and the internal capsule of each 10-day-old, wild-type mouse Jcl:B6C3F1 (n = 4 per group). The injection method was the same as in Example 2. Thereafter, expression of the PLP1 mRNA in Venus-positive cells was examined through quantitative PCR to evaluate PLP1 miRNA knockdown efficiency.

To sort Venus-positive cells, each Jcl:B6C3F1 mouse was anesthetized with Forane inhalation solution, and subjected to transcardial perfusion with 20 mL of PBS. The brain was removed, and roughly fragmented with micro dice scissors. To each brain, 1 mL of Accutase (Millipore Corporation) was added, and the resultant was incubated at 37°C for 30 minutes. To each sample, 2 mL of Hanks' balanced salt solution (HBSS) containing 10% FBS was added, and the tissue was homogenized with a micropipette. Each sample was filtered with a 100-µm cell strainer, and the collected cell suspension was centrifuged. To purify cells from myelin debris, cells were resuspended in HBSS containing 40% Percoll (Amersham plc), and centrifuged at 700 ×g at room temperature for 25 minutes. The upper myelin layer was sucked, and monocytes were resuspended in 1 mL of a DMEM/F12 medium. These cells were separated by an FACS Canto flow cytometer (BD Biosciences), and the result was analyzed by using the software FlowJo (Tree Star Inc., OR, RRID: NIF-0000 to 30575). The result is plotted as percent to all cells.

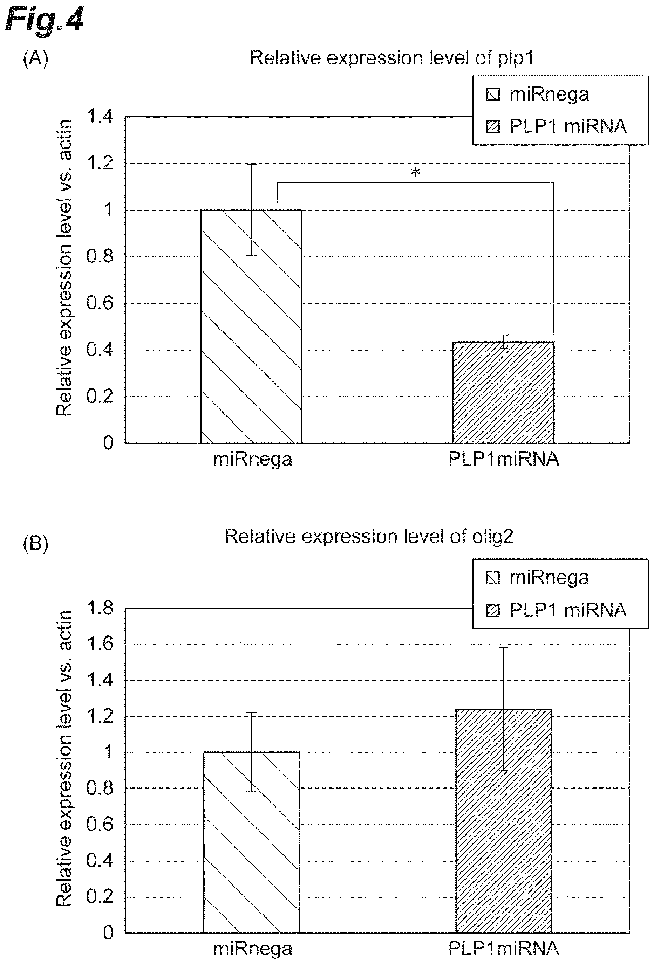

For quantitative PCR, the total RNA was isolated from the selected Venus-positive cells using an RNeasy Mini Kit (Qiagen), and a cDNA was synthesized by using a SuperScript (R) Reverse Transcriptase Kit (Thermo Fisher Scientific, USA). Gene expression was analyzed through quantitative RT-PCR using LightCycler (R) 480 SYBR Green I Master and a LightCycler (R) 480 Instrument with specific primers below (F. Hoffmann-La Roche Ltd., Switzerland). The results were normalized to the house keeping gene β-actin, and relative expression levels of PLP1 and Olig2 were calculated. CP values (crossing point values) obtained for each specimen were converted into log values to plot relative expression levels ± SD, and the plotted results are shown in Figure 4.
PLP1 forward primer (SEQ ID NO: 18): 5'-GTTCCAGAGGCCAACATCAAGCTC -3'
PLP1 reverse primer (SEQ ID NO: 19): 5'-AGCCATACAACAGTCAGGGCATAG -3'
Olig2 forward primer (SEQ ID NO: 20): 5'-GGGAGGTCATGCCTTACGC -3'
Olig2 reverse primer (SEQ ID NO: 21): 5'-CTCCAGCGAGTTGGTGAGC -3'
β-actin forward primer (SEQ ID NO: 22): 5'-CACAGCTTCTTTGCAGCTCCTT -3'
β-actin reverse primer (SEQ ID NO: 23): 5'-GACGACCAGCGCAGCGATA -3'

It can be seen from Figure 4 that for the expression level of the mRNA of Olig2, an oligodendrocyte marker, there was no significant difference between the miR-neg vector and the PLP1 miRNA vector (B), whereas for the expression level of the PLP1 mRNA, injection of the PLP1 miRNA vector provided approximately 60% of expression-suppressing effect on the PLP1 gene (A).

### <Example 5 Effect of Administration to PLP1 Transgenic (PLP1-Tg) Mice>

To both striata and the internal capsule of each of a litter of 10-day-old (body weight: 5 to 6 g) PLP1-Tg/B6C3 mice overexpressing mouse PLP1, the PLP1 miRNA vector (group with treatment, n = 16) or the miR-neg vector (group with mock treatment, n = 16) was injected to conduct treatment test. PLP1-Tg/B6C3 was prepared by backcrossing PLP1-Tg/BDF1, which was gifted from Dr. Tetsushi Kagawa in National Institute for Physiological Sciences, with a B6C3 strain (Kagawa T et al., Neuron. 1994 Aug; 13 (2): 427-42). For PLP1-Tg-homozygous individuals, 1 µL of a solution of each of the two scAAV-AAV1/2 vectors (titer: 1.2 × 10¹² vg/mL) obtained in Example 1 was injected into both striata and the internal capsule of each 10-day-old mouse in the same manner as in Example 2. For a positive control, wild-type litter mate mice injected with the miR-neg vector (n = 16) were used. Some of those mice were removed of brain tissue on day 25 after birth (15 days after the injection) and histological analysis (immunostaining, morphometric analysis) was conducted for the brain tissue, and the residual mice were observed for body weights and survival rates.

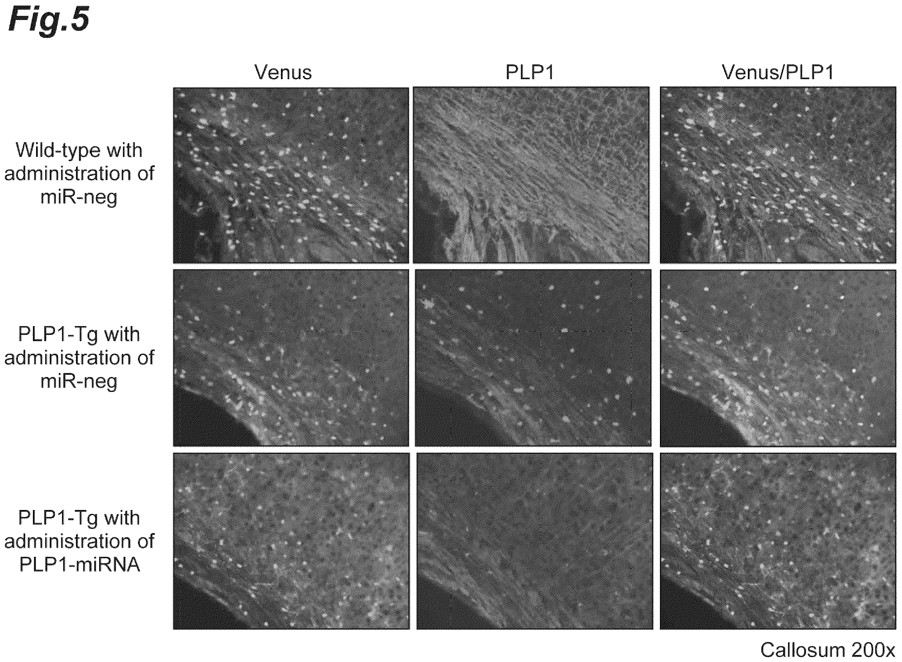

Histological analysis by immunostaining was carried out as follows. For the group of wild-type mice, and the group of PLP1-Tg mice with treatment and that with mock treatment, where n = 5 per group, each of the callosum, striatum, and internal capsule were immunostained in the same manner as in Example 3 on day 25 after birth (15 days after the injection). For the wild-type mice as a positive control group, PLP1 provided fibrous, strongly stained images corresponding to myelin sheaths in the callosum, striatum, and internal capsule. In analysis of the PLP1-Tg mice, almost no stained image corresponding to myelin sheaths was found for the mice with mock treatment, whereas a stained image indicating abnormal accumulation of PLP1 in the cytoplasm of oligodendrocytes were obtained. For the mice with treatment, by contrast, almost no stained image indicating the accumulation of PLP1 in cytoplasm was found, and a fibrous stained image inferred to correspond to myelin sheaths were found, which revealed that administration of the PLP1 miRNA vector provides improved stained images of PLP1 as compared with abnormal ones. Figure 5 shows micrographs of callosum.

Micromorphological analysis with an electron microscope was performed in the following manner. For the wild-type mice and the group of PLP1-Tg mice with treatment and that with mock treatment, where n = 2 per group, fixing by reflux was performed with 0.1 M phosphate buffer containing 2% glutaraldehyde and 2% paraformaldehyde on day 25 after birth (15 days after the injection), and then the brain was removed and further fixed by soaking in the same buffer. Each of the callosum, striatum, and internal capsule was checked for AAV-infected sites under a stereoscopic fluorescence microscope, a tissue section of 600-µm square was cut out, and this was post-fixed with 0.1 M phosphate buffer containing 1% osmium. After dehydration, the tissue section was embedded in epoxy resin, a 70-nm ultrathin section was prepared, and this was observed through an electron microscope (Tecnai Spirit, Thermo Fisher Scientific, USA) at a magnification of 7300×.

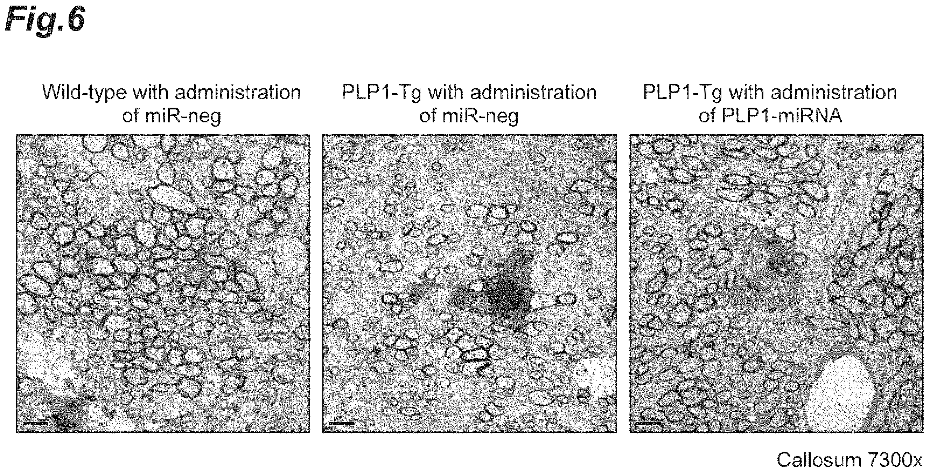

Figure 6 shows electron micrographs. It was clear that for the wild-type mice almost all axons were myelinated in each of the callosum, striatum, and internal capsule. In analysis of the PLP1-Tg mice, the fraction of myelinated axons was clearly low in the mice with mock treatment, and moreover the axons were narrowed. In the mice with treatment, on the other hand, the fraction of myelinated axons was higher, and moreover diameters of axons were generally larger than those in the mice with mock treatment. From these results, it was revealed from the viewpoint of ultrafine morphology that administration of the PLP1 miRNA vector provides therapeutic effect owing to enrichment of myelinated fibers.

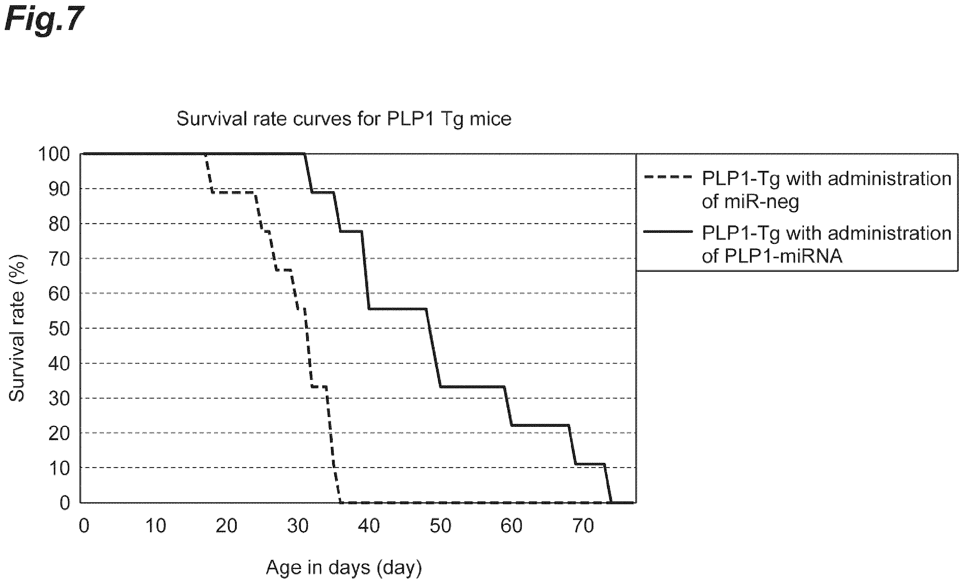

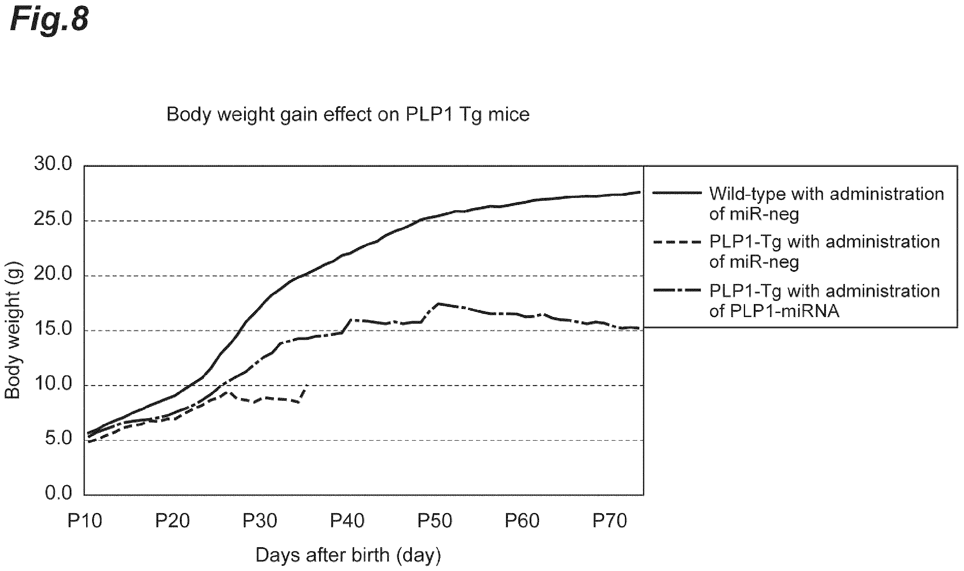

The group of mice with treatment and group of mice with mock treatment, where n = 9 per group, were observed even after 25 days in age until their death to evaluate body weights and survival rates for the groups. Administration to the PLP1-Tg mice was found to provide significant survival rate-improving (life-prolonging) effect (Figure 7) and body weight gain effect (Figure 8), and thus the efficacy of the present therapy was confirmed.

The thus-described results revealed that the oligodendrocyte-specific promoter and the AAV vector including the promoter can be applicable as a fundamental technique to develop therapy for PMD, and that the gene expression AAV vector with a PLP1-specific miRNA using the promoter can be an effective approach for treatment of PMD.

### <Example 6 Evaluation of Suppression Efficiency of Expression (Knockdown) in Terms of PLP1 Gene Expression Levels - Part 3>

To evaluate the knockdown efficiency of the human PLP1-miRNA designed with BLOCK-iT (TM) RNAi Designer (Thermo Fisher Scientific, USA), pcDNA (TM) 6.2-hPLP1, which is a plasmid capable of overexpressing a sequence encoding the human PLP1 gene, and pscw.CAG.Venus.PLP1-miRNA, which is a plasmid obtained by cloning a sequence into a 3' untranslated region in the downstream of a cDNA encoding Venus, a fluorescent protein that is expressed under the control of a CAG promoter, were concomitantly introduced into HeLa cells through a transfection method. For the transfection, TransIt-LT1 (Takara Bio Inc.) was used. After 24 hours cells were collected, and the total RNA was extracted by using an RNeasy Mini Kit (Qiagen). For quantitative PCR, a cDNA was synthesized by using a SuperScript (R) Reverse Transcriptase Kit (Thermo Fisher Scientific, USA). Gene expression was analyzed through quantitative PCR using LightCycler (R) 480 SYBR Green I Master and a LightCycler (R) 480 Instrument with specific primers below (F. Hoffmann-La Roche Ltd., Switzerland). The primers used in the quantitative PCR for PLP1 gene expression are listed in the following.
Human PLP1 forward primer (SEQ ID NO: 280): 5'-GCTCCAACCTTCTGTCCATCT -3'
Human PLP1 reverse primer (SEQ ID NO: 281): 5'-ACGGCAAAGTTGTAAGTGGC -3'
Human β-actin forward primer (SEQ ID NO: 282): 5'-GACAGGATGCAGAAGGAGATTACT -3'
Human β-actin reverse primer (SEQ ID NO: 283): 5'-TGATCCACATCTGCTGGAAGGT -3'

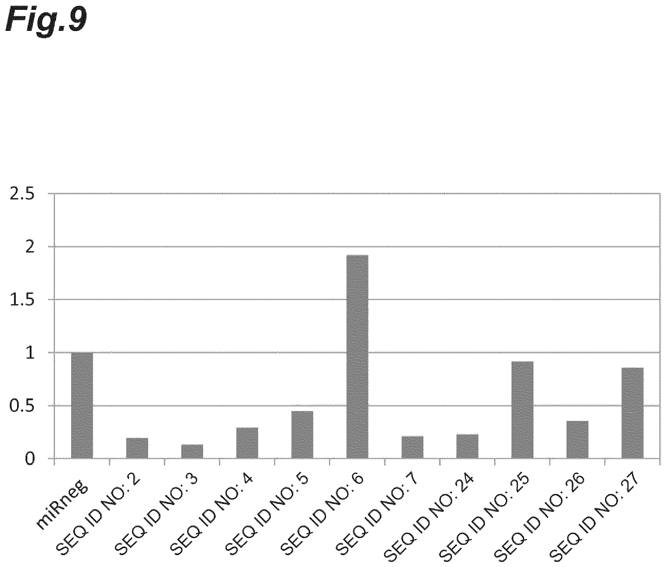

Figure 9 shows results of the knockdown. Knockdown efficiencies were calculated assuming the PLP1 expression level of cells with miR-neg expression as 1. The results were normalized to the house keeping gene β-actin, and calculated as relative expression levels ± SD. Seven sequences that lower PLP1 gene expression levels were identified (SEQ ID NOs: 2, 3, 4, 5, 7, 24, and 26).

### <Example 7 Evaluation of Suppression Efficiency of Expression (Knockdown) in Terms of PLP1 Gene Expression Levels - Part 4>

A pair of siRNAs consisting of a sense strand and the corresponding antisense strand shown in Table 3 and transfection solution (RNAi MAX, Invitrogen) were mixed together, and the solution prepared was added to U-251MG cells, and the cells were cultured for 48 hours.

### [Table 3]

**Table 3. siRNA Sequences designed for human PLP1 gene**

| No. | SEQ ID NO: | Sense/Antisense strand | siRNA Sequence |
|---|---|---|---|
| 1 | 118 | hPLP1-001 | UGACCUUCCACCUGUUUAUdTdT |
| | 119 | hPLP1-001anti | AUAAACAGGUGGAAGGUCAdTdT |
| 2 | 120 | hPLP1-002 | GUGCCUGUGUACAUUUACUdTdT |
| | 121 | hPLP1-002anti | AGUAAAUGUACACAGGCACdTdT |
| 3 | 122 | hPLP1-003 | UUGCCCAAAUCUGCCUAUUdTdT |
| | 123 | hPLP1-003anti | AAUAGGCAGAUUUGGGCAAdTdT |
| 4 | 124 | hPLP1-004 | AGGAAUGGAGGCUCUAAUUdTdT |
| | 125 | hPLP1-004anti | AAUUAGAGCCUCCAUUCCUdTdT |
| 5 | 126 | hPLP1-005 | UGGCCACUGGAUUGUGUUUdTdT |
| | 127 | hPLP1-005anti | AAACACAAUCCAGUGGCCAdTdT |
| 6 | 128 | hPLP1-006 | GCGGGUGUGUCAUUGUUUGdTdT |
| | 129 | hPLP1-006anti | CAAACAAUGACACACCCGCdTdT |
| 7 | 130 | hPLP1-007 | GACCUUCCACCUGUUUAUUdTdT |
| | 131 | hPLP1-007anti | AAUAAACAGGUGGAAGGUCdTdT |
| 8 | 132 | hPLP1-008 | ACUCCCUUCUCCUUGAUAAdTdT |
| | 133 | hPLP1-008anti | UUAUCAAGGAGAAGGGAGUdTdT |
| 9 | 134 | hPLP1-009 | CUGCAAGUCACAAAGGAAUdTdT |
| | 135 | hPLP1-009anti | AUUCCUUUGUGACUUGCAGdTdT |
| 10 | 136 | hPLP1-010 | GGUCCUCUUGCCAUCUAUAdTdT |
| | 137 | hPLP1-010anti | UAUAGAUGGCAAGAGGACCdTdT |
| 11 | 138 | hPLP1-011 | CCUCGUUAGGGAAGAGAAAdTdT |
| | 139 | hPLP1-011anti | UUUCUCUUCCCUAACGAGGdTdT |
| 12 | 140 | hPLP1-012 | GGAGAAGAGGACAAAGAUAdTdT |
| | 141 | hPLP1-012anti | UAUCUUUGUCCUCUUCUCCdTdT |
| 13 | 142 | hPLP1-013 | GGCCAACAUCAAGCUCAUUdTdT |
| | 143 | hPLP1-013anti | AAUGAGCUUGAUGUUGGCCdTdT |
| 14 | 144 | hPLP1-014 | AGGCCAACAUCAAGCUCAUdTdT |
| | 145 | hPLP1-014anti | AUGAGCUUGAUGUUGGCCUdTdT |
| 15 | 146 | hPLP1-015 | CUCCCUUCUCCUUGAUAACdTdT |
| | 147 | hPLP1-015anti | GUUAUCAAGGAGAAGGGAGdTdT |
| 16 | 148 | hPLP1-016 | GCCUCUUUCUUCUUCCUUUdTdT |
| | 149 | hPLP1-016anti | AAAGGAAGAAGAAAGAGGCdTdT |
| 17 | 150 | hPLP1-017 | CUGUGCCUGUGUACAUUUAdTdT |
| | 151 | hPLP1-017anti | UAAAUGUACACAGGCACAGdTdT |
| 18 | 152 | hPLP1-018 | AAGGAAUGGAGGCUCUAAUdTdT |
| | 153 | hPLP1-018anti | AUUAGAGCCUCCAUUCCUUdTdT |
| 19 | 154 | hPLP1-019 | AGGGUGUUGUCAGCAUCUUdTdT |
| | 155 | hPLP1-019anti | AAGAUGCUGACAACACCCUdTdT |
| 20 | 156 | hPLP1-020 | ACAGCUGAGUUCCAAAUGAdTdT |
| | 157 | hPLP1-020anti | UCAUUUGGAACUCAGCUGUdTdT |
| 21 | 158 | hPLP1-021 | GCCACUGGAUUGUGUUUCUdTdT |
| | 159 | hPLP1-021anti | AGAAACACAAUCCAGUGGCdTdT |
| 22 | 160 | hPLP1-022 | ACAACUUUGCCGUCCUUAAdTdT |
| | 161 | hPLP1-022anti | UUAAGGACGGCAAAGUUGUdTdT |
| 23 | 162 | hPLP1-023 | GCUGAUGCCAGAAUGUAUGdTdT |
| | 163 | hPLP1-023anti | CAUACAUUCUGGCAUCAGCdTdT |
| 24 | 164 | hPLP1-024 | UUGCCGUCCUUAAACUCAUdTdT |
| | 165 | hPLP1-024anti | AUGAGUUUAAGGACGGCAAdTdT |
| 25 | 166 | hPLP1-025 | CUGCCUCUUUCUUCUUCCUdTdT |
| | 167 | hPLP1-025anti | AGGAAGAAGAAAGAGGCAGdTdT |
| 26 | 168 | hPLP1-026 | CCACUGGAUUGUGUUUCUUdTdT |
| | 169 | hPLP1-026anti | AAGAAACACAAUCCAGUGGdTdT |
| 27 | 170 | hPLP1-027 | GGAUCAGAAAGUAAUUUCUdTdT |
| | 171 | hPLP1-027anti | AGAAAUUACUUUCUGAUCCdTdT |
| 28 | 172 | hPLP1-028 | UAGCCAGCCGGCUACAAUUdTdT |
| | 173 | hPLP1-028anti | AAUUGUAGCCGGCUGGCUAdTdT |
| 29 | 174 | hPLP1-029 | AGCGUAGAAUCUGUGUAGAdTdT |
| | 175 | hPLP1-029anti | UCUACACAGAUUCUACGCUdTdT |
| 30 | 176 | hPLP1-030 | UCCCUUCUCCUUGAUAACAdTdT |
| | 177 | hPLP1-030anti | UGUUAUCAAGGAGAAGGGAdTdT |
| 31 | 178 | hPLP1-031 | UUCCAAGGAGCUGAGAAUAdTdT |
| | 179 | hPLP1-031anti | UAUUCUCAGCUCCUUGGAAdTdT |
| 32 | 180 | hPLP1-032 | UGCCAGAAUGUAUGGUGUUdTdT |
| | 181 | hPLP1-032anti | AACACCAUACAUUCUGGCAdTdT |
| 33 | 182 | hPLP1-033 | AUGCCUUCCAGUAUGUCAUdTdT |
| | 183 | hPLP1-033anti | AUGACAUACUGGAAGGCAUdTdT |
| 34 | 184 | hPLP1-034 | CAACUUUGCCGUCCUUAAAdTdT |
| | 185 | hPLP1-034anti | UUUAAGGACGGCAAAGUUGdTdT |
| 35 | 186 | hPLP1-035 | CCAACAUCAAGCUCAUUCUdTdT |
| | 187 | hPLP1-035anti | AGAAUGAGCUUGAUGUUGGdTdT |
| 36 | 188 | hPLP1-036 | UGGCCUUACACCUCGUUAGdTdT |
| | 189 | hPLP1-036anti | CUAACGAGGUGUAAGGCCAdTdT |
| 37 | 190 | hPLP1-037 | AGGACAUCCCGACAAGUUUdTdT |
| | 191 | hPLP1-037anti | AAACUUGUCGGGAUGUCCUdTdT |
| 38 | 192 | hPLP1-038 | GUCAGAGUCCCAAAGACAUdTdT |
| | 193 | hPLP1-038anti | AUGUCUUUGGGACUCUGACdTdT |
| 39 | 194 | hPLP1-039 | UUGCAGGAGAAGAGGACAAdTdT |
| | 195 | hPLP1-039anti | UUGUCCUCUUCUCCUGCAAdTdT |
| 40 | 196 | hPLP1-040 | GGUAGACACAGGAUAGAUAdTdT |
| | 197 | hPLP1-040anti | UAUCUAUCCUGUGUCUACCdTdT |
| 41 | 198 | hPLP1-041 | UCAGCCAACCUUACUUACAdTdT |
| | 199 | hPLP1-041anti | UGUAAGUAAGGUUGGCUGAdTdT |
| 42 | 200 | hPLP1-042 | AUGGAACUGCCUCUUUCUUdTdT |
| | 201 | hPLP1-042anti | AAGAAAGAGGCAGUUCCAUdTdT |
| 43 | 202 | hPLP1-043 | ACUCAGCCAACCUUACUUAdTdT |
| | 203 | hPLP1-043anti | UAAGUAAGGUUGGCUGAGUdTdT |
| 44 | 204 | hPLP1-044 | CUUCCACUGAUGGAAACAAdTdT |
| | 205 | hPLP1-044anti | UUGUUUCCAUCAGUGGAAGdTdT |
| 45 | 206 | hPLP1-045 | AUGACCUUCCACCUGUUUAdTdT |
| | 207 | hPLP1-045anti | UAAACAGGUGGAAGGUCAUdTdT |
| 46 | 208 | hPLP1-046 | UCUCCUGAGGAUCAGAAAGdTdT |
| | 209 | hPLP1-046anti | CUUUCUGAUCCUCAGGAGAdTdT |
| 47 | 210 | hPLP1-047 | GAGGAUCAGAAAGUAAUUUdTdT |
| | 211 | hPLP1-047anti | AAAUUACUUUCUGAUCCUCdTdT |
| 48 | 212 | hPLP1-048 | GGAUCUUUCACCCACAGAAdTdT |
| | 213 | hPLP1-048anti | UUCUGUGGGUGAAAGAUCCdTdT |
| 49 | 214 | hPLP1-049 | CUGAGGAUCAGAAAGUAAUdTdT |
| | 215 | hPLP1-049anti | AUUACUUUCUGAUCCUCAGdTdT |
| 50 | 216 | hPLP1-050 | CUGGUAGACACAGGAUAGAdTdT |
| | 217 | hPLP1-050anti | UCUAUCCUGUGUCUACCAGdTdT |
| 51 | 218 | hPLP1-051 | GCAGUUGCUGGUGGCUAAUdTdT |
| | 219 | hPLP1-051anti | AUUAGCCACCAGCAACUGCdTdT |
| 52 | 220 | hPLP1-052 | AAGACAUGGGCUUGUUAGAdTdT |
| | 221 | hPLP1-052anti | UCUAACAAGCCCAUGUCUUdTdT |
| 53 | 222 | hPLP1-053 | UUGCUGCCACUUACAACUUdTdT |
| | 223 | hPLP1-053anti | AAGUUGUAAGUGGCAGCAAdTdT |
| 54 | 224 | hPLP1-054 | UCGUUAGGGAAGAGAAACAdTdT |
| | 225 | hPLP1-054anti | UGUUUCUCUUCCCUAACGAdTdT |
| 55 | 226 | hPLP1-055 | UCCACUGAUGGAAACAAAGdTdT |
| | 227 | hPLP1-055anti | CUUUGUUUCCAUCAGUGGAdTdT |
| 56 | 228 | hPLP1-056 | AGAGCGUUUGCCCAAAUCUdTdT |
| | 229 | hPLP1-056anti | AGAUUUGGGCAAACGCUCUdTdT |
| 57 | 230 | hPLP1-057 | UGAUUGCUGCCACUUACAAdTdT |
| | 231 | hPLP1-057anti | UUGUAAGUGGCAGCAAUCAdTdT |
| 58 | 232 | hPLP1-058 | GGAGCGUAGAAUCUGUGUAdTdT |
| | 233 | hPLP1-058anti | UACACAGAUUCUACGCUCCdTdT |
| 59 | 234 | hPLP1-059 | UGAGGAUCAGAAAGUAAUUdTdT |
| | 235 | hPLP1-059anti | AAUUACUUUCUGAUCCUCAdTdT |
| 60 | 236 | hPLP1-060 | GGUGUUGUCAGCAUCUUCUdTdT |
| | 237 | hPLP1-060anti | AGAAGAUGCUGACAACACCdTdT |
| 61 | 238 | hPLP1-061 | GUCAAAGCAAGGAUCUUUCdTdT |
| | 239 | hPLP1-061anti | GAAAGAUCCUUGCUUUGACdTdT |
| 62 | 240 | hPLP1-062 | AGAUCUUUGGCGACUACAAdTdT |
| | 241 | hPLP1-062anti | UUGUAGUCGCCAAAGAUCUdTdT |
| 63 | 242 | hPLP1-063 | UGCAGGAGAAGAGGACAAAdTdT |
| | 243 | hPLP1-063anti | UUUGUCCUCUUCUCCUGCAdTdT |
| 64 | 244 | hPLP1-064 | UGGUGGCUAAUGGUGUAACdTdT |
| | 245 | hPLP1-064anti | GUUACACCAUUAGCCACCAdTdT |
| 65 | 246 | hPLP1-065 | AGCUAAUUGAGACCUAUUUdTdT |
| | 247 | hPLP1-065anti | AAAUAGGUCUCAAUUAGCUdTdT |
| 66 | 248 | hPLP1-066 | GUGCUGAUGCCAGAAUGUAdTdT |
| | 249 | hPLP1-066anti | UACAUUCUGGCAUCAGCACdTdT |
| 67 | 250 | hPLP1-067 | UUUCCAAGGAGCUGAGAAUdTdT |
| | 251 | hPLP1-067anti | AUUCUCAGCUCCUUGGAAAdTdT |
| 68 | 252 | hPLP1-068 | UGUGCCUGUGUACAUUUACdTdT |
| | 253 | hPLP1-068anti | GUAAAUGUACACAGGCACAdTdT |
| 69 | 254 | hPLP1-069 | CAGGUACAGAGAAGGAAUGdTdT |
| | 255 | hPLP1-069anti | CAUUCCUUCUCUGUACCUGdTdT |
| 70 | 256 | hPLP1-070 | GCAUAAGGGAGCGUAGAAUdTdT |
| | 257 | hPLP1-070anti | AUUCUACGCUCCCUUAUGCdTdT |
| 71 | 258 | hPLP1-071 | UCAGGUACAGAGAAGGAAUdTdT |
| | 259 | hPLP1-071anti | AUUCCUUCUCUGUACCUGAdTdT |
| 72 | 260 | hPLP1-072 | GCUGCAGCUACACUGGUUUdTdT |
| | 261 | hPLP1-072anti | AAACCAGUGUAGCUGCAGCdTdT |
| 73 | 262 | hPLP1-073 | UCUCCCAUGGAAUGCUUUCdTdT |
| | 263 | hPLP1-073anti | GAAAGCAUUCCAUGGGAGAdTdT |
| 74 | 264 | hPLP1-074 | UGCCUUCCAGUAUGUCAUCdTdT |
| | 265 | hPLP1-074anti | GAUGACAUACUGGAAGGCAdTdT |
| 75 | 266 | hPLP1-075 | GCUGCCACUUACAACUUUGdTdT |
| | 267 | hPLP1-075anti | CAAAGUUGUAAGUGGCAGCdTdT |
| 76 | 268 | hPLP1-076 | ACUUUGCCGUCCUUAAACUdTdT |
| | 269 | hPLP1-076anti | AGUUUAAGGACGGCAAAGUdTdT |
| 77 | 270 | hPLP1-077 | ACCUUACUUACAGCAUAAGdTdT |
| | 271 | hPLP1-077anti | CUUAUGCUGUAAGUAAGGUdTdT |
| 78 | 272 | hPLP1-078 | UGGACUACUGAAGCCCUAAdTdT |
| | 273 | hPLP1-078anti | UUAGGGCUUCAGUAGUCCAdTdT |
| 79 | 274 | hPLP1-079 | CUUCCAGUAUGUCAUCUAUdTdT |
| | 275 | hPLP1-079anti | AUAGAUGACAUACUGGAAGdTdT |
| 80 | 276 | hPLP1-080 | UCUGGUCUCUCUAUUACCAdTdT |
| | 277 | hPLP1-080anti | UGGUAAUAGAGAGACCAGAdTdT |
| 81 | 278 | hPLP1-081 | GUCCCAAAGACAUGGGCUUdTdT |
| | 279 | hPLP1-081anti | AAGCCCAUGUCUUUGGGACdTdT |

Thereafter, an RNA was extracted from the cells, and PLP1 gene expression was evaluated through a quantitative PCR method (n = 3). A SuperPrep Cell Lysis RT Kit for qPCR (TOYOBO CO., LTD.) was used for RNA preparation and cDNA synthesis. For the quantitative PCR method, a Taqman probe for the PLP1 gene (Hs00166914_m1, Applied Biosystems) and that for the S18 gene (Hs99999901_s1, Applied Biosystems) were used. Results of the gene expression were normalized to the house keeping gene S18.

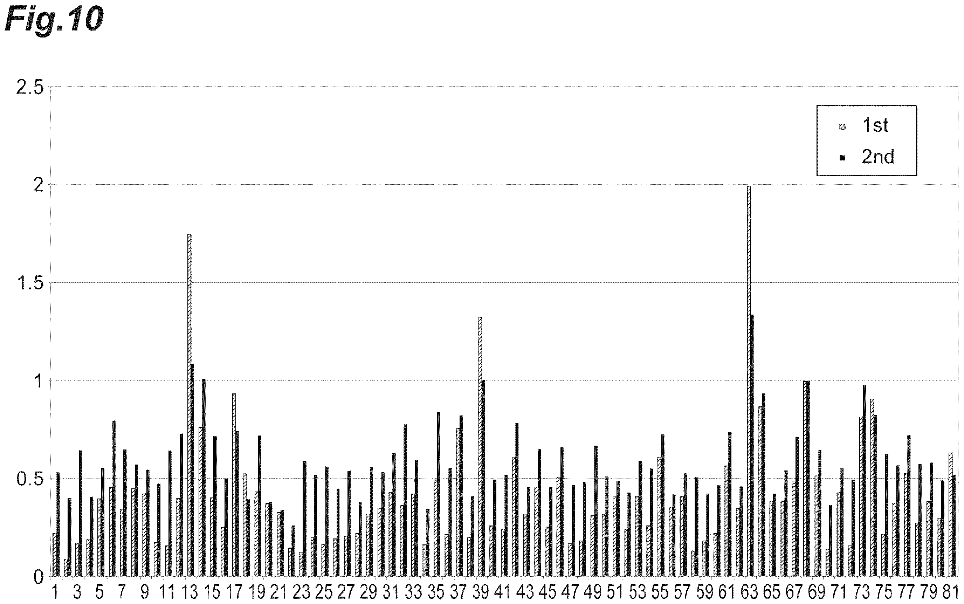

Figure 10 shows the results. The vertical axis in Figure 10 represents knockdown efficiencies with reference to U-251MG cells with addition only of transfection solution. Knockdown efficiencies for the PLP1 gene were calculated as relative expression levels assuming the expression level in U-251MG cells with addition only of transfection solution as 1. Selected were 24 pairs of siRNA sequences that lower PLP1 gene expression levels to 50% or less as a mean value both in the first and second trials (Nos. 2, 4, 10, 16, 21, 22, 26, 28, 34, 38, 40, 43, 45, 47, 48, 52, 56, 59, 60, 62, 65, 70, 72, and 80).

### <Example 8 Evaluation of Suppression Efficiency of Expression (Knockdown) in Terms of PLP1 Gene Expression Levels - Part 5>

On the basis of the 24 pairs of siRNA sequences obtained in Example 7, miRNA sequences were designed (Table 2). Among them, miRNA sequences targeting the sequence encoding the human PLP1 gene were selected (SEQ ID NOs: 2, 29, 30, 31, 32, 33, 35, 36, 42, and 51). Among them, SEQ ID NOs: 2, 29, 31, 32, and 33 are each a sequence directed to a sequence common to mice and primates such as common marmosets.

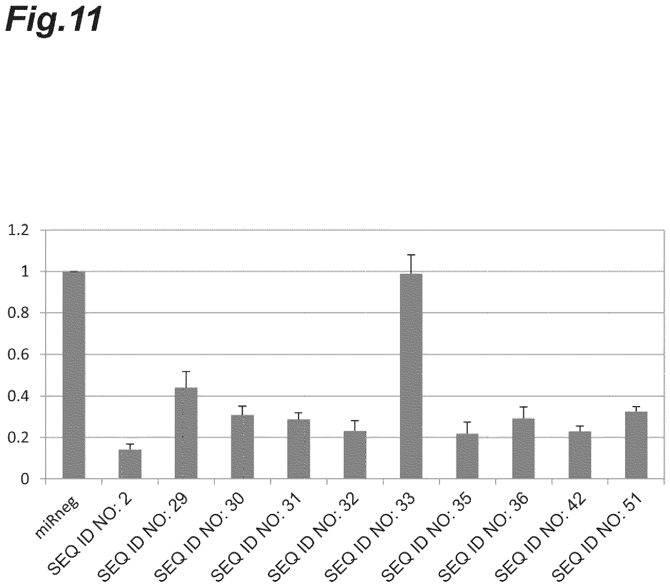

To evaluate the 10 miRNA sequences, each plasmid obtained by cloning any of the sequences into the downstream of a CAG promoter was introduced through a transfection method into HeLa cells overexpressing the coding sequences of the human PLP1 gene. The experimental details of primers used in quantitative PCR for PLP1 gene expression are the same as in Example 6. Figure 11 shows results of the knockdown. The vertical axis in Figure 11 represents the PLP1 expression levels ± SD relative to the expression when a vector including the miR-neg was added, which was set as 1. Figure 11 indicates that miRNAs having any of SEQ ID NOs: 2, 29, 30, 31, 32, 35, 36, 42, and 51 knocked down the human PLP1 gene.

### <Example 9 Evaluation of Suppression Efficiency of Expression (Knockdown) in Terms of PLP1 Gene Expression Levels - Part 6>

Each of the vectors used in Examples 6 and 8, including an miRNA having a nucleotide sequence set forth in any of SEQ ID NOs: 2, 29, 31, 32, and 35, was transfected into U-251MG cells endogenously expressing the human PLP1 gene to evaluate knockdown efficiencies for PLP1 gene expression. A plasmid obtained by cloning into the downstream of a CAG promoter was introduced into cells through a transfection method, and GFP-expressing cells were then exclusively selected by a FACS method. Thereafter, PLP1 gene expression was analyzed though a quantitative PCR method. For the primers, primers having nucleotide sequences set forth in SEQ ID NOs: 280 to 283 were used.

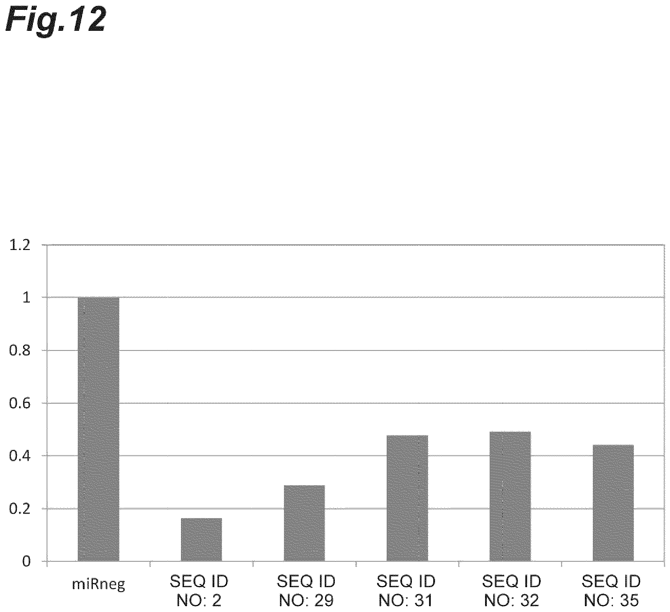

Figure 12 shows the results. The vertical axis in Figure 12 represents relative expression levels assuming the PLP1 expression level when a vector including the miR-neg was added as 1. From Figure 12, vectors including an miRNA having any of SEQ ID NOs: 2, 29, 31, 32, and 35 were found to also have suppressing effect on endogenous PLP1 gene expression.

### <Example 10 Evaluation of Suppression Efficiency of Expression (Knockdown) in Terms of PLP1 Gene Expression Levels - Part 7>

One microliter of a solution of an scAAV-AAV1/2 vector including the miRNA having SEQ ID NO: 2 from Example 9 (titer: 1.2 × 10¹² vg/mL) was injected into one striatum, the internal capsule, and the cerebellum of each 10-day-old, wild-type mouse Jcl:B6C3F1 (n = 3 per group). The injection method was the same as in Example 2. Thereafter, expression of PLP1 protein in Venus-positive cells was examined through immunostaining to evaluate PLP1 miRNA knockdown efficiencies. Immunostaining was performed with the same method as in Example 2 except that an anti-PLP1 rabbit polyclonal antibody (Numata Y et al., J Biol Chem. 2013 Mar 15; 288 (11): 7451-66) was used as a primary antibody, and an anti-rabbit fluorescent antibody (Thermo Fisher Scientific, USA) was used as a secondary antibody. Each site of the callosum, striatum, and internal capsule were photographed with a KEYENCE fluorescence microscope (KEYENCE CORPORATION, Japan).

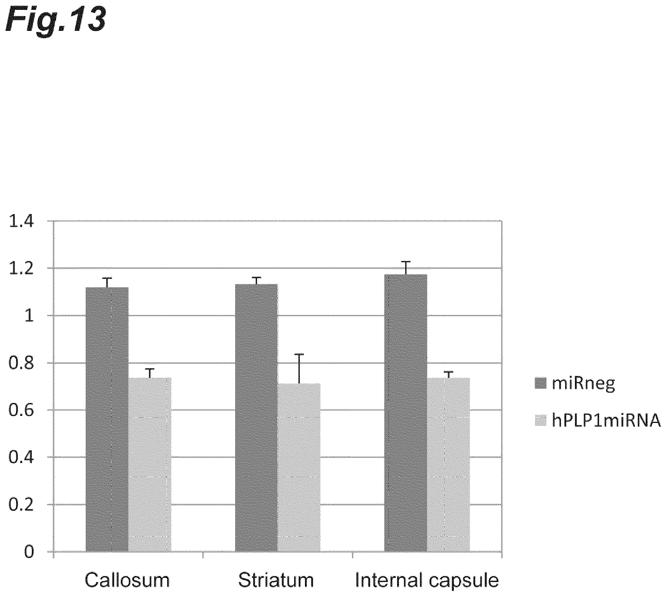

Mean fluorescence intensities of immunostained PLP1 were quantified by using Image J 1.45s (Wayne Rasband, National Institutes of Health, USA), and Figure 13 shows quantitative analysis of fluorescence intensity through PLP1 immunofluorescence staining. The vertical axis in Figure 13 represents relative PLP1 expression levels in the infected side with reference to the non-infected side. It can be seen from Figure 13 that there was almost no difference in expression of PLP1 protein between the side without injection of the miR-neg vector (non-infected side) and the side with vector injection (infected side), whereas for the PLP1 miRNA vector there was 40% of expression-suppressing effect on PLP1 protein in the infected side as compared with the non-infected side.

### Industrial Applicability

The promoter of the present invention and the AAV vector of the present invention can serve as a therapeutic drug for PMD caused by PLP1 duplication. In addition, they can serve as a therapeutic drug for PMD caused by PLP1 point mutation. Furthermore, since PLP1 is one of target antigens for multiple sclerosis, it is expected that they can serve as a therapeutic drug for multiple sclerosis.

## Claims

1. An oligodendrocyte-specific promoter comprising a nucleic acid having a sequence identity of at least 90% to a nucleotide sequence set forth in SEQ ID NO: 1.

2. The promoter according to claim 1, comprising a nucleic acid having the nucleotide sequence set forth in SEQ ID NO: 1.

3. An oligodendrocyte-specific vector comprising the promoter according to claim 1 or 2.

4. The vector according to claim 3, further comprising an miRNA sequence specific to a human PLP1 gene operably linked to the promoter.

5. An miRNA specific to a PLP1 gene, comprising a pair of nucleotide sequences consisting of an antisense sequence and a sense sequence, wherein the pair of nucleotide sequences is selected from the group consisting of pairs of an antisense sequence set forth in a left column of a table below and a sense sequence set forth in the just right of the antisense sequence in a right column of the table.
[Table 1]
**Table 1. Antisense and sense sequences for miRNA designed for human PLP1 gene**
| Antisense sequence | Sense sequence |
|---|---|
| AAAGGAAGAAGAAAGAGGCAG (SEQ ID NO: 52) | CTGCCTCTCTTCTTCCTTT (SEQ ID NO: 53) |
| AACACCAGGAGCCACACAACG (SEQ ID NO: 54) | CGTTGTGTCTCCTGGTGTT (SEQ ID NO: 55) |
| TTCCATGGGAGAACACCATAC (SEQ ID NO: 56) | GTATGGTGCTCCCATGGAA (SEQ ID NO: 57) |
| TGAGCAGGGAAACCAGTGTAG (SEQ ID NO: 58) | CTACACTGTTCCCTGCTCA (SEQ ID NO: 59) |
| AGGGCTTTCTGATTGACAGCC (SEQ ID NO: 60) | GGCTGTCACAGAAAGCCCT (SEQ ID NO: 61) |
| ACCCCAAAGAAACACAATCCA (SEQ ID NO: 62) | TGGATTGTTTCTTTGGGGT (SEQ ID NO: 63) |
| ACAAATGCAGCAATAAACAGG (SEQ ID NO: 64) | CCTGTTTAGCTGCATTTGT (SEQ ID NO: 65) |
| AATAGACTGGCAGGTGGTCCA (SEQ ID NO: 66) | TGGACCACGCCAGTCTATT (SEQ ID NO: 67) |
| AAAGAATGAGCTTGATGTTGG (SEQ ID NO: 68) | CCAACATCGCTCATTCTTT (SEQ ID NO: 69) |
| AGATACTCATAGTCTTGGTAG (SEQ ID NO: 70) | CTACCAAGTATGAGTATCT (SEQ ID NO: 71) |
| AGAAACACAATCCAGTGGCCA (SEQ ID NO: 72) | TGGCCACTATTGTGTTTCT (SEQ ID NO: 73) |
| AAATAGGTCTCAATTAGCTTT (SEQ ID NO: 74) | AAAGCTAAGAGACCTATTT (SEQ ID NO: 75) |
| AAGAAACACAATCCAGTGGCC (SEQ ID NO: 76) | GGCCACTGTTGTGTTTCTT (SEQ ID NO: 77) |
| TAAACAGGTGGAAGGTCATTT (SEQ ID NO: 78) | AAATGACCCCACCTGTTTA (SEQ ID NO: 79) |
| TTGTAGTCGCCAAAGATCTGC (SEQ ID NO: 80) | GCAGATCTGGCGACTACAA (SEQ ID NO: 81) |
| AATTAGAGCCTCCATTCCTTT (SEQ ID NO: 82) | AAAGGAATAGGCTCTAATT (SEQ ID NO: 83) |
| TTAAGGACGGCAAAGTTGTAA (SEQ ID NO: 84) | TTACAACTGCCGTCCTTAA (SEQ ID NO: 85) |
| TTTAAGGACGGCAAAGTTGTA (SEQ ID NO: 86) | TACAACTTCCGTCCTTAAA (SEQ ID NO: 87) |
| ATGTCTTTGGGACTCTGACTC (SEQ ID NO: 88) | GAGTCAGACCCAAAGACAT (SEQ ID NO: 89) |
| TATCTATCCTGTGTCTACCAG (SEQ ID NO: 90) | CTGGTAGACAGGATAGATA (SEQ ID NO: 91) |
| AAATTACTTTCTGATCCTCAG (SEQ ID NO: 92) | CTGAGGATGAAAGTAATTT (SEQ ID NO: 93) |
| TCTAACAAGCCCATGTCTTTG (SEQ ID NO: 94) | CAAAGACAGGCTTGTTAGA (SEQ ID NO: 95) |
| AATTACTTTCTGATCCTCAGG (SEQ ID NO: 96) | CCTGAGGAAGAAAGTAATT (SEQ ID NO: 97) |
| AGTAAATGTACACAGGCACAG (SEQ ID NO: 98) | CTGTGCCTGTACATTTACT (SEQ ID NO: 99) |
| TAAGTAAGGTTGGCTGAGTTA (SEQ ID NO: 100) | TAACTCAGAACCTTACTTA (SEQ ID NO: 101) |
| TTCTGTGGGTGAAAGATCCTT (SEQ ID NO: 102) | AAGGATCTCACCCACAGAA (SEQ ID NO: 103) |
| AGAAGATGCTGACAACACCCT (SEQ ID NO: 104) | AGGGTGTTCAGCATCTTCT (SEQ ID NO: 105) |
| AATTGTAGCCGGCTGGCTAGT (SEQ ID NO: 106) | ACTAGCCACGGCTACAATT (SEQ ID NO: 107) |
| AGATTTGGGCAAACGCTCTTA (SEQ ID NO: 108) | TAAGAGCGTGCCCAAATCT (SEQ ID NO: 109) |
| ATTCTACGCTCCCTTATGCTG (SEQ ID NO: 110) | CAGCATAAGAGCGTAGAAT (SEQ ID NO: 111) |
| TGGTAATAGAGAGACCAGAAT (SEQ ID NO: 112) | ATTCTGGTCTCTATTACCA (SEQ ID NO: 113) |
| TATAGATGGCAAGAGGACCAA (SEQ ID NO: 114) | TTGGTCCTTGCCATCTATA (SEQ ID NO: 115) |
| AAACCAGTGTAGCTGCAGCCC (SEQ ID NO: 116) | GGGCTGCATACACTGGTTT (SEQ ID NO: 117) |

6. The miRNA according to claim 5, comprising a nucleotide sequence selected from the group consisting of SEQ ID NOs: 2 to 7 and 24 to 51.

7. The miRNA according to claim 5, comprising a nucleotide sequence selected from the group consisting of SEQ ID NOs: 2 to 5, 7, 24, 26, 29 to 32, 35, 36, 42, and 51.

8. The miRNA according to claim 5, comprising a nucleotide sequence selected from the group consisting of SEQ ID NOs: 2, 29, 31, and 32.

9. A vector comprising a sequence of the miRNA according to any one of claims 5 to 8.

10. The vector according to claim 9, wherein the sequence of the miRNA is operably linked to an oligodendrocyte-specific promoter.

11. The vector according to claim 10, wherein the oligodendrocyte-specific promoter is the promoter according to claim 1 or 2.

12. The vector according to any one of claims 3, 4, and 9 to 11, wherein the vector is an adeno-associated virus (AAV) vector.

13. A pharmaceutical composition comprising the vector according to any one of claims 3, 4, and 9 to 12.

14. The pharmaceutical composition according to claim 13, wherein the pharmaceutical composition is a pharmaceutical composition for treating a disease associated with abnormality in a PLP1 gene.

15. The pharmaceutical composition according to claim 14, wherein the disease is Pelizaeus-Merzbacher disease, spastic paraplegia type 2, or multiple sclerosis.

16. The pharmaceutical composition according to claim 14, wherein the disease is Pelizaeus-Merzbacher disease.

17. The pharmaceutical composition according to claim 13, wherein the pharmaceutical composition is a PLP1 gene expression suppressor which suppresses expression of the PLP1 gene.

18. A method for treating a disease associated with abnormality of a PLP1 gene, the method comprising administering an effective amount of the vector according to any one of claims 3, 4, and 9 to 12 to a patient in need of treatment.

19. The method according to claim 18, wherein the disease is Pelizaeus-Merzbacher disease, spastic paraplegia type 2, or multiple sclerosis.

20. The method according to claim 18, wherein the disease is Pelizaeus-Merzbacher disease.

21. A method for suppressing expression of a PLP1 gene, the method comprising administering an effective amount of the vector according to any one of claims 3, 4, and 9 to 12 to a patient in need of treatment.

22. The vector according to any one of claims 3, 4, and 9 to 12 for use in treatment of a disease associated with abnormality of a PLP1 gene.

23. The vector according to claim 22, wherein the disease is Pelizaeus-Merzbacher disease, spastic paraplegia type 2, or multiple sclerosis.

24. The vector according to claim 22, wherein the disease is Pelizaeus-Merzbacher disease.
